(19) Europäisches Patentamt
European Patent Office
Office européen des brevets

(11) **EP 1 939 278 A1**

(12) **EUROPEAN PATENT APPLICATION**
published in accordance with Art. 153(4) EPC

(43) Date of publication:
**02.07.2008 Bulletin 2008/27**

(51) Int Cl.:
*C12N 5/06* (2006.01)        *A61K 35/14* (2006.01)
*A61P 1/16* (2006.01)        *A61P 31/04* (2006.01)
*A61P 31/10* (2006.01)       *A61P 31/12* (2006.01)
*A61P 31/16* (2006.01)       *A61P 35/00* (2006.01)
*A61P 37/04* (2006.01)       *C12N 5/10* (2006.01)
*A61K 35/76* (2006.01)       *C07K 14/78* (2006.01)
*C12N 15/09* (2006.01)

(21) Application number: **06810596.4**

(22) Date of filing: **27.09.2006**

(86) International application number:
**PCT/JP2006/319105**

(87) International publication number:
**WO 2007/040105 (12.04.2007 Gazette 2007/15)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **30.09.2005 JP 2005288983
03.04.2006 JP 2006102103
19.07.2006 JP 2006196950
06.09.2006 JP 2006241773**

(71) Applicant: **TAKARA BIO INC.
Otsu-shi,
Shiga 520-2193 (JP)**

(72) Inventors:
• **ENOKI, Tatsuji,**
**c/o Takara Bio Inc.**
**Otsu-shi, Shiga 520-2193 (JP)**
• **KATO, Akiko,**
**c/o Takara Bio Inc.**
**Otsu-shi, Shiga 520-2193 (JP)**

• **MURAKI, Nobuko,**
**c/o Takara Bio Inc.**
**Otsu-shi, Shiga 520-2193 (JP)**
• **IDENO, Mitsuko,**
**c/o Takara Bio Inc.**
**Otsu-shi, Shiga 520-2193 (JP)**
• **MARUI, Takahiro,**
**c/o Takara Bio Inc.**
**Otsu-shi, Shiga 520-2193 (JP)**
• **SAGAWA, Hiroaki,**
**c/o Takara Bio Inc.**
**Otsu-shi, Shiga 520-2193 (JP)**
• **KATO, Ikunoshin,**
**c/o Takara Bio Inc.**
**Otsu-shi, Shiga 520-2193 (JP)**

(74) Representative: **Vossius & Partner
Siebertstrasse 4
81675 München (DE)**

(54) **METHOD FOR PRODUCTION OF T CELL POPULATION**

(57)    A method for preparing a T cell population, wherein the T cell population expresses CD45RA and expresses at least one selected from the group consisting of CD62L, CCR7, CD27, and CD28, **characterized in that** the method comprises the step of culturing a cell population comprising a T cell, in the presence of fibronectin, a fragment thereof or a mixture thereof.

```
                              SEQUENCE LISTING

    <110>  TAKARA BIO INC.

    <120>  Method for Production of T cell Population

    <130>  P1599 EP

    <150>  JP 2005-288983
    <151>  2005-09-30

    <150>  JP 2006-102103
    <151>  2006-04-03

    <150>  JP 2006-196950
    <151>  2006-07-19

    <150>  JP 2006-241773
    <151>  2006-09-06

    <160>  23

    <170>  PatentIn version 3.3

    <210>  1
    <211>  87
    <212>  PRT
    <213>  Artificial Sequence

    <220>
    <221>  source
    <223>  /note="Description of artificial sequence: partial region of fibronectin
    named III-8"

    <400>  1

    Pro Thr Asp Leu Arg Phe Thr Asn Ile Gly Pro Asp Thr Met Arg Val
    1               5                   10                  15


    Thr Trp Ala Pro Pro Pro Ser Ile Asp Leu Thr Asn Phe Leu Val Arg
                20                  25                  30


    Tyr Ser Pro Val Lys Asn Glu Glu Asp Val Ala Glu Leu Ser Ile Ser
            35                  40                  45


    Pro Ser Asp Asn Ala Val Val Leu Thr Asn Leu Leu Pro Gly Thr Glu
        50                  55                  60


    Tyr Val Val Ser Val Ser Ser Val Tyr Glu Gln His Glu Ser Thr Pro
    65                  70                  75                  80


    Leu Arg Gly Arg Gln Lys Thr
                        85


    <210>  2
    <211>  90
    <212>  PRT
    <213>  Artificial Sequence
```

```
<220>
<221>   source
<223>   /note="Description of artificial sequence: partial region of fibronectin
named III-9"

<400>   2

Gly Leu Asp Ser Pro Thr Gly Ile Asp Phe Ser Asp Ile Thr Ala Asn
1               5                   10                  15

Ser Phe Thr Val His Trp Ile Ala Pro Arg Ala Thr Ile Thr Gly Tyr
            20                  25                  30

Arg Ile Arg His His Pro Glu His Phe Ser Gly Arg Pro Arg Glu Asp
            35                  40                  45

Arg Val Pro His Ser Arg Asn Ser Ile Thr Leu Thr Asn Leu Thr Pro
        50                  55                  60

Gly Thr Glu Tyr Val Val Ser Ile Val Ala Leu Asn Gly Arg Glu Glu
65                  70                  75                  80

Ser Pro Leu Leu Ile Gly Gln Gln Ser Thr
                85                  90


<210>   3
<211>   94
<212>   PRT
<213>   Artificial Sequence

<220>
<221>   source
<223>   /note="Description of artificial sequence: partial region of fibronectin
named III-10"

<400>   3

Val Ser Asp Val Pro Arg Asp Leu Glu Val Val Ala Ala Thr Pro Thr
1               5                   10                  15

Ser Leu Leu Ile Ser Trp Asp Ala Pro Ala Val Thr Val Arg Tyr Tyr
            20                  25                  30

Arg Ile Thr Tyr Gly Glu Thr Gly Gly Asn Ser Pro Val Gln Glu Phe
            35                  40                  45

Thr Val Pro Gly Ser Lys Ser Thr Ala Thr Ile Ser Gly Leu Lys Pro
        50                  55                  60

Gly Val Asp Tyr Thr Ile Thr Val Tyr Ala Val Thr Gly Arg Gly Asp
65                  70                  75                  80
```

```
Ser Pro Ala Ser Ser Lys Pro Ile Ser Ile Asn Tyr Arg Thr
                85                  90
```

```
<210>  4
<211>  84
<212>  PRT
<213>  Artificial Sequence

<220>
<221>  source
<223>  /note="Description of artificial sequence: partial region of fibronectin
named III-11"

<400>  4
```

```
Gln Met Gln Val Thr Asp Val Gln Asp Asn Ser Ile Ser Val Lys Trp
1               5                   10                  15


Leu Pro Ser Ser Ser Pro Val Thr Gly Tyr Arg Val Thr Thr Thr Pro
            20                  25                  30


Lys Asn Gly Pro Gly Pro Thr Lys Thr Lys Thr Ala Gly Pro Asp Gln
        35                  40                  45


Thr Glu Met Thr Ile Glu Gly Leu Gln Pro Thr Val Glu Tyr Val Val
    50                  55                  60


Ser Val Tyr Ala Gln Asn Pro Ser Gly Glu Ser Gln Pro Leu Val Gln
65                  70                  75                  80


Thr Ala Val Thr
```

```
<210>  5
<211>  92
<212>  PRT
<213>  Artificial Sequence

<220>
<221>  source
<223>  /note="Description of artificial sequence: partial region of fibronectin
named III-12"

<400>  5
```

```
Ala Ile Pro Ala Pro Thr Asp Leu Lys Phe Thr Gln Val Thr Pro Thr
1               5                   10                  15


Ser Leu Ser Ala Gln Trp Thr Pro Pro Asn Val Gln Leu Thr Gly Tyr
            20                  25                  30


Arg Val Arg Val Thr Pro Lys Glu Lys Thr Gly Pro Met Lys Glu Ile
        35                  40                  45
```

```
Asn Leu Ala Pro Asp Ser Ser Ser Val Val Val Ser Gly Leu Met Val
    50                  55              60
```

```
Ala Thr Lys Tyr Glu Val Ser Val Tyr Ala Leu Lys Asp Thr Leu Thr
65                  70              75              80
```

```
Ser Arg Pro Ala Gln Gly Val Val Thr Thr Leu Glu
                85              90
```

```
<210>   6
<211>   89
<212>   PRT
<213>   Artificial Sequence

<220>
<221>   source
<223>   /note="Description of artificial sequence: partial region of fibronectin
named III-13"

<400>   6
```

```
Asn Val Ser Pro Pro Arg Arg Ala Arg Val Thr Asp Ala Thr Glu Thr
1               5               10              15
```

```
Thr Ile Thr Ile Ser Trp Arg Thr Lys Thr Glu Thr Ile Thr Gly Phe
            20              25              30
```

```
Gln Val Asp Ala Val Pro Ala Asn Gly Gln Thr Pro Ile Gln Arg Thr
            35              40              45
```

```
Ile Lys Pro Asp Val Arg Ser Tyr Thr Ile Thr Gly Leu Gln Pro Gly
    50              55              60
```

```
Thr Asp Tyr Lys Ile Tyr Leu Tyr Thr Leu Asn Asp Asn Ala Arg Ser
65              70              75              80
```

```
Ser Pro Val Val Ile Asp Ala Ser Thr
                85
```

```
<210>   7
<211>   90
<212>   PRT
<213>   Artificial Sequence

<220>
<221>   source
<223>   /note="Description of artificial sequence: partial region of fibronectin
named III-14"

<400>   7
```

```
Ala Ile Asp Ala Pro Ser Asn Leu Arg Phe Leu Ala Thr Thr Pro Asn
1               5               10              15
```

Ser Leu Leu Val Ser Trp Gln Pro Pro Arg Ala Arg Ile Thr Gly Tyr
              20                  25                  30

Ile Ile Lys Tyr Glu Lys Pro Gly Ser Pro Pro Arg Glu Val Val Pro
              35                  40                  45

Arg Pro Arg Pro Gly Val Thr Glu Ala Thr Ile Thr Gly Leu Glu Pro
         50                  55                  60

Gly Thr Glu Tyr Thr Ile Tyr Val Ile Ala Leu Lys Asn Asn Gln Lys
65                  70                  75                  80

Ser Glu Pro Leu Ile Gly Arg Lys Lys Thr
                   85                  90


<210>   8
<211>   25
<212>   PRT
<213>   Artificial Sequence

<220>
<221>   source
<223>   /note="Description of artificial sequence: partial region of fibronectin named CS-1"

<400>   8

Asp Glu Leu Pro Gln Leu Val Thr Leu Pro His Pro Asn Leu His Gly
1                   5                   10                  15

Pro Glu Ile Leu Asp Val Pro Ser Thr
                   20                  25


<210>   9
<211>   274
<212>   PRT
<213>   Artificial Sequence

<220>
<221>   source
<223>   /note="Description of artificial sequence: fibronectin fragment named C-274"

<400>   9

Pro Thr Asp Leu Arg Phe Thr Asn Ile Gly Pro Asp Thr Met Arg Val
1                   5                   10                  15

Thr Trp Ala Pro Pro Pro Ser Ile Asp Leu Thr Asn Phe Leu Val Arg
              20                  25                  30

Tyr Ser Pro Val Lys Asn Glu Glu Asp Val Ala Glu Leu Ser Ile Ser
              35                  40                  45

```
Pro Ser Asp Asn Ala Val Val Leu Thr Asn Leu Leu Pro Gly Thr Glu
    50                  55                  60

Tyr Val Val Ser Val Ser Ser Val Tyr Glu Gln His Glu Ser Thr Pro
65                  70                  75                  80

Leu Arg Gly Arg Gln Lys Thr Gly Leu Asp Ser Pro Thr Gly Ile Asp
                85                  90                  95

Phe Ser Asp Ile Thr Ala Asn Ser Phe Thr Val His Trp Ile Ala Pro
                100                 105                 110

Arg Ala Thr Ile Thr Gly Tyr Arg Ile Arg His His Pro Glu His Phe
            115                 120                 125

Ser Gly Arg Pro Arg Glu Asp Arg Val Pro His Ser Arg Asn Ser Ile
    130                 135                 140

Thr Leu Thr Asn Leu Thr Pro Gly Thr Glu Tyr Val Val Ser Ile Val
145                 150                 155                 160

Ala Leu Asn Gly Arg Glu Glu Ser Pro Leu Leu Ile Gly Gln Gln Ser
                165                 170                 175

Thr Val Ser Asp Val Pro Arg Asp Leu Glu Val Val Ala Ala Thr Pro
            180                 185                 190

Thr Ser Leu Leu Ile Ser Trp Asp Ala Pro Ala Val Thr Val Arg Tyr
    195                 200                 205

Tyr Arg Ile Thr Tyr Gly Glu Thr Gly Gly Asn Ser Pro Val Gln Glu
    210                 215                 220

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
                245                 250                 255

Asp Ser Pro Ala Ser Ser Lys Pro Ile Ser Ile Asn Tyr Arg Thr Glu
                260                 265                 270

Ile Asp


<210>  10
<211>  271
<212>  PRT
<213>  Artificial Sequence
```

```
<220>
<221>  source
<223>  /note="Description of artificial sequence: fibronectin fragment named
H-271"

<400>  10

Ala Ile Pro Ala Pro Thr Asp Leu Lys Phe Thr Gln Val Thr Pro Thr
1               5                   10                  15

Ser Leu Ser Ala Gln Trp Thr Pro Pro Asn Val Gln Leu Thr Gly Tyr
            20                  25                  30

Arg Val Arg Val Thr Pro Lys Glu Lys Thr Gly Pro Met Lys Glu Ile
        35                  40                  45

Asn Leu Ala Pro Asp Ser Ser Ser Val Val Val Ser Gly Leu Met Val
    50                  55                  60

Ala Thr Lys Tyr Glu Val Ser Val Tyr Ala Leu Lys Asp Thr Leu Thr
65                  70                  75                  80

Ser Arg Pro Ala Gln Gly Val Val Thr Thr Leu Glu Asn Val Ser Pro
            85                  90                  95

Pro Arg Arg Ala Arg Val Thr Asp Ala Thr Glu Thr Thr Ile Thr Ile
        100                 105                 110

Ser Trp Arg Thr Lys Thr Glu Thr Ile Thr Gly Phe Gln Val Asp Ala
        115                 120                 125

Val Pro Ala Asn Gly Gln Thr Pro Ile Gln Arg Thr Ile Lys Pro Asp
    130                 135                 140

Val Arg Ser Tyr Thr Ile Thr Gly Leu Gln Pro Gly Thr Asp Tyr Lys
145                 150                 155                 160

Ile Tyr Leu Tyr Thr Leu Asn Asp Asn Ala Arg Ser Ser Pro Val Val
                165                 170                 175

Ile Asp Ala Ser Thr Ala Ile Asp Ala Pro Ser Asn Leu Arg Phe Leu
            180                 185                 190

Ala Thr Thr Pro Asn Ser Leu Leu Val Ser Trp Gln Pro Pro Arg Ala
        195                 200                 205

Arg Ile Thr Gly Tyr Ile Ile Lys Tyr Glu Lys Pro Gly Ser Pro Pro
        210                 215                 220
```

```
Arg Glu Val Val Pro Arg Pro Arg Pro Gly Val Thr Glu Ala Thr Ile
225                 230             235                 240


Thr Gly Leu Glu Pro Gly Thr Glu Tyr Thr Ile Tyr Val Ile Ala Leu
            245             250             255


Lys Asn Asn Gln Lys Ser Glu Pro Leu Ile Gly Arg Lys Lys Thr
            260             265             270
```

```
<210>  11
<211>  296
<212>  PRT
<213>  Artificial Sequence

<220>
<221>  source
<223>  /note="Description of artificial sequence: fibronectin fragment named
H-296"

<400>  11
```

```
Ala Ile Pro Ala Pro Thr Asp Leu Lys Phe Thr Gln Val Thr Pro Thr
1               5                   10                  15


Ser Leu Ser Ala Gln Trp Thr Pro Pro Asn Val Gln Leu Thr Gly Tyr
            20              25              30


Arg Val Arg Val Thr Pro Lys Glu Lys Thr Gly Pro Met Lys Glu Ile
        35              40              45


Asn Leu Ala Pro Asp Ser Ser Ser Val Val Val Ser Gly Leu Met Val
    50              55              60


Ala Thr Lys Tyr Glu Val Ser Val Tyr Ala Leu Lys Asp Thr Leu Thr
65              70              75                  80


Ser Arg Pro Ala Gln Gly Val Val Thr Thr Leu Glu Asn Val Ser Pro
            85              90              95


Pro Arg Arg Ala Arg Val Thr Asp Ala Thr Glu Thr Thr Ile Thr Ile
            100             105             110


Ser Trp Arg Thr Lys Thr Glu Thr Ile Thr Gly Phe Gln Val Asp Ala
        115             120             125


Val Pro Ala Asn Gly Gln Thr Pro Ile Gln Arg Thr Ile Lys Pro Asp
    130             135             140


Val Arg Ser Tyr Thr Ile Thr Gly Leu Gln Pro Gly Thr Asp Tyr Lys
145             150             155             160
```

```
Ile Tyr Leu Tyr Thr Leu Asn Asp Asn Ala Arg Ser Ser Pro Val Val
                165                 170                 175

Ile Asp Ala Ser Thr Ala Ile Asp Ala Pro Ser Asn Leu Arg Phe Leu
            180                 185                 190

Ala Thr Thr Pro Asn Ser Leu Leu Val Ser Trp Gln Pro Pro Arg Ala
        195                 200                 205

Arg Ile Thr Gly Tyr Ile Ile Lys Tyr Glu Lys Pro Gly Ser Pro Pro
    210                 215                 220

Arg Glu Val Val Pro Arg Pro Arg Pro Gly Val Thr Glu Ala Thr Ile
225                 230                 235                 240

Thr Gly Leu Glu Pro Gly Thr Glu Tyr Thr Ile Tyr Val Ile Ala Leu
                245                 250                 255

Lys Asn Asn Gln Lys Ser Glu Pro Leu Ile Gly Arg Lys Lys Thr Asp
                260                 265                 270

Glu Leu Pro Gln Leu Val Thr Leu Pro His Pro Asn Leu His Gly Pro
        275                 280                 285

Glu Ile Leu Asp Val Pro Ser Thr
    290                 295


<210>  12
<211>  549
<212>  PRT
<213>  Artificial Sequence

<220>
<221>  source
<223>  /note="Description of artificial sequence: fibronectin fragment named
CH-271"

<400>  12

Pro Thr Asp Leu Arg Phe Thr Asn Ile Gly Pro Asp Thr Met Arg Val
1               5                   10                  15

Thr Trp Ala Pro Pro Pro Ser Ile Asp Leu Thr Asn Phe Leu Val Arg
            20                  25                  30

Tyr Ser Pro Val Lys Asn Glu Glu Asp Val Ala Glu Leu Ser Ile Ser
        35                  40                  45

Pro Ser Asp Asn Ala Val Val Leu Thr Asn Leu Leu Pro Gly Thr Glu
        50                  55                  60
```

```
Tyr Val Val Ser Val Ser Ser Val Tyr Glu Gln His Glu Ser Thr Pro
65              70              75                  80

Leu Arg Gly Arg Gln Lys Thr Gly Leu Asp Ser Pro Thr Gly Ile Asp
            85              90              95

Phe Ser Asp Ile Thr Ala Asn Ser Phe Thr Val His Trp Ile Ala Pro
            100             105             110

Arg Ala Thr Ile Thr Gly Tyr Arg Ile Arg His His Pro Glu His Phe
    115             120             125

Ser Gly Arg Pro Arg Glu Asp Arg Val Pro His Ser Arg Asn Ser Ile
    130             135             140

Thr Leu Thr Asn Leu Thr Pro Gly Thr Glu Tyr Val Val Ser Ile Val
145             150             155             160

Ala Leu Asn Gly Arg Glu Glu Ser Pro Leu Leu Ile Gly Gln Gln Ser
            165             170             175

Thr Val Ser Asp Val Pro Arg Asp Leu Glu Val Val Ala Ala Thr Pro
            180             185             190

Thr Ser Leu Leu Ile Ser Trp Asp Ala Pro Ala Val Thr Val Arg Tyr
    195             200             205

Tyr Arg Ile Thr Tyr Gly Glu Thr Gly Gly Asn Ser Pro Val Gln Glu
    210             215             220

Phe Thr Val Pro Gly Ser Lys Ser Thr Ala Thr Ile Ser Gly Leu Lys
225             230             235             240

Pro Gly Val Asp Tyr Thr Ile Thr Val Tyr Ala Val Thr Gly Arg Gly
            245             250             255

Asp Ser Pro Ala Ser Ser Lys Pro Ile Ser Ile Asn Tyr Arg Thr Glu
            260             265             270

Ile Asp Lys Pro Ser Met Ala Ile Pro Ala Pro Thr Asp Leu Lys Phe
    275             280             285

Thr Gln Val Thr Pro Thr Ser Leu Ser Ala Gln Trp Thr Pro Pro Asn
    290             295             300

Val Gln Leu Thr Gly Tyr Arg Val Arg Val Thr Pro Lys Glu Lys Thr
305             310             315             320
```

```
Gly Pro Met Lys Glu Ile Asn Leu Ala Pro Asp Ser Ser Ser Val Val
            325                 330                 335

Val Ser Gly Leu Met Val Ala Thr Lys Tyr Glu Val Ser Val Tyr Ala
            340                 345                 350

Leu Lys Asp Thr Leu Thr Ser Arg Pro Ala Gln Gly Val Val Thr Thr
            355                 360                 365

Leu Glu Asn Val Ser Pro Pro Arg Arg Ala Arg Val Thr Asp Ala Thr
        370                 375                 380

Glu Thr Thr Ile Thr Ile Ser Trp Arg Thr Lys Thr Glu Thr Ile Thr
385                 390                 395                 400

Gly Phe Gln Val Asp Ala Val Pro Ala Asn Gly Gln Thr Pro Ile Gln
                405                 410                 415

Arg Thr Ile Lys Pro Asp Val Arg Ser Tyr Thr Ile Thr Gly Leu Gln
            420                 425                 430

Pro Gly Thr Asp Tyr Lys Ile Tyr Leu Tyr Thr Leu Asn Asp Asn Ala
            435                 440                 445

Arg Ser Ser Pro Val Val Ile Asp Ala Ser Thr Ala Ile Asp Ala Pro
        450                 455                 460

Ser Asn Leu Arg Phe Leu Ala Thr Thr Pro Asn Ser Leu Leu Val Ser
465                 470                 475                 480

Trp Gln Pro Pro Arg Ala Arg Ile Thr Gly Tyr Ile Ile Lys Tyr Glu
                485                 490                 495

Lys Pro Gly Ser Pro Pro Arg Glu Val Val Pro Arg Pro Arg Pro Gly
            500                 505                 510

Val Thr Glu Ala Thr Ile Thr Gly Leu Glu Pro Gly Thr Glu Tyr Thr
            515                 520                 525

Ile Tyr Val Ile Ala Leu Lys Asn Asn Gln Lys Ser Glu Pro Leu Ile
            530                 535                 540

Gly Arg Lys Lys Thr
545


<210>  13
<211>  574
<212>  PRT
<213>  Artificial Sequence
```

```
<220>
<221>  source
<223>  /note="Description of artificial sequence: fibronectin fragment named
CH-296"

<400>  13

Pro Thr Asp Leu Arg Phe Thr Asn Ile Gly Pro Asp Thr Met Arg Val
1               5                   10                  15


Thr Trp Ala Pro Pro Pro Ser Ile Asp Leu Thr Asn Phe Leu Val Arg
            20                  25                  30


Tyr Ser Pro Val Lys Asn Glu Glu Asp Val Ala Glu Leu Ser Ile Ser
        35                  40                  45


Pro Ser Asp Asn Ala Val Val Leu Thr Asn Leu Leu Pro Gly Thr Glu
        50                  55                  60


Tyr Val Val Ser Val Ser Ser Val Tyr Glu Gln His Glu Ser Thr Pro
65                  70                  75                  80


Leu Arg Gly Arg Gln Lys Thr Gly Leu Asp Ser Pro Thr Gly Ile Asp
                85                  90                  95


Phe Ser Asp Ile Thr Ala Asn Ser Phe Thr Val His Trp Ile Ala Pro
            100                 105                 110


Arg Ala Thr Ile Thr Gly Tyr Arg Ile Arg His His Pro Glu His Phe
            115                 120                 125


Ser Gly Arg Pro Arg Glu Asp Arg Val Pro His Ser Arg Asn Ser Ile
        130                 135                 140


Thr Leu Thr Asn Leu Thr Pro Gly Thr Glu Tyr Val Val Ser Ile Val
145                 150                 155                 160


Ala Leu Asn Gly Arg Glu Glu Ser Pro Leu Leu Ile Gly Gln Gln Ser
                165                 170                 175


Thr Val Ser Asp Val Pro Arg Asp Leu Glu Val Val Ala Ala Thr Pro
            180                 185                 190


Thr Ser Leu Leu Ile Ser Trp Asp Ala Pro Ala Val Thr Val Arg Tyr
            195                 200                 205


Tyr Arg Ile Thr Tyr Gly Glu Thr Gly Gly Asn Ser Pro Val Gln Glu
        210                 215                 220
```

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
                245                 250                 255

Asp Ser Pro Ala Ser Ser Lys Pro Ile Ser Ile Asn Tyr Arg Thr Glu
                260                 265                 270

Ile Asp Lys Pro Ser Met Ala Ile Pro Ala Pro Thr Asp Leu Lys Phe
                275                 280                 285

Thr Gln Val Thr Pro Thr Ser Leu Ser Ala Gln Trp Thr Pro Pro Asn
                290                 295                 300

Val Gln Leu Thr Gly Tyr Arg Val Arg Val Thr Pro Lys Glu Lys Thr
305                 310                 315                 320

Gly Pro Met Lys Glu Ile Asn Leu Ala Pro Asp Ser Ser Ser Val Val
                325                 330                 335

Val Ser Gly Leu Met Val Ala Thr Lys Tyr Glu Val Ser Val Tyr Ala
                340                 345                 350

Leu Lys Asp Thr Leu Thr Ser Arg Pro Ala Gln Gly Val Val Thr Thr
                355                 360                 365

Leu Glu Asn Val Ser Pro Pro Arg Arg Ala Arg Val Thr Asp Ala Thr
                370                 375                 380

Glu Thr Thr Ile Thr Ile Ser Trp Arg Thr Lys Thr Glu Thr Ile Thr
385                 390                 395                 400

Gly Phe Gln Val Asp Ala Val Pro Ala Asn Gly Gln Thr Pro Ile Gln
                405                 410                 415

Arg Thr Ile Lys Pro Asp Val Arg Ser Tyr Thr Ile Thr Gly Leu Gln
                420                 425                 430

Pro Gly Thr Asp Tyr Lys Ile Tyr Leu Tyr Thr Leu Asn Asp Asn Ala
                435                 440                 445

Arg Ser Ser Pro Val Val Ile Asp Ala Ser Thr Ala Ile Asp Ala Pro
                450                 455                 460

Ser Asn Leu Arg Phe Leu Ala Thr Thr Pro Asn Ser Leu Leu Val Ser
465                 470                 475                 480

```
Trp Gln Pro Pro Arg Ala Arg Ile Thr Gly Tyr Ile Ile Lys Tyr Glu
            485             490                 495

Lys Pro Gly Ser Pro Pro Arg Glu Val Val Pro Arg Pro Arg Pro Gly
            500             505                 510

Val Thr Glu Ala Thr Ile Thr Gly Leu Glu Pro Gly Thr Glu Tyr Thr
            515             520                 525

Ile Tyr Val Ile Ala Leu Lys Asn Asn Gln Lys Ser Glu Pro Leu Ile
        530             535                 540

Gly Arg Lys Lys Thr Asp Glu Leu Pro Gln Leu Val Thr Leu Pro His
545             550                 555                 560

Pro Asn Leu His Gly Pro Glu Ile Leu Asp Val Pro Ser Thr
            565                 570
```

```
<210>  14
<211>  302
<212>  PRT
<213>  Artificial Sequence

<220>
<221>  source
<223>  /note="Description of artificial sequence: fibronectin fragment named
C-CS1"

<400>  14
```

```
Pro Thr Asp Leu Arg Phe Thr Asn Ile Gly Pro Asp Thr Met Arg Val
1               5               10                  15

Thr Trp Ala Pro Pro Pro Ser Ile Asp Leu Thr Asn Phe Leu Val Arg
            20              25                  30

Tyr Ser Pro Val Lys Asn Glu Glu Asp Val Ala Glu Leu Ser Ile Ser
        35              40                  45

Pro Ser Asp Asn Ala Val Val Leu Thr Asn Leu Leu Pro Gly Thr Glu
        50              55                  60

Tyr Val Val Ser Val Ser Ser Val Tyr Glu Gln His Glu Ser Thr Pro
65              70              75                  80

Leu Arg Gly Arg Gln Lys Thr Gly Leu Asp Ser Pro Thr Gly Ile Asp
            85              90                  95

Phe Ser Asp Ile Thr Ala Asn Ser Phe Thr Val His Trp Ile Ala Pro
            100             105                 110
```

```
Arg Ala Thr Ile Thr Gly Tyr Arg Ile Arg His His Pro Glu His Phe
        115                 120                 125

Ser Gly Arg Pro Arg Glu Asp Arg Val Pro His Ser Arg Asn Ser Ile
        130                 135                 140

Thr Leu Thr Asn Leu Thr Pro Gly Thr Glu Tyr Val Val Ser Ile Val
145                 150                 155                 160

Ala Leu Asn Gly Arg Glu Glu Ser Pro Leu Leu Ile Gly Gln Gln Ser
                165                 170                 175

Thr Val Ser Asp Val Pro Arg Asp Leu Glu Val Val Ala Ala Thr Pro
                180                 185                 190

Thr Ser Leu Leu Ile Ser Trp Asp Ala Pro Ala Val Thr Val Arg Tyr
        195                 200                 205

Tyr Arg Ile Thr Tyr Gly Glu Thr Gly Gly Asn Ser Pro Val Gln Glu
        210                 215                 220

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
                245                 250                 255

Asp Ser Pro Ala Ser Ser Lys Pro Ile Ser Ile Asn Tyr Arg Thr Glu
                260                 265                 270

Ile Asp Lys Pro Ser Asp Glu Leu Pro Gln Leu Val Thr Leu Pro His
        275                 280                 285

Pro Asn Leu His Gly Pro Glu Ile Leu Asp Val Pro Ser Thr
        290                 295                 300


<210>  15
<211>  658
<212>  PRT
<213>  Artificial Sequence

<220>
<221>  source
<223>  /note="Description of artificial sequence: fibronectin fragment named
CH-296Na"

<400>  15

Met Pro Thr Asp Leu Arg Phe Thr Asn Ile Gly Pro Asp Thr Met Arg
1                   5                   10                  15
```

Val Thr Trp Ala Pro Pro Pro Ser Ile Asp Leu Thr Asn Phe Leu Val
20 25 30

Arg Tyr Ser Pro Val Lys Asn Glu Glu Asp Val Ala Glu Leu Ser Ile
35 40 45

Ser Pro Ser Asp Asn Ala Val Val Leu Thr Asn Leu Leu Pro Gly Thr
50 55 60

Glu Tyr Val Val Ser Val Ser Ser Val Tyr Glu Gln His Glu Ser Thr
65 70 75 80

Pro Leu Arg Gly Arg Gln Lys Thr Gly Leu Asp Ser Pro Thr Gly Ile
85 90 95

Asp Phe Ser Asp Ile Thr Ala Asn Ser Phe Thr Val His Trp Ile Ala
100 105 110

Pro Arg Ala Thr Ile Thr Gly Tyr Arg Ile Arg His His Pro Glu His
115 120 125

Phe Ser Gly Arg Pro Arg Glu Asp Arg Val Pro His Ser Arg Asn Ser
130 135 140

Ile Thr Leu Thr Asn Leu Thr Pro Gly Thr Glu Tyr Val Val Ser Ile
145 150 155 160

Val Ala Leu Asn Gly Arg Glu Glu Ser Pro Leu Leu Ile Gly Gln Gln
165 170 175

Ser Thr Val Ser Asp Val Pro Arg Asp Leu Glu Val Val Ala Ala Thr
180 185 190

Pro Thr Ser Leu Leu Ile Ser Trp Asp Ala Pro Ala Val Thr Val Arg
195 200 205

Tyr Tyr Arg Ile Thr Tyr Gly Glu Thr Gly Gly Asn Ser Pro Val Gln
210 215 220

Glu Phe Thr Val Pro Gly Ser Lys Ser Thr Ala Thr Ile Ser Gly Leu
225 230 235 240

Lys Pro Gly Val Asp Tyr Thr Ile Thr Val Tyr Ala Val Thr Gly Arg
245 250 255

Gly Asp Ser Pro Ala Ser Ser Lys Pro Ile Ser Ile Asn Tyr Arg Thr
260 265 270

```
Glu Ile Asp Lys Pro Ser Gln Met Gln Val Thr Asp Val Gln Asp Asn
        275                 280                 285

Ser Ile Ser Val Lys Trp Leu Pro Ser Ser Pro Val Thr Gly Tyr
        290                 295                 300

Arg Val Thr Thr Thr Pro Lys Asn Gly Pro Gly Pro Thr Lys Thr Lys
305                 310                 315                 320

Thr Ala Gly Pro Asp Gln Thr Glu Met Thr Ile Glu Gly Leu Gln Pro
                325                 330                 335

Thr Val Glu Tyr Val Val Ser Val Tyr Ala Gln Asn Pro Ser Gly Glu
                340                 345                 350

Ser Gln Pro Leu Val Gln Thr Ala Val Thr Ala Ile Pro Ala Pro Thr
        355                 360                 365

Asp Leu Lys Phe Thr Gln Val Thr Pro Thr Ser Leu Ser Ala Gln Trp
        370                 375                 380

Thr Pro Pro Asn Val Gln Leu Thr Gly Tyr Arg Val Arg Val Thr Pro
385                 390                 395                 400

Lys Glu Lys Thr Gly Pro Met Lys Glu Ile Asn Leu Ala Pro Asp Ser
                405                 410                 415

Ser Ser Val Val Val Ser Gly Leu Met Val Ala Thr Lys Tyr Glu Val
        420                 425                 430

Ser Val Tyr Ala Leu Lys Asp Thr Leu Thr Ser Arg Pro Ala Gln Gly
        435                 440                 445

Val Val Thr Thr Leu Glu Asn Val Ser Pro Pro Arg Arg Ala Arg Val
        450                 455                 460

Thr Asp Ala Thr Glu Thr Thr Ile Thr Ile Ser Trp Arg Thr Lys Thr
465                 470                 475                 480

Glu Thr Ile Thr Gly Phe Gln Val Asp Ala Val Pro Ala Asn Gly Gln
                485                 490                 495

Thr Pro Ile Gln Arg Thr Ile Lys Pro Asp Val Arg Ser Tyr Thr Ile
                500                 505                 510

Thr Gly Leu Gln Pro Gly Thr Asp Tyr Lys Ile Tyr Leu Tyr Thr Leu
        515                 520                 525
```

```
Asn Asp Asn Ala Arg Ser Ser Pro Val Val Ile Asp Ala Ser Thr Ala
    530                 535                 540

Ile Asp Ala Pro Ser Asn Leu Arg Phe Leu Ala Thr Thr Pro Asn Ser
545                 550                 555                 560

Leu Leu Val Ser Trp Gln Pro Pro Arg Ala Arg Ile Thr Gly Tyr Ile
                565                 570                 575

Ile Lys Tyr Glu Lys Pro Gly Ser Pro Pro Arg Glu Val Val Pro Arg
                580                 585                 590

Pro Arg Pro Gly Val Thr Glu Ala Thr Ile Thr Gly Leu Glu Pro Gly
        595                 600                 605

Thr Glu Tyr Thr Ile Tyr Val Ile Ala Leu Lys Asn Asn Gln Lys Ser
    610                 615                 620

Glu Pro Leu Ile Gly Arg Lys Lys Thr Asp Glu Leu Pro Gln Leu Val
625                 630                 635                 640

Thr Leu Pro His Pro Asn Leu His Gly Pro Glu Ile Leu Asp Val Pro
                645                 650                 655

Ser Thr
```

```
<210>  16
<211>  367
<212>  PRT
<213>  Artificial Sequence

<220>
<221>  source
<223>  /note="Description of artificial sequence: fibronectin fragment named
CHV-89"

<400>  16
```

```
Pro Thr Asp Leu Arg Phe Thr Asn Ile Gly Pro Asp Thr Met Arg Val
1               5                   10                  15

Thr Trp Ala Pro Pro Pro Ser Ile Asp Leu Thr Asn Phe Leu Val Arg
                20                  25                  30

Tyr Ser Pro Val Lys Asn Glu Glu Asp Val Ala Glu Leu Ser Ile Ser
        35                  40                  45

Pro Ser Asp Asn Ala Val Val Leu Thr Asn Leu Leu Pro Gly Thr Glu
        50                  55                  60
```

```
Tyr Val Val Ser Val Ser Ser Val Tyr Glu Gln His Glu Ser Thr Pro
65              70              75              80

Leu Arg Gly Arg Gln Lys Thr Gly Leu Asp Ser Pro Thr Gly Ile Asp
            85              90              95

Phe Ser Asp Ile Thr Ala Asn Ser Phe Thr Val His Trp Ile Ala Pro
            100             105             110

Arg Ala Thr Ile Thr Gly Tyr Arg Ile Arg His His Pro Glu His Phe
        115             120             125

Ser Gly Arg Pro Arg Glu Asp Arg Val Pro His Ser Arg Asn Ser Ile
    130             135             140

Thr Leu Thr Asn Leu Thr Pro Gly Thr Glu Tyr Val Val Ser Ile Val
145             150             155             160

Ala Leu Asn Gly Arg Glu Glu Ser Pro Leu Leu Ile Gly Gln Gln Ser
            165             170             175

Thr Val Ser Asp Val Pro Arg Asp Leu Glu Val Val Ala Ala Thr Pro
            180             185             190

Thr Ser Leu Leu Ile Ser Trp Asp Ala Pro Ala Val Thr Val Arg Tyr
        195             200             205

Tyr Arg Ile Thr Tyr Gly Glu Thr Gly Gly Asn Ser Pro Val Gln Glu
    210             215             220

Phe Thr Val Pro Gly Ser Lys Ser Thr Ala Thr Ile Ser Gly Leu Lys
225             230             235             240

Pro Gly Val Asp Tyr Thr Ile Thr Val Tyr Ala Val Thr Gly Arg Gly
            245             250             255

Asp Ser Pro Ala Ser Ser Lys Pro Ile Ser Ile Asn Tyr Arg Thr Glu
            260             265             270

Ile Asp Lys Pro Ser Met Asn Val Ser Pro Pro Arg Arg Ala Arg Val
        275             280             285

Thr Asp Ala Thr Glu Thr Thr Ile Thr Ile Ser Trp Arg Thr Lys Thr
    290             295             300

Glu Thr Ile Thr Gly Phe Gln Val Asp Ala Val Pro Ala Asn Gly Gln
305             310             315             320
```

```
Thr Pro Ile Gln Arg Thr Ile Lys Pro Asp Val Arg Ser Tyr Thr Ile
            325                 330                 335

Thr Gly Leu Gln Pro Gly Thr Asp Tyr Lys Ile Tyr Leu Tyr Thr Leu
            340                 345                 350

Asn Asp Asn Ala Arg Ser Ser Pro Val Val Ile Asp Ala Ser Thr
            355                 360                 365
```

```
<210>  17
<211>  368
<212>  PRT
<213>  Artificial Sequence

<220>
<221>  source
<223>  /note="Description of artificial sequence: fibronectin fragment named
CHV-90"

<400>  17
```

```
Pro Thr Asp Leu Arg Phe Thr Asn Ile Gly Pro Asp Thr Met Arg Val
1               5                   10                  15

Thr Trp Ala Pro Pro Pro Ser Ile Asp Leu Thr Asn Phe Leu Val Arg
            20                  25                  30

Tyr Ser Pro Val Lys Asn Glu Glu Asp Val Ala Glu Leu Ser Ile Ser
            35                  40                  45

Pro Ser Asp Asn Ala Val Val Leu Thr Asn Leu Leu Pro Gly Thr Glu
            50                  55                  60

Tyr Val Val Ser Val Ser Ser Val Tyr Glu Gln His Glu Ser Thr Pro
65                  70                  75                  80

Leu Arg Gly Arg Gln Lys Thr Gly Leu Asp Ser Pro Thr Gly Ile Asp
            85                  90                  95

Phe Ser Asp Ile Thr Ala Asn Ser Phe Thr Val His Trp Ile Ala Pro
            100                 105                 110

Arg Ala Thr Ile Thr Gly Tyr Arg Ile Arg His His Pro Glu His Phe
            115                 120                 125

Ser Gly Arg Pro Arg Glu Asp Arg Val Pro His Ser Arg Asn Ser Ile
            130                 135                 140

Thr Leu Thr Asn Leu Thr Pro Gly Thr Glu Tyr Val Val Ser Ile Val
145                 150                 155                 160
```

Ala Leu Asn Gly Arg Glu Glu Ser Pro Leu Leu Ile Gly Gln Gln Ser
165                170                175

Thr Val Ser Asp Val Pro Arg Asp Leu Glu Val Val Ala Ala Thr Pro
180                185                190

Thr Ser Leu Leu Ile Ser Trp Asp Ala Pro Ala Val Thr Val Arg Tyr
195                200                205

Tyr Arg Ile Thr Tyr Gly Glu Thr Gly Gly Asn Ser Pro Val Gln Glu
210                215                220

Phe Thr Val Pro Gly Ser Lys Ser Thr Ala Thr Ile Ser Gly Leu Lys
225                230                235                240

Pro Gly Val Asp Tyr Thr Ile Thr Val Tyr Ala Val Thr Gly Arg Gly
245                250                255

Asp Ser Pro Ala Ser Ser Lys Pro Ile Ser Ile Asn Tyr Arg Thr Glu
260                265                270

Ile Asp Lys Pro Ser Met Ala Ile Asp Ala Pro Ser Asn Leu Arg Phe
275                280                285

Leu Ala Thr Thr Pro Asn Ser Leu Leu Val Ser Trp Gln Pro Pro Arg
290                295                300

Ala Arg Ile Thr Gly Tyr Ile Ile Lys Tyr Glu Lys Pro Gly Ser Pro
305                310                315                320

Pro Arg Glu Val Val Pro Arg Pro Arg Pro Gly Val Thr Glu Ala Thr
325                330                335

Ile Thr Gly Leu Glu Pro Gly Thr Glu Tyr Thr Ile Tyr Val Ile Ala
340                345                350

Leu Lys Asn Asn Gln Lys Ser Glu Pro Leu Ile Gly Arg Lys Lys Thr
355                360                365

<210> 18
<211> 370
<212> PRT
<213> Artificial Sequence

<220>
<221> source
<223> /note="Description of artificial sequence: fibronectin fragment named CHV-92"

<400> 18

```
Pro Thr Asp Leu Arg Phe Thr Asn Ile Gly Pro Asp Thr Met Arg Val
1               5                   10                  15

Thr Trp Ala Pro Pro Pro Ser Ile Asp Leu Thr Asn Phe Leu Val Arg
            20              25                  30

Tyr Ser Pro Val Lys Asn Glu Glu Asp Val Ala Glu Leu Ser Ile Ser
        35              40                  45

Pro Ser Asp Asn Ala Val Val Leu Thr Asn Leu Leu Pro Gly Thr Glu
    50              55                  60

Tyr Val Val Ser Val Ser Ser Val Tyr Glu Gln His Glu Ser Thr Pro
65              70                  75                  80

Leu Arg Gly Arg Gln Lys Thr Gly Leu Asp Ser Pro Thr Gly Ile Asp
                85                  90                  95

Phe Ser Asp Ile Thr Ala Asn Ser Phe Thr Val His Trp Ile Ala Pro
            100                 105                 110

Arg Ala Thr Ile Thr Gly Tyr Arg Ile Arg His His Pro Glu His Phe
        115                 120                 125

Ser Gly Arg Pro Arg Glu Asp Arg Val Pro His Ser Arg Asn Ser Ile
    130                 135                 140

Thr Leu Thr Asn Leu Thr Pro Gly Thr Glu Tyr Val Val Ser Ile Val
145                 150                 155                 160

Ala Leu Asn Gly Arg Glu Glu Ser Pro Leu Leu Ile Gly Gln Gln Ser
                165                 170                 175

Thr Val Ser Asp Val Pro Arg Asp Leu Glu Val Val Ala Ala Thr Pro
            180                 185                 190

Thr Ser Leu Leu Ile Ser Trp Asp Ala Pro Ala Val Thr Val Arg Tyr
        195                 200                 205

Tyr Arg Ile Thr Tyr Gly Glu Thr Gly Gly Asn Ser Pro Val Gln Glu
    210                 215                 220

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
            245                 250                 255
```

Asp Ser Pro Ala Ser Ser Lys Pro Ile Ser Ile Asn Tyr Arg Thr Glu
        260             265             270

Ile Asp Lys Pro Ser Met Ala Ile Pro Ala Pro Thr Asp Leu Lys Phe
        275             280             285

Thr Gln Val Thr Pro Thr Ser Leu Ser Ala Gln Trp Thr Pro Pro Asn
        290             295             300

Val Gln Leu Thr Gly Tyr Arg Val Arg Val Thr Pro Lys Glu Lys Thr
305             310             315             320

Gly Pro Met Lys Glu Ile Asn Leu Ala Pro Asp Ser Ser Ser Val Val
        325             330             335

Val Ser Gly Leu Met Val Ala Thr Lys Tyr Glu Val Ser Val Tyr Ala
        340             345             350

Leu Lys Asp Thr Leu Thr Ser Arg Pro Ala Gln Gly Val Val Thr Thr
        355             360             365

Leu Glu
    370


<210> 19
<211> 457
<212> PRT
<213> Artificial Sequence

<220>
<221> source
<223> /note="Description of artificial sequence: fibronectin fragment named
CHV-179"

<400> 19

Pro Thr Asp Leu Arg Phe Thr Asn Ile Gly Pro Asp Thr Met Arg Val
1               5               10              15

Thr Trp Ala Pro Pro Pro Ser Ile Asp Leu Thr Asn Phe Leu Val Arg
        20              25              30

Tyr Ser Pro Val Lys Asn Glu Glu Asp Val Ala Glu Leu Ser Ile Ser
        35              40              45

Pro Ser Asp Asn Ala Val Val Leu Thr Asn Leu Leu Pro Gly Thr Glu
        50              55              60

Tyr Val Val Ser Val Ser Ser Val Tyr Glu Gln His Glu Ser Thr Pro
65              70              75              80

Leu Arg Gly Arg Gln Lys Thr Gly Leu Asp Ser Pro Thr Gly Ile Asp
                85                  90                  95

Phe Ser Asp Ile Thr Ala Asn Ser Phe Thr Val His Trp Ile Ala Pro
            100                 105                 110

Arg Ala Thr Ile Thr Gly Tyr Arg Ile Arg His His Pro Glu His Phe
            115                 120                 125

Ser Gly Arg Pro Arg Glu Asp Arg Val Pro His Ser Arg Asn Ser Ile
        130                 135                 140

Thr Leu Thr Asn Leu Thr Pro Gly Thr Glu Tyr Val Val Ser Ile Val
145                 150                 155                 160

Ala Leu Asn Gly Arg Glu Glu Ser Pro Leu Leu Ile Gly Gln Gln Ser
                165                 170                 175

Thr Val Ser Asp Val Pro Arg Asp Leu Glu Val Val Ala Ala Thr Pro
            180                 185                 190

Thr Ser Leu Leu Ile Ser Trp Asp Ala Pro Ala Val Thr Val Arg Tyr
            195                 200                 205

Tyr Arg Ile Thr Tyr Gly Glu Thr Gly Gly Asn Ser Pro Val Gln Glu
        210                 215                 220

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
                245                 250                 255

Asp Ser Pro Ala Ser Ser Lys Pro Ile Ser Ile Asn Tyr Arg Thr Glu
            260                 265                 270

Ile Asp Lys Pro Ser Met Asn Val Ser Pro Pro Arg Arg Ala Arg Val
        275                 280                 285

Thr Asp Ala Thr Glu Thr Thr Ile Thr Ile Ser Trp Arg Thr Lys Thr
        290                 295                 300

Glu Thr Ile Thr Gly Phe Gln Val Asp Ala Val Pro Ala Asn Gly Gln
305                 310                 315                 320

Thr Pro Ile Gln Arg Thr Ile Lys Pro Asp Val Arg Ser Tyr Thr Ile
            325                 330                 335

```
Thr Gly Leu Gln Pro Gly Thr Asp Tyr Lys Ile Tyr Leu Tyr Thr Leu
            340             345             350

Asn Asp Asn Ala Arg Ser Ser Pro Val Val Ile Asp Ala Ser Thr Ala
            355             360             365

Ile Asp Ala Pro Ser Asn Leu Arg Phe Leu Ala Thr Thr Pro Asn Ser
        370             375             380

Leu Leu Val Ser Trp Gln Pro Pro Arg Ala Arg Ile Thr Gly Tyr Ile
385             390             395             400

Ile Lys Tyr Glu Lys Pro Gly Ser Pro Pro Arg Glu Val Val Pro Arg
            405             410             415

Pro Arg Pro Gly Val Thr Glu Ala Thr Ile Thr Gly Leu Glu Pro Gly
            420             425             430

Thr Glu Tyr Thr Ile Tyr Val Ile Ala Leu Lys Asn Asn Gln Lys Ser
        435             440             445

Glu Pro Leu Ile Gly Arg Lys Lys Thr
    450             455
```

```
<210>  20
<211>  459
<212>  PRT
<213>  Artificial Sequence

<220>
<221>  source
<223>  /note="Description of artificial sequence: fibronectin fragment named
CHV-181"

<400>  20
```

```
Pro Thr Asp Leu Arg Phe Thr Asn Ile Gly Pro Asp Thr Met Arg Val
1               5               10              15

Thr Trp Ala Pro Pro Pro Ser Ile Asp Leu Thr Asn Phe Leu Val Arg
            20              25              30

Tyr Ser Pro Val Lys Asn Glu Glu Asp Val Ala Glu Leu Ser Ile Ser
        35              40              45

Pro Ser Asp Asn Ala Val Val Leu Thr Asn Leu Leu Pro Gly Thr Glu
    50              55              60

Tyr Val Val Ser Val Ser Ser Val Tyr Glu Gln His Glu Ser Thr Pro
65              70              75              80
```

```
Leu Arg Gly Arg Gln Lys Thr Gly Leu Asp Ser Pro Thr Gly Ile Asp
                  85                90                    95

Phe Ser Asp Ile Thr Ala Asn Ser Phe Thr Val His Trp Ile Ala Pro
                100                105                110

Arg Ala Thr Ile Thr Gly Tyr Arg Ile Arg His His Pro Glu His Phe
        115                120                125

Ser Gly Arg Pro Arg Glu Asp Arg Val Pro His Ser Arg Asn Ser Ile
    130                135                140

Thr Leu Thr Asn Leu Thr Pro Gly Thr Glu Tyr Val Val Ser Ile Val
145                150                155                160

Ala Leu Asn Gly Arg Glu Glu Ser Pro Leu Leu Ile Gly Gln Gln Ser
                165                170                175

Thr Val Ser Asp Val Pro Arg Asp Leu Glu Val Val Ala Ala Thr Pro
            180                185                190

Thr Ser Leu Leu Ile Ser Trp Asp Ala Pro Ala Val Thr Val Arg Tyr
        195                200                205

Tyr Arg Ile Thr Tyr Gly Glu Thr Gly Gly Asn Ser Pro Val Gln Glu
    210                215                220

Phe Thr Val Pro Gly Ser Lys Ser Thr Ala Thr Ile Ser Gly Leu Lys
225                230                235                240

Pro Gly Val Asp Tyr Thr Ile Thr Val Tyr Ala Val Thr Gly Arg Gly
            245                250                255

Asp Ser Pro Ala Ser Ser Lys Pro Ile Ser Ile Asn Tyr Arg Thr Glu
            260                265                270

Ile Asp Lys Pro Ser Met Ala Ile Pro Ala Pro Thr Asp Leu Lys Phe
        275                280                285

Thr Gln Val Thr Pro Thr Ser Leu Ser Ala Gln Trp Thr Pro Pro Asn
    290                295                300

Val Gln Leu Thr Gly Tyr Arg Val Arg Val Thr Pro Lys Glu Lys Thr
305                310                315                320

Gly Pro Met Lys Glu Ile Asn Leu Ala Pro Asp Ser Ser Ser Val Val
            325                330                335
```

```
Val Ser Gly Leu Met Val Ala Thr Lys Tyr Glu Val Ser Val Tyr Ala
        340             345             350

Leu Lys Asp Thr Leu Thr Ser Arg Pro Ala Gln Gly Val Val Thr Thr
        355             360             365

Leu Glu Asn Val Ser Pro Pro Arg Arg Ala Arg Val Thr Asp Ala Thr
        370             375             380

Glu Thr Thr Ile Thr Ile Ser Trp Arg Thr Lys Thr Glu Thr Ile Thr
385             390             395             400

Gly Phe Gln Val Asp Ala Val Pro Ala Asn Gly Gln Thr Pro Ile Gln
            405             410             415

Arg Thr Ile Lys Pro Asp Val Arg Ser Tyr Thr Ile Thr Gly Leu Gln
            420             425             430

Pro Gly Thr Asp Tyr Lys Ile Tyr Leu Tyr Thr Leu Asn Asp Asn Ala
        435             440             445

Arg Ser Ser Pro Val Val Ile Asp Ala Ser Thr
    450             455


<210>   21
<211>   276
<212>   PRT
<213>   Artificial Sequence

<220>
<221>   source
<223>   /note="Description of artificial sequence: fibronectin fragment named
H-275-Cys"

<400>   21

Met Ala Ala Ser Ala Ile Pro Ala Pro Thr Asp Leu Lys Phe Thr Gln
1               5               10              15

Val Thr Pro Thr Ser Leu Ser Ala Gln Trp Thr Pro Pro Asn Val Gln
            20              25              30

Leu Thr Gly Tyr Arg Val Arg Val Thr Pro Lys Glu Lys Thr Gly Pro
        35              40              45

Met Lys Glu Ile Asn Leu Ala Pro Asp Ser Ser Ser Val Val Val Ser
        50              55              60

Gly Leu Met Val Ala Thr Lys Tyr Glu Val Ser Val Tyr Ala Leu Lys
65              70              75              80
```

69

Asp Thr Leu Thr Ser Arg Pro Ala Gln Gly Val Val Thr Thr Leu Glu
                85                      90                  95

Asn Val Ser Pro Pro Arg Arg Ala Arg Val Thr Asp Ala Thr Glu Thr
               100             105             110

Thr Ile Thr Ile Ser Trp Arg Thr Lys Thr Glu Thr Ile Thr Gly Phe
           115             120             125

Gln Val Asp Ala Val Pro Ala Asn Gly Gln Thr Pro Ile Gln Arg Thr
   130             135             140

Ile Lys Pro Asp Val Arg Ser Tyr Thr Ile Thr Gly Leu Gln Pro Gly
145             150             155             160

Thr Asp Tyr Lys Ile Tyr Leu Tyr Thr Leu Asn Asp Asn Ala Arg Ser
               165             170             175

Ser Pro Val Val Ile Asp Ala Ser Thr Ala Ile Asp Ala Pro Ser Asn
               180             185             190

Leu Arg Phe Leu Ala Thr Thr Pro Asn Ser Leu Leu Val Ser Trp Gln
           195             200             205

Pro Pro Arg Ala Arg Ile Thr Gly Tyr Ile Ile Lys Tyr Glu Lys Pro
           210             215             220

Gly Ser Pro Pro Arg Glu Val Val Pro Arg Pro Arg Pro Gly Val Thr
225             230             235             240

Glu Ala Thr Ile Thr Gly Leu Glu Pro Gly Thr Glu Tyr Thr Ile Tyr
               245             250             255

Val Ile Ala Leu Lys Asn Asn Gln Lys Ser Glu Pro Leu Ile Gly Arg
               260             265             270

Lys Lys Thr Cys
        275

<210>    22
<211>    10
<212>    PRT
<213>    Artificial Sequence

<220>
<221>    source
<223>    /note="Description of artificial sequence: epitope peptide derived from melanoma antigen MART-1"

<400>    22

```
Glu Leu Ala Gly Ile Gly Ile Leu Thr Val
1               5                   10
```

<210> 23
<211> 574
<212> PRT
<213> Artificial Sequence

<220>
<221> source
<223> /note="Description of artificial sequence: mouse fibronectin fragment named CH-296"

<400> 23

```
Pro Thr Asp Leu Arg Phe Thr Asn Ile Gly Pro Asp Thr Met Arg Val
1               5                   10                  15


Thr Trp Ala Pro Pro Pro Ser Ile Glu Leu Thr Asn Leu Leu Val Arg
            20              25                  30


Tyr Ser Pro Val Lys Asn Glu Glu Asp Val Ala Glu Leu Ser Ile Ser
        35                  40                  45


Pro Ser Asp Asn Ala Val Val Leu Thr Asn Leu Leu Pro Gly Thr Glu
    50                  55                  60


Tyr Leu Val Ser Val Ser Ser Val Tyr Glu Gln His Glu Ser Ile Pro
65                  70                  75                  80


Leu Arg Gly Arg Gln Lys Thr Gly Leu Asp Ser Pro Thr Gly Phe Asp
                85                  90                  95


Ser Ser Asp Ile Thr Ala Asn Ser Phe Thr Val His Trp Val Ala Pro
            100                 105                 110


Arg Ala Pro Ile Thr Gly Tyr Ile Ile Arg His His Ala Glu His Ser
            115                 120                 125


Val Gly Arg Pro Arg Gln Asp Arg Val Pro Pro Ser Arg Asn Ser Ile
    130                 135                 140


Thr Leu Thr Asn Leu Asn Pro Gly Thr Glu Tyr Val Val Ser Ile Ile
145                 150                 155                 160


Ala Val Asn Gly Arg Glu Glu Ser Pro Pro Leu Ile Gly Gln Gln Ala
                165                 170                 175


Thr Val Ser Asp Ile Pro Arg Asp Leu Glu Val Ile Ala Ser Thr Pro
                180                 185                 190
```

```
Thr Ser Leu Leu Ile Ser Trp Glu Pro Pro Ala Val Ser Val Arg Tyr
        195             200             205

Tyr Arg Ile Thr Tyr Gly Glu Thr Gly Gly Asn Ser Pro Val Gln Glu
        210             215             220

Phe Thr Val Pro Gly Ser Lys Ser Thr Ala Thr Ile Asn Asn Ile Lys
225             230             235             240

Pro Gly Ala Asp Tyr Thr Ile Thr Leu Tyr Ala Val Thr Gly Arg Gly
                245             250             255

Asp Ser Pro Ala Ser Ser Lys Pro Val Ser Ile Asn Tyr Lys Thr Glu
                260             265             270

Ile Asp Lys Pro Ser Met Ala Ile Pro Ala Pro Thr Asn Leu Lys Phe
        275             280             285

Ser Gln Val Thr Pro Thr Ser Phe Thr Ala Gln Trp Ile Ala Pro Ser
        290             295             300

Val Gln Leu Thr Gly Tyr Arg Val Arg Val Asn Pro Lys Glu Lys Thr
305             310             315             320

Gly Pro Met Lys Glu Ile Asn Leu Ser Pro Asp Ser Ser Ser Val Ile
                325             330             335

Val Ser Gly Leu Met Val Ala Thr Lys Tyr Glu Val Ser Val Tyr Ala
                340             345             350

Leu Lys Asp Thr Leu Thr Ser Arg Pro Ala Gln Gly Val Ile Thr Thr
        355             360             365

Leu Glu Asn Val Ser Pro Pro Arg Arg Ala Arg Val Thr Asp Ala Thr
        370             375             380

Glu Thr Thr Ile Thr Ile Ser Trp Arg Thr Lys Thr Glu Thr Ile Thr
385             390             395             400

Gly Phe Gln Val Asp Ala Ile Pro Ala Asn Gly Gln Thr Pro Val Gln
                405             410             415

Arg Ser Ile Ser Pro Asp Val Arg Ser Tyr Thr Ile Thr Gly Leu Gln
                420             425             430

Pro Gly Thr Asp Tyr Lys Ile His Leu Tyr Thr Leu Asn Asp Asn Ala
                435             440             445
```

```
Arg Ser Ser Pro Val Ile Ile Asp Ala Ser Thr Ala Ile Asp Ala Pro
    450                 455             460

Ser Asn Leu Arg Phe Leu Thr Thr Thr Pro Asn Ser Leu Leu Val Ser
465             470             475                 480

Trp Gln Ala Pro Arg Ala Arg Ile Thr Gly Tyr Ile Ile Lys Tyr Glu
            485             490             495

Lys Pro Gly Ser Pro Pro Arg Glu Val Val Pro Arg Pro Arg Pro Gly
            500             505             510

Val Thr Glu Ala Thr Ile Thr Gly Leu Glu Pro Gly Thr Glu Tyr Thr
        515             520             525

Ile Tyr Val Ile Ala Leu Lys Asn Asn Gln Lys Ser Glu Pro Leu Ile
    530             535             540

Gly Arg Lys Lys Thr Asp Glu Leu Pro Gln Leu Val Thr Leu Pro His
545             550             555             560

Pro Asn Leu His Gly Pro Glu Ile Leu Asp Val Pro Ser Thr
            565             570
```

**Description**

TECHNICAL FIELD

The present invention relates to a method for preparing a T cell population, which is useful in the medical field.

BACKGROUND ART

A living body is protected from foreign substances mainly by an immune response, and an immune system has been established by various cells and the soluble factors produced thereby. Among them, leukocytes, especially lymphocytes, play a key role. The lymphocytes are classified in two major types, B lymphocyte (which may be hereinafter referred to as B cell) and T lymphocyte (which may be hereinafter referred to as T cell), both of which specifically recognize an antigen and act on the antigen to protect the living body.

In the periphery, T cells are mostly made up of CD4 T cells having CD (Cluster of Differentiation)4 marker and CD8 T cells having CD8 marker. A majority of the CD4 T cells is referred to as helper T cell (hereinafter referred to as $T_H$), involves assistance in antibody production and inductions of various immune responses, and differentiates into Th1 or Th2 that is different in the kind of a cytokine produced by antigen stimulation. A majority of the CD8 T cells differentiates into cytotoxic T cell ($T_c$: cytotoxic T lymphocyte, also referred to as killer T cell, which may be hereinafter referred to as CTL), exhibiting a cytotoxic activity by antigen stimulation.

For example, in the pathologic condition of cancer, immunotherapy has drawn an interest in recent years, as a fourth therapy following surgery, chemotherapy, and radiotherapy. Since immunotherapy utilizes immunocompetence inherently owned by a human, it is said that a physical burden on a patient caused by immunotherapy is light as compared to that caused by other therapies. As immunotherapy, a therapy including the step of transferring lymphokine-activated cells obtained by expansion of CTLs or peripheral blood lymphocytes induced *ex vivo*, or the like according to various methods, NKT cells, γδT cells, or the like; a dendritic cell-transfer therapy or a peptide vaccine therapy by which an induction of antigen-specific CTLs *in vivo* is expected; Th1 cell therapy; immune gene therapy further including the step of transducing a gene for which various effects can be expected to the above-mentioned cells *ex vivo* and transferring a transduced cell to the body; and the like, have been known.

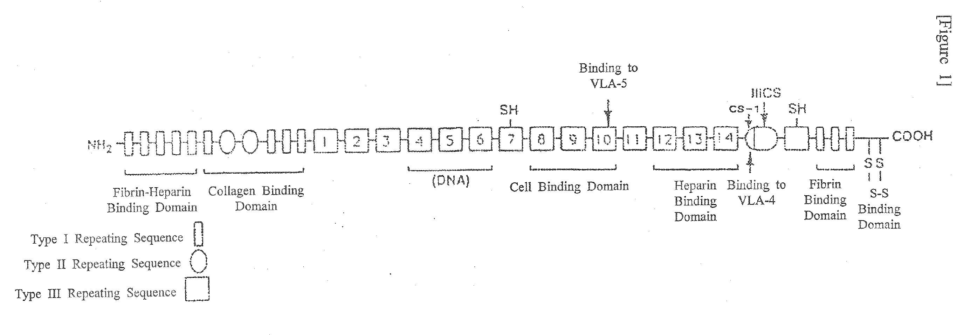

Fibronectin is a gigantic glycoprotein having a molecular weight of 250 thousands, which exists in an animal blood, on the surface of a cultured cell, or in an extracellular of a tissue, and has been known to have various functions. A domain structure thereof is into seven portions (Figure 1 et seq.), wherein three kinds of similar sequences are contained in an amino acid sequence thereof, repetitions of each of these sequences constituting the entire sequence. Three kinds of the similar sequences are referred to as type I, type II and type III. Among them, the type III is constituted by 71 to 96 amino acid residues, wherein an identity of these amino acid residues is 17 to 40%. In fibronectin, there are fourteen type III sequences, among which the 8th, 9th or 10th sequence (each being hereinafter referred to as III-8, III-9 or III-10) is contained in a cell binding domain, and the 12th, 13th or 14th sequence (each being hereinafter referred to as III-12, III-13 or III-14) is contained in a heparin binding domain. In addition, a VLA (very late activation antigen)-5 binding region is contained in III-10, and its core sequence is RGDS. In addition, a region referred to as IIICS exists at a C-terminal side of the heparin binding domain. A region referred to as CS-1 consisting of 25 amino acids and having a binding activity to VLA-4 exists in IIICS (for example, Non-Patent Publications 1 to 3).

Among the immunotherapies, in a therapy including the step of transferring lymphokine-activated cells obtained by expansion from CTLs or peripheral blood lymphocytes induced *ex vivo,* or the like according to actions of IL-2 and an anti-CD3 antibody, regarding disadvantages such as how a cytotoxic activity is maintained when the antigen-specific CTLs induced *ex vivo* are expanded, how lymphocytes can be effectively expanded, and the like, the present inventors have already studied the effects of using fibronectin or a fragment thereof (for example, Patent Publications 1 to 3).

Recent years, it has been reported that as T lymphocytes used in immunotherapy, in a case where naive T cells or central memory T cells in more undifferentiated are administered, a far higher therapeutic effect can be expected in the administration to a living body, rather than a case where effector T cells already terminally differentiated are administered (for example, Non-Patent Publications 4 and 5). Further, also in a dendritic cell-transfer therapy, a peptide vaccine therapy or the like for which induction of antigen-specific CTLs *in vivo* is expected, it is considered naive T cells, the origins from which CTLs can be induced, for example, in the body of a patient with a progressive cancer is few; therefore, a sufficient effect often cannot be expected.

Non-Patent Publication 1: FIBRONECTIN, ACADEMIC PRESS INC., 1-8, authored by Deane F. Momer, published in 1988
Non-Patent Publication 2: Kimizuka F. and eight others, J. Biochem., 1991, 110(2), 284-291
Non-Patent Publication 3: Hanenberg H. and five others, Human Gene Therapy, 1997, 8(18), 2193-2206
Non-Patent Publication 4: Gattinoni L. and nine others, J. Clin. Invest., 2005, 115(6), 1616-1626

Non-Patent Publication 5: Benigni F and ten others, J. Immunol., 2005, 175(2), 739-748
Patent Publication 1: WO 03/016511
Patent Publication 2: WO 03/080817
Patent Publication 3: WO 2005/019450

DISCLOSURE OF THE INVENTION

PROBLEMS TO BE SOLVED BY THE INVENTION

An object of the present invention is to provide a method for preparing a T cell population, which is effective in administration to a living body.

MEANS TO SOLVE THE PROBLEMS

A first invention of the present invention relates to a method for preparing a T cell population, wherein the T cell population expresses CD45RA and expresses at least one selected from the group consisting of CD62L, CCR7, CD27, and CD28, characterized in that the method includes the step of culturing a cell population containing T cells, in the presence of fibronectin, a fragment thereof or a mixture thereof. In the first invention of the present invention, the total cultured days including the culture step are exemplified by from 4 to 14 days. Also, it is exemplified that the culture in the presence of fibronectin, a fragment thereof or a mixture thereof is carried out at least at the initiation of culture, and further, it is preferable that the culture is carried out at least for one day or more. In addition, in the invention of the present invention, it is exemplified that the culturing step in the presence of fibronectin, a fragment thereof or a mixture thereof is carried out in the presence of a CD3 ligand. Also, the CD3 ligand is exemplified by an anti-CD3 antibody. In the first invention of the present invention, the fibronectin fragment is exemplified by a polypeptide (m) containing at least of the amino acid sequences shown in SEQ ID NOs: 1 to 8 of Sequence Listing, or a polypeptide (n) containing at least one amino acid sequence having substitution, deletion, insertion or addition of one or the plural number of amino acids in any of the above amino acid sequences, wherein the polypeptide (n) has a function equivalent to that of the above polypeptide (m). Also, the fibronectin fragment is exemplified by a polypeptide comprising all of the amino acid shown in SEQ ID NOs: 1 to 3 and 5 to 8 of Sequence Listing. In addition, in the first invention of the present invention, the preparation method further including the step of separating cells that express at least one selected from the group consisting of CD45RA, CD62L, CCR7, CD27, and CD28 is also exemplified. Further, in the first invention of the present invention, the preparation method further including the step of transducing a foreign gene into the cell population is also exemplified. In the preparation method, in the transduction of the foreign gene, retrovirus vector, adenovirus vector, adeno-associated virus vector, lentivirus vector or simian virus vector can be used.

A invention of the present invention relates to a T cell population obtained by the method of the first invention of the present invention, wherein the T cell population expresses CD45RA and expresses at least one selected from the group consisting of CD62L, CCR7, CD27, and CD28.

A third invention of the present invention relates to a medicament containing as an effective ingredient the T cell population obtained by the method of the first invention of the present invention, wherein the T cell population expresses CD45RA and expresses at least one selected from the group consisting of CD62L, CCR7, CD27, and CD28.

A fourth invention of the invention relates to a for treating or preventing a disease, the of administering to a subject an effective amount of the T cell population obtained by the method of the invention of the present invention, wherein the T cell population expresses CD45RA and expresses at least one selected from the group consisting of CD62L, CCR7, CD27, and CD28.

A fifth invention of the present invention relates to use of the T cell population obtained by the method of the first invention of the present invention, wherein the T cell population expresses CD45RA and expresses at least one selected from the group consisting of CD62L, CCR7, CD27, and CD28, in the manufacture of a medicament.

A sixth invention of the present invention relates to a method for preparing a T cell population, characterized in that the method includes the step of stimulating the T cell population obtained by the method of the first invention of the present invention, wherein the T cell population expresses CD45RA and expresses at least one selected from the group consisting of CD62L, CCR7, CD27, and CD28, with at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine.

A seventh invention of the present invention relates to a T cell population obtained by the method of the sixth invention of the present invention.

An eighth invention of the present invention relates to a medicament containing as an effective ingredient the T cell population obtained by the method of the sixth invention of the present invention.

A ninth invention of the present invention relates to a method for treating or preventing a disease, including the step of administering to a subject an effective amount of the T cell population obtained by the method of the sixth invention of the present invention.

A tenth invention of the present invention relates to use of the T cell population obtained by the method of the sixth invention of the present invention in the manufacture of a medicament.

An eleventh invention of the present invention relates to a medicament containing:

(a) a preparation containing as an effective ingredient the T cell population obtained by the method of the first invention of the present invention, wherein the T cell population expresses CD45RA and expresses at least one selected from the group consisting of CD62L, CCR7, CD27, and CD28, and
(b) a preparation containing as an effective ingredient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine,

wherein the preparations are contained in the medicament as two separate preparations simultaneously or separately administered.

A twelfth invention of the invention relates to a method for treating a disease, characterized in that the method includes the following steps (a) and (b) of:

(a) administering to a patient the T cell population obtained by the method of the first invention of the present invention, wherein the T cell population expresses CD45RA and expresses at least one selected from the group consisting of CD62L, CCR7, CD27, and and
(b) administering to a patient at one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine.

EFFECTS OF THE INVENTION

According to the preparation method of the present invention, there is provided a T cell population which expresses CD45RA and expresses at least one selected from the group consisting of CD62L, CCR7, CD27, and CD28. A cell population obtained by the preparation method has a high ratio of T cells which express CD45RA and express at least one selected from the group consisting of CD62L, CCR7, CD27, and CD28, and is highly useful in a treatment of a disease with cell therapy.

BEST MODE FOR CARRYING OUT THE INVENTION

The present invention has been completed by the findings that, by including the culturing step in the presence of fibronectin, a fragment thereof or a mixture thereof (which may be hereinafter referred to as the effective ingredient in the present invention), there is obtained a cell population that contains in a high ratio T cells which express CD45RA and express at least one selected from the group consisting of CD62L, CCR7, CD27, and CD28.

the T cell which expresses CD45RA and expresses at least one selected from the group consisting of CD62L, CCR7, CD27, and CD28 as used herein means a T cell population that contains in a ratio T cells which express CD45RA and express at least one selected from the group consisting of CD62L, CCR7, CD27, and CD28. Also, a high ratio herein means that, when the culture is carried out in the same conditions except for the presence or absence of the effective ingredient in the present invention, a ratio of the T cells which express CD45RA and express at least one selected from the group consisting of CD62L, CCR7, CD27, and CD28 in a T cell population obtained by a culture in the presence of the effective ingredient in the present invention is high as compared to that in the case of the absence of the effective ingredient in the present invention. It is preferable that the T cell population contains the above-mentioned T cells in higher ratio preferably by 5% or more, and more preferably by 10% or more, as compared to that in the case of the absence of the effective ingredient in the present invention. The ratio of the T cells which express CD45RA and express at least one selected from the group consisting of CD62L, CCR7, CD27, and CD28, in the resulting cell population, varies depending on various kinds of environmental factors, for example, individual differences and physical conditions of a person who provides cells used in the preparation of T cells such as peripheral blood mononuclear cells (PBMCs). Therefore, it is impossible to unconditionally define the above-mentioned "high ratio" by a numerical value. Also, the T cell population obtained by the preparation method of the present invention means a population containing T cells, and cells other than T cells, for example, other lymphocytes such as NK cells and a hemocyte component other than lymphocytes may be contained.

The invention will be concretely explained hereinbelow.

(1) Fibronectin and Fragment Thereof Used in the Present Invention

The fibronectin and a fragment thereof described herein may be either those obtained from nature, or those artificially synthesized. The fibronectin and a fragment thereof can be prepared in a substantially pure form from a substance of natural origin, on the basis of the disclosure, for example, of Ruoslahti E., et al. [J. Biol. Chem., 256(14), 7277-7281 (1981)]. Here, the term "substantially pure fibronectin or fibronectin fragment" described herein means that these fibronectin and fibronectin fragment do not essentially contain other proteins existing together with fibronectin in nature. Each of the above-mentioned fibronectin and a fragment thereof can be used alone or in admixture of plural kinds in the present invention.

Incidentally, it is known that there are a large number of splicing variants of fibronectin. As the used in the invention, any variant can be so long as the desired effects of the present invention are For example, in the of fibronectin derived from plasma, it is that a region referred to as ED-B present in upstream of a cell binding and a region referred to as ED-A present the cell binding domain the heparin binding domain are deleted. Such derived from plasma can also be used in the invention.

The useful information on the fibronectin which can be used in the present invention and the preparation of the fragments can be obtained from Kimiduka F., et al. [J. Biochem., 110, 284-291 (1991)], Kornbrihtt A. R., et al. [EMBO J., 4(7), 1755-1759 (1985)], Sekiguchi K., et al. [Biochemistry, 25(17), 4936-4941 (1986)], and the like. In addition, the nucleic acid sequence encoding fibronectin or the amino acid sequence of fibronectin is disclosed in GenBank Accession No. NM_002026 and NP_002017.

In the present invention, the fibronectin fragment is exemplified by, for example, a polypeptide (m) containing at least one amino acid sequence containing any of the of III-8 (amino acid sequence shown in SEQ ID NO: 1 of Sequence Listing), III-9 (amino acid sequence shown in SEQ ID NO: 2 of Sequence Listing), III-10 (amino acid sequence shown in SEQ ID NO: 3 of Sequence Listing), III-11 (amino acid sequence shown in SEQ ID NO: 4 of Sequence Listing), III-12 (amino acid sequence shown in SEQ ID NO: 5 of Sequence Listing), III-13 (amino acid sequence shown in SEQ ID NO: 6 of Sequence Listing), III-14 (amino acid sequence shown in SEQ ID NO: 7 of Sequence Listing), and CS-1 (amino acid sequence shown in SEQ ID NO: 8 of Sequence Listing) (see Figure 1), and a polypeptide (n) containing at least one amino acid sequence having substitution, deletion, insertion or addition of one or a plural number of amino acids in any of the amino acid sequences described above, wherein the polypeptide (n) has a function equivalent to of the above-mentioned polypeptide (m). The length of the fragment is, for example, in terms of the number of amino acids, preferably from 20 to 1000, and preferably from 100 to 800. Here, the plural number herein is a concept that includes the several number, and is preferably from 2 to 12, more preferably from 2 to 10, and further more preferably from 2 to 8, which is hereinafter referred to the same.

In addition, as the fragment, a fragment having a cell adhesion activity and/or a heparin binding activity can be preferably used. The cell adhesion activity can be evaluated by assaying binding of the fragment (its cell binding domain) used in the present invention to a cell using a known method. For example, the method as mentioned above includes a method of Williams D. A., et al. [Nature, 352, 438-441 (1991)]. The method is a method of determining the binding of a cell to a fragment immobilized to a culture plate. In addition, the heparin binding activity can be evaluated by assaying binding of the fragment (its heparin binding domain) used in the invention to heparin using a known method. For example, the binding of the fragment to heparin can be evaluated in the same manner by heparin, for example, a labeled heparin in place of the cell in the above-mentioned method of Williams D. A., et al.

Further, the fibronectin fragment is exemplified by a polypeptide selected from the group consisting of C-274 (amino acid shown in SEQ ID NO: 9 of Sequence Listing), H-271 (amino acid sequence shown in SEQ ID NO: 10 of Sequence Listing), H-296 (amino acid sequence shown in SEQ ID NO: 11 of Sequence Listing), CH-271 (amino acid sequence shown in SEQ ID NO: 12 of Sequence Listing), CH-296 (amino acid shown in SEQ ID NO: 13 of Sequence Listing), C-CS1 (amino acid sequence shown in SEQ ID NO: 14 of Sequence Listing), and CH-296Na (amino acid shown in SE ID NO: 15 of Sequence Listing).

Each of the above-mentioned fragments CH-271, CH-296, CH-296Na, C-274 and C-CS1 is a polypeptide having a cell binding domain with a binding activity to VLA-5. Also, C-CS1, H-296, CH-296 and CH-296Na are polypeptides having CS-1 with a binding activity to VLA-4. Further, H-271, H-296, CH-271, CH-296 and CH-296Na are polypeptides having a heparin binding domain. Here, CH-296Na is a polypeptide containing a region from the cell binding domain to CS-1 of fibronectin derived from plasma.

In the present invention, a fragment in which each of the above domains is modified can also be used. The heparin binding domain of the fibronectin is constituted by three type III sequences (III-12, III-13 and III-14). A fragment containing a heparin binding domain having deletion of one or two of the above type III sequences can also be used in the present invention. For example, the fragments may be exemplified by CHV-89 (amino acid sequence shown in SEQ ID NO: 16 of Sequence Listing), CHV-90 (amino acid sequence shown in SEQ ID NO: 17 of Sequence Listing) and CHV-92 (amino acid sequence shown in SEQ ID NO: 18 of Sequence Listing), which are fragments in which a cell binding site of the fibronectin (VLA-5 binding domain: Pro1239 to Ser1515) and one of the III type sequences are bound, or CHV-179 (amino acid sequence shown in SEQ ID NO: 19 of Sequence Listing) and CHV-181 (amino acid sequence shown in SEQ ID NO: 20 of Sequence Listing), which are fragments in which the cell binding site of the fibronectin and two of the type III sequences are bound. CHV-89, CHV-90 and CHV-92 contain III-13, III-14 and III-12, respectively, and CHV-179 contains III-13 and III-14, and CHV-181 contains III-12 and III-13, respectively.

In addition, a fragment having addition of an additional amino acid to each of the above-mentioned fragments can be used in the present invention. For example, the fragment can be prepared by adding a desired amino acid to each of the above-mentioned fragments. For example, H-275-Cys (amino acid sequence shown in SEQ ID NO: 21 of Sequence Listing) is a fragment having a heparin binding domain of the fibronectin, and cysteine residue at a C-terrninal.

The fragment used in the present invention may be those containing a polypeptide comprising an amino acid sequence having substitution, deletion, insertion or addition of one or the plural number of amino acids in an amino acid sequence of a polypeptide constituting the fragment containing at least a part of an amino acid sequence of naturally occurring fibronectin exemplified above, wherein the polypeptide has a function equivalent to that of the fragment, so long as the desired effects of the present invention are obtained.

It is preferable that the substitution or the like of the amino acids is carried out to an extent that it can change physicochemical characteristics and the like of a polypeptide within the range that the inherent function of the polypeptide can be maintained. For example, it is preferable that the substitution or the of the amino acids is conservative, the range that the characteristics inherently owned by the polypeptide (for example, hydrophobicity, hydrophilicity, electric charge, pK and the like) are not substantially changed. For example, it is preferable that the substitution of the amino acids is substitutions within of the of: 1. glycine, alanine; 2. valine, isoleucine, leucine; 3. aspartic acid, glutamic acid, asparagine, glutamine; 4. serine, threonine; 5. lysine, arginine; 6. phenylalanine, tyrosine, and that deletion, addition or insertion of amino acids is deletion, addition or insertion in the amino acids having characteristics similar to the characteristics of the surroundings of the subject site in the polypeptide within the range that the characteristics of the surroundings of the subject site are not substantially changed.

Here, when the fragment used in the present invention has been obtained by genetic engineering technique, in a case where, for example, the polypeptide is prepared using *Escherichia coli* or the like as a host, methionine at an N-terminal is sometimes deleted by the effect of methionine or the like, derived from *Escherichia coli,* and the polypeptide as mentioned above can be used in the present invention. In words, a polypeptide having deletion of at an N-terminal the polypeptides shown in SEQ ID NOs: 15 and 21 of Sequence Listing can be also preferably used in the present invention.

The substitution or the like of the amino may be those naturally occurring being caused by difference between or or may be those artificially induced. Artificial induction may be carried out by a known and is not particularly limited. The artificial induction may be out by for a given nucleic acid having substitution, deletion, addition or insertion of one or the plural number of nucleotides in the nucleic acid encoding the above-mentioned region and the given fragment derived from naturally occurring fibronectin, in accordance with a known method, and using the nucleic acid, whereby a polypeptide containing an amino acid sequence having substitution or the like in the amino acid sequence of the polypeptide constituting the fragments and the like, having a function equivalent to that of the above-mentioned region and the given fragment derived from naturally occurring fibronectin can be prepared.

In addition, the phrase "having a function equivalent" herein refers to that a proportion of the T cells which express CD45RA and express at least one selected from the group consisting of CD62L, CCR7, CD27, and CD28 in a T cell population obtained by using polypeptide is higher than that in a T cell population obtained in the absence of the polypeptide, which is a comparative control. The above-mentioned action can be appropriately confirmed in accordance with the method and the like described in Examples 1, 2, and 6 set forth below. In addition, as the fragment comprising a polypeptide having the substitution or the like of the amino acids, a fragment having a cell adhesion activity and/or a heparin binding activity is preferred, and a fragment having CS-1 domain is also preferred. The cell adhesion activity and the heparin binding activity can be evaluated in accordance with the above-mentioned methods for determining those activities.

As the fragment comprising a polypeptide having the substitution or the like of the amino acids, for example, a fragment having one or more amino acids inserted as a linker between two different domains can also be used in the present invention.

Incidentally, as the fibronectin, similarly to the above-mentioned fragment, there can be used in the present invention a polypeptide having an amino acid sequence having substitution, deletion, insertion or addition of one or the plural number of amino acids in an amino acid sequence of the polypeptide of the fibronectin, wherein the ratio of the T cells which express CD45RA and express at least one selected from the group consisting of CD62L, CCR7, CD27, and CD28 in a T cell population obtained by using the polypeptide is higher than that in a T cell population obtained in the absence of the polypeptide, which is a comparative control.

In addition, as the fibronectin or the fragment thereof used in the present invention, so long as the desired effects of the present invention are obtained, there can be used a polypeptide having a homology of 50% or more, preferably a polypeptide having a homology of 70% or more, more preferably a polypeptide having a homology of 90% or more, and further more preferably a peptide having a homology of 95% or more, to an amino acid sequence of a polypeptide constituting the fibronectin or the fragment thereof, wherein the polypeptide constituting the fibronectin or the fragment thereof has a function equivalent to that of the naturally occurring fibronectin or the fragment containing at least a part of an amino acid sequence thereof exemplified above. Incidentally, for example, DNASIS Pro Ver.2.6 (manufactured by TAKARA BIO INC.) can be used for the calculation of the homology.

Incidentally, the fibronectin fragment most preferably used in the present invention includes a polypeptide containing in the acid sequence all of III-8 (amino acid sequence shown in SEQ ID NO: 1 of Sequence Listing), III-9 (amino acid sequence shown in SEQ ID NO: 2 of Sequence Listing), III-10 (amino acid sequence shown in SEQ ID NO: 3 of Sequence Listing), III-12 (amino acid sequence shown in SEQ ID NO: 5 of Sequence Listing), III-13 (amino acid sequence shown in SEQ ID NO: 6 of Sequence Listing), III-14 (amino acid sequence shown in SEQ ID NO: 7 of Sequence Listing), and CS-1 (amino acid sequence shown in SEQ ID NO: 8 of Sequence Listing), in other words, a polypeptide containing a heparin binding domain, a cell binding domain, and CS-1, and more preferably, the fibronectin fragment includes the above-mentioned CH-296, or a polypeptide comprising an amino acid sequence having substitution, deletion, insertion or addition of one or the plural number of amino acids in an amino acid sequence of the polypeptide constituting the fragment, having a function equivalent to that of the CH-296. Other fibronectin polypeptide preferably used in the present invention includes H-296, CH-271, H-271, and C-CS1, as shown in Example 18, or polypeptides comprising an amino acid sequence having substitution, deletion, insertion or addition of one or the plural number of amino acids in an amino acid sequence of the polypeptide constituting the fragment, having a function equivalent to that of these polypeptides.

The fibronectin fragment described herein can be also prepared as a recombinant fibronectin fragment from a genetic recombinant on the basis of, for example, the description of the specification of U.S. Patent No. 5,198,423. For example, each of the above-mentioned fragments of H-271 (SEQ ID NO: 10), H-296 (SEQ ID NO: 11), CH-271 (SEQ ID NO: 12) and CH-296 (SEQ ID NO: 13) and a of obtaining these fragments are described in in the specification of this patent. In addition, CH-296Na (SEQ ID NO: 15) and the preparation method thereof are described in WO 2005/019450. In addition, the above-mentioned C-274 (SEQ ID NO: 9) fragment can be obtained in accordance with the method described in the specification of U.S. Patent No. 5,102,988. Further, the C-CS1 (SEQ ID NO: 14) fragment can be obtained in accordance with the method described in the specification of Japanese Patent Gazette No. 3104178. Each of the above-mentioned fragment of CHV-89 (SEQ ID NO: 16), CHV-90 (SE ID NO: 17) or CHV-179 (SEQ ID NO: 19) can be obtained in accordance with the method described in the specification of Japanese Patent Gazette No. 2729712. In addition, the CHV-181 (SEQ ID NO: 20) fragment can be obtained in accordance with the method described in WO 97/18318. The CHV-92 (SEQ ID NO: 18) fragment can be obtained by genetic engineering technique using a plasmid constructed in a conventional manner on the basis of the plasmid described in the literatures by referring to the specification of Japanese Patent Gazette No. 2729712 and WO 97/18318.

These fragments or fragments which can be derived from these fragments in a conventional can be prepared by using microorganisms to the International Patent Depositary, National of Advanced Industrial Science and Technology, Tsukuba Central 6, 1-1, Higashi 1-chome, Tsukuba-shi, Ibaraki-ken, Japan (Zip code 305-8566) under the following accession numbers, or also prepared by modifying a plasmid carried in each microorganism in accordance with a known method.

FERM BP-2264 (*Escherichia coli* carrying a plasmid encoding H-271, Date of Deposit: January 30, 1989);
FERM BP-2800 *(Escherichia coli* carrying a plasmid encoding CH-296, Date of Deposit: May 12, 1989);
FERM BP-2799 *(Escherichia coli* carrying a encoding CH-271, Date of Deposit: May 12, 1959);
FERM BP-7420 *(Escherichia coli* carrying a plasmid encoding H-296, Date of Deposit: May 12, 1989);
FERM BP-1915 *(Escherichia coli* carrying a plasmid encoding C-274, Date of Deposit: June 17, 1988);
FERM BP-5723 *(Escherichia coli* carrying a plasmid encoding C-CS1, Date of Deposit: March 5, 1990);
FERM BP-10073 (plasmid encoding CH-296Na, Date of Deposit: July 23, 2004);
FERM P-12182 (*Escherichia coli* carrying a plasmid encoding CHV-89, Date of Deposit: April 8, 1991); and
FERM P-12183 (*Escherichia coli* carrying a plasmid encoding CHV-179, Date of Deposit: April 8, 1991).

Since the fibronectin is a gigantic glycoprotein, it is not necessarily easy industrially and in the manufacture of the medicament to prepare and use a naturally occurring protein. In addition, since the fibronectin is a multifunctional protein, there may be considered some disadvantages caused by a region different from the region exhibiting the effect by the method of the present invention depending on the conditions of its use. For these reasons, it is preferable that a fibronectin fragment is preferably used, and more preferably a recombinant fibronectin fragment obtained as described above, in the present invention, from the viewpoint of availability, easy handling, and safety. In addition, it is preferable to use the above-mentioned fibronectin fragment, from the viewpoint of realizing a expansion fold. In addition, the molecular weight of the fibronectin fragment used in the present invention is not particularly limited, and is preferably from 1 to 200 kD, more preferably from 5 to 190 kD, and even preferably from 10 to 180 kD. The molecular weight can be determined, for example, by SDS-polyacrylamide gel electrophoresis.

Here, in the amino acid sequence of the polypeptide constituting the fibronectin fragment of the present invention, the part of amino acid sequence other the amino acid sequence of the polypeptide constituting a naturally occurring fibronectin fragment is arbitrary and is not particularly limited, so long as the exhibition of the desired effects of the present invention is not inhibited.

(2) Method for Preparing T Cell Population

The method for preparing a T cell population of the present invention will be concretely explained hereinbelow. The present invention is a method for preparing a cell population that contains in a high ratio T cells which express CD45RA and express at least one selected from the group consisting of CD62L, CCR7, CD27, and CD28, and preferably T cells which express CD45RA and express at least one selected from the group consisting of CD62L and CCR7. The method of the present invention is characterized in that the method includes the step of culturing a cell population containing T cells, in the presence of the above-mentioned fibronectin, a fragment thereof or a mixture thereof.

CD45RA, CD62L, CCR7, CD27, and CD28 are all cell surface antigen markers of lymphocytes, and known to be expressed in undifferentiated cells such as naive T cells. In other words, the T cells which express CD45RA and express at least one selected from the group consisting of CD62L, CCR7, CD27, and CD28, contained in a high ratio in a cell population obtained by the preparation method of the present invention can be classified into undifferentiated cells before differentiation into memory T cells, i.e., naive T-like cells, in view of a phenotype of cell surface antigen marker. As described in the above-mentioned Non-Patent Publications 4 and 5, there are described that naive T cells are high in survival rate, cell proliferation effect, effect of accumulation to a tumor, production rate of tumor-specific effector cells, in a living body, when the naive T cells are administered to the living body, and that the naive T cells are useful in the field of cell therapy.

Further, as shown in Example 3 set forth below, the T cell population obtained by the preparation method of the invention is turned into activated T cells producing a large amount of IL-2, by stimulating the T cell population by an anti-CD3 antibody or an anti-CD28 antibody,. Also, as shown in Example 4 set forth below, since the T cell population obtained by the preparation method of the present invention shows chemotaxis in response to CCL21 that is a chemokine, and the T cell population also has an ability to migrate to the lymph node. In addition, as shown in Examples 5 to 8 set forth below, antigen stimulation is applied to the T cell population, whereby CTLs antigen-specific cytotoxic activities are induced. Further, as shown in Example 9 set forth below, since the T cell population shows a high viability in the presence of a small amount of IL-2 or absence thereof, as compared to a T cell population prepared in the absence of fibronectin, a fragment thereof or a mixture thereof, it is expected that the T cell population also shows a high viability in a living body. In fact, Example 11 set forth below shows that in a case where the T cell population obtained by the preparation method of the invention is administered to a NOD/scid mouse, as compared to a case that of the T cell population prepared in the absence of fibronectin, a fragment thereof or a mixture thereof, the T cells have a high rate of engraftment to the spleen and also have a high percentage of viability. Further, Example 11 also shows that, since the administered T cell population also causes a GVHD reaction, cells having high cytotoxic activities are induced. In view of the above, the T cell population obtained by the preparation method of the present invention has not only the phenotype of cell surface antigen marker, but also a function suitable for use in the field of cell therapy, owned by naive T cells.

However, surprisingly, as shown in item (4) of Example 7, item (2) of Example 8, item (4) of Example 15, and item (2) of Example 16, CTLs induced from a T cell population obtained by further subjecting the T cell population prepared according to the above-mentioned method to the step of separating a cell population that expresses at least one selected from the group consisting of CD45RA, CD62L, CCR7, CD27, and CD28 mentioned later have high cytotoxic activities and also have high abilities to specific antigen, as compared to those of naive T cells obtained from PBMCs and T cells showing the similar phenotype obtained in the absence of the effective ingredient in the invention. In other words, the T cell population obtained by the above-mentioned separation procedures has a significantly high cytotoxic activity upon differentiation into CTLs, as compared to those of normal naive T cells. In this regard, the above-mentioned T cell population is a cell population containing novel naive T-like cells having more effective features, which are different from the naive T cells.

High therapeutic effects of the T cell population obtained by the preparation method of the present invention as mentioned above can be similarly expected to be exhibited in both CD8$^+$ and CD4$^+$ naive T-like cells. Further, as described in the above-mentioned Patent Publication 2, expansion of T cells is carried out in the presence of fibronectin, a fragment thereof or a mixture thereof, whereby a very high proliferation rate can be realized. Therefore, the T cell population obtained by the method of the present invention is highly suitable for use in the field of cell therapy.

The cell population containing T cells used in the preparation method of the present invention is exemplified by PBMCs, naive T cells, memory T cells, hemopoietic stem cells, umbilical cord blood mononuclear cells, and the like. In addition, so long as cells are hemocytes, the cells can be used in the present invention. Any of these cells which are collected from a living body can be used, or which are obtained via culture *ex vivo,* for example, the T cell population obtained by the method of the present invention, can be directly used or those which has been subjected to frozen storage can be used. In addition, for example, a cell population obtained via various separation procedures from the cells used in the preparation of the above-mentioned T cell population obtained from a living body, for example, any of cell population obtained by separating cells such as PBMCs into CD8$^+$ cells or CD4$^+$ cells, can be also used. Incidentally, in the method for preparing a T cell population of the present invention, a material containing the above-mentioned cells, for example, a blood such as peripheral blood or umbilical cord blood; one obtained by removing components such as erythrocyte and plasma from the blood; a bone marrow fluid; and the like can be used.

It is preferable that the method for preparing a T cell population of the present invention is carried out in total cultured days of preferably from 4 to 14 days. In other words, when the total cultured days are from 4 to 14 days, a ratio of the T cells which express CD45RA and express at least one selected from the group consisting of CD62L, CCR7, CD27, and CD28 in the resulting T cell population is high, thereby making it highly suitable for use in the field of cell therapy. Incidentally, when the total cultured days are less than 4 days, the satisfactory number of cells for use in the general immunotherapy cannot be obtained. In the present invention, the total cultured days are more preferably from 5 to 14 days, and even more preferably from 7 to 14 days.

In the method for preparing a T cell population of the present invention, it is preferable that the culture is carried out in the presence of the effective ingredient in the present invention, particularly preferably at least in an early stage of the entire culture period. It is preferable that the culture in the presence of the effective ingredient in the present invention is carried out more preferably at least at the initiation of the culture. The culture in the presence of the effective ingredient in the present invention may be carried out during the entire period of the culture period, or during any part of the period. In other words, the present invention encompasses those embodiments which include the above-mentioned step in a part of the steps of preparing T cells. It is preferable that the culture in the presence of the effective ingredient in the present invention is carried out for one day or more, more preferably for 3 days or more, and even more preferably for 4 days or more, from the initiation of the culture.

In the present invention, the concentration of fibronectin, a fragment thereof or a thereof during the culture is not particularly limited, and is, for example, preferably from 0.001 to 500 $\mu$g/mL, and particularly preferably from 0.01 to 500 $\mu$g/mL.

In the present invention, it is preferable that the culture in the presence of fibronectin, a fragment thereof or a mixture thereof is carried out in the presence of a CD3 ligand, from the viewpoint of effectively stimulating a TCR-CD3 complex with T cells to proliferate the cells.

In the invention, a CD3 ligand is not particularly limited, so as a substance has a activity to CD3, and is exemplified by, for example, an anti-CD3 antibody, particularly preferably by an anti-CD3 monoclonal antibody, for example, OKT3. The concentration of a CD3 ligand in the medium is not particularly limited. For example, in a case where the anti-CD3 monoclonal antibody is used, the concentration is, for example, preferably from 0.001 to 100 $\mu$g/mL, and particularly preferably from 0.01 to 100 $\mu$g/mL.

In addition, in the present invention, other co-stimulation factor such as a CD28 ligand can be added to introduce co-stimulation as occasion demands. For example, an anti-CD28 antibody, CD80, B7-1, 7-2, and the like are exemplified.

The medium used in the method for preparing a T cell population of the present invention is not particularly limited, and a known medium prepared by mixing components necessary for expanding T cells can be used. For example, a commercially available medium can be appropriately selected to be used. These media may contain cytokines, appropriate proteins, and other components in addition to the inherent constituents. The cytokines are exemplified by, for example, IL-2, IL-7, IL-12, IFN-$\gamma$, and the like, and preferably, a medium containing IL-2 is used. The concentration of IL-2 in the medium is not particularly limited, and is, for example, preferably from 0.01 to $1 \times 10^5$ U/mL, and more preferably from 0.1 to $1 \times 10^4$ U/mL. Also, the appropriate proteins are exemplified by, for example, an anti-IL-4 antibody. Also, besides the above, a lymphocyte-stimulating factor such as lectin can be added. The concentration of the component in the medium is not particularly limited, so long as the desired effects can be obtained.

Furthermore, in the culture, serum and plasma can be also added to the medium. The amounts of serum and plasma added to the medium are not particularly limited, and are exemplified by from 0% by volume to 20% by volume, and the amounts of serum and plasma used can be changed depending on the stage of culture. For example, serum or plasma can be used by stepwise decreasing the concentration thereof. Incidentally, origin of the serum or plasma may be either autologous (meaning that the origin is the same as that of the cell cultured) or nonautologous (meaning that the origin is different from that of the cell cultured). Preferably, autologous serum or plasma can be used, from the viewpoint of safety.

The preparation of a T cell population of the present invention is usually carried out in a medium containing given components in the presence of the above-mentioned effective ingredient in the present invention. The number of cells at the initiation of culture used in the present invention is not particularly limited, and is exemplified by, for example, preferably from 1 cell/mL to $1 \times 10^8$ cells/mL, more preferably from 1 cell/mL to $5 \times 10^7$ cells/mL, even more preferably from 1 cell/mL to $2 \times 10^7$ cells/mL. In addition, the culture conditions are not particularly limited, and usual conditions used for cell culture can be employed. For example, cells can be cultured under the conditions of 37˚C in the presence of 5% $CO_2$ and the like. In addition, the medium can be diluted by adding a fresh medium to the cell culture solution, the medium can be exchanged, or the cell culture equipment can be exchanged, at appropriate intervals.

The cell culture equipment used in the method for preparing a T cell population of the present invention is not particularly limited, for example, a petri dish, a flask, a bag, a large culture bath, a bioreactor and the like can be used. Here, as a bag, a $CO_2$ gas-permeable bag for cell culture can be used. In addition, in a case where T cells are industrially mass-produced, a large culture tank can be used. Furthermore, the culture can be carried out in either an open system or a system. Preferably, the culture is carried out in a closed system, from the viewpoint of safety of the resulting T cells.

Incidentally, the effective ingredient in the present invention, a CD3 ligand, other co-stimulation factor, and appropriate proteins, cytokines, and other components, contained in the above-mentioned medium, may be dissolved to be co-present, or the above components may be immobilized to an appropriate solid phase, for example, a cell culture equipment (including any of those of an open system and a closed system), such as a petri dish, a or a bag, or to a cell culture carrier such as beads, a membrane or a slide glass, and used. The materials for those solid phases are not particularly limited, so long as the materials can be used for cell culture. When the components are immobilized to, for example, the above-mentioned equipment, it is preferable to immobilize a given amount of each component based on the amount of the medium to be placed in the equipment so that, upon placing the medium in the equipment, the medium is contained in a proportion similar to that of a desired concentration in the case where the components are dissolved in the medium and used. The amount of the components immobilized is not particularly limited, so long as the desired effects can be obtained. The above-mentioned carrier is used by immersing the carrier in a culture medium in the cell culture equipment during the cell culture. When the above-mentioned components are immobilized to the above-mentioned carrier, it is preferable to immobilize a given amount of each component based on the amount of the medium placed in the equipment so that the medium is contained in a proportion similar to that of a desired concentration upon placing the carrier in the medium, in the case where the components are dissolved in the medium and used. The amount of the components immobilized is not particularly limited, so long as the desired effects can be obtained.

A method for immobilizing the effective ingredient in the present invention, a CD3 ligand, and other co-stimulation factor, to the solid phase is not particularly limited. For example, the above-mentioned substances can be immobilized by contacting them with the solid phase in an appropriate buffer solution.

In addition, regarding the immobilization of the fibronectin fragment to the solid phase, the immobilization can be also carried out in accordance with the methods described in WO97/18318 and WO 00/09168.

Once various components mentioned above or the effective ingredient in the present invention is immobilized to the solid phase, the T cell population can be easily separated from the effective ingredient or the like only by separating the T cell population from the solid phase after the T cell population is obtained by the method of the present invention, so that the contamination of the T cell population with the effective ingredient or the like can be prevented.

Also, the preparation method of the present invention can further include the step of separating a T cell population that expresses at least one selected from the group consisting of CD45RA, CD62L, CCR7, CD27, and CD28, from the T cell population obtained by a culture in the presence of the effective ingredient in the invention. In words, the T cell population obtained by the culture in the presence of the effective ingredient in the present invention as described above contains in a high ratio T cells which express CD45RA and express at least one selected from the group consisting of CD62L, CCR7, CD27, and CD28. Therefore, the procedures of separating cells that express at least one surface antigen marker selected from the group consisting of CD45RA, CD62L, CCR7, CD27, and CD28 are further carried out, and whereby naive T-like cells or a T cell population containing even more naive T-like cells can be more effectively obtained. In this case, T cells to be separated are not particularly limited, and are exemplified by, for example, cells express CD45RA, and are exemplified by, preferably cells that express CD45RA, and express at least one selected from the group consisting of CD62L, CCR7, CD27, and CD28, and more preferably cells that express CD45RA and CD62L, and cells that express CD45RA and CCR7. The separation procedures are not particularly limited, and the separation can be carried out according to a known method, for example, by using a cell sorter, magnetic beads, column, or the like. For example, when cells that express CD45RA and CCR7 are separated, the separation can be carried out as described in item (3) of Example 3 set forth below.

In addition, the T cells prepared by the method of the present invention are cloned, whereby the T cells can be maintained as stable T cells. Also, the T cell population is further cultured according to the method of the present invention or a known method, using the T cell population obtained by the method of the present invention, whereby a T cell population can be newly obtained. Moreover, antigen stimulation or the like is applied according to a known method, for example, in the same manner as in Examples 5 to 8 set forth below, the T cell population obtained by the method of the present invention, whereby antigen-specific CTLs can be prepared.

The T cell population obtained by the method of the present invention contains in a ratio T cells which express CD45RA and express at least one selected from the group consisting of CD62L, CCR7, CD27, and CD28, that are undifferentiated T cells as mentioned above, and the T cell population also contains T cells having cytotoxic activities depending on the kind of cells used in the culture. The cytotoxic activity of the T cell population can be also assessed by a known test *in vitro.* Since the T cell population obtained by the present invention contains in a high ratio undifferentiated naive T-like cells, as mentioned above, the T cell population obtained by the preparation method of the present invention does not necessarily show a high cytotoxic activity in the above assessment system.

Diseases to which the T cell population prepared by the method of the present invention is administered are not particularly limited, and are exemplified by, for example, cancer, leukemia, malignant tumor, hepatitis, and infectious diseases caused by a virus such as influenza or HIV, a bacteria, or a fungus, for example, tuberculosis, MRSA, VRE, and deep-seated mycosis. In addition, when a foreign gene is further transduced thereinto as described below, the effects can be also expected for various genetic diseases. The T cell population prepared by the method of the present invention can also be utilized for donor lymphocyte infusion and the like for the purpose of prevention from an infectious disease after bone marrow transplantation or X-ray irradiation, and remission of recurrent leukemia.

Further, the present invention provides a T cell population obtained by the method for preparing a T cell population of the present invention mentioned above, wherein the T cell population expresses CD45RA and expresses at least one selected from the group consisting of CD62L, CCR7, CD27, and CD28. In addition, the present invention provides a medicament (therapeutic agent) containing the T cell population as an effective ingredient. The above-mentioned therapeutic agent containing the T cell population is suitably used in immunotherapy. In the immunotherapy, the T cells suitable for treating a patient are administered to the patient by, for example, administration method such as intravenous, intra-arterial, subcutaneous, or intraperitoneal by injection or drip. The therapeutic agent is very useful for use in the above-mentioned diseases and donor lymphocyte infusion. The therapeutic agent can be prepared as drops or an injection by, for example, blending the T cell population prepared by the method of the present invention as an effective ingredient with, for example, a known organic or inorganic carrier suitable for parenteral administration, an excipient, a stabilizing agent and the like, according to a method known in the pharmaceutical field. Incidentally, the amount of the T cell population of the present invention contained in the therapeutic agent, the dose of the therapeutic agent, and conditions for the therapeutic agent can be appropriately determined according to the known immunotherapy. For example, the amount of the T cell population of the present invention contained in the medicament is not particularly limited, and, for example, is exemplified by, preferably from $1 \times 10^3$ to $1 \times 10^{11}$ cells/mL, preferably from $1 \times 10^4$ to $1 \times 10^{10}$ cells/mL, and even more preferably from $1 \times 10^5$ to $1 \times 10^9$ cells/mL. Also, the dose of the medicament of the present invention is not particularly limited, and, for example, is exemplified by, preferably from $1 \times 10^6$ to $1 \times 10^{12}$ cells/day, more preferably from $1 \times 10^7$ to $5 \times 10^{11}$ cells/day, and even more preferably from $1 \times 10^8$ to $2 \times 10^{11}$ cells/day, for adult per day. Further, an immunotherapy by the therapeutic agent can be used in combination with a known pharmacotherapy or radiotherapy by administration of a drug, or therapy by surgery.

The method for preparing a T cell population of the present invention can further include the step of transducing a foreign gene into the T cells. In other words, one embodiment of the present invention provides a method for preparing a T cell population, further including the step of transducing a foreign gene into T cells. Here, the term "foreign gene" means a gene which is artificially transduced into T cells into which a gene is to be transduced, and also encompasses a gene derived from the same species as the one from which T cells into which a gene is to be transduced is derived.

By carrying out the method for preparing T cells of the present invention, the ability for proliferation of the cultured T cells is enhanced. By combining the method for preparing T cells of the present invention with the of transducing a gene, increase in the gene-transducing efficiency is expected.

A means for transducing a foreign gene is not particularly limited, and an appropriate method can be selected from known methods for transducing a gene to be used. The step of transducing a gene can be carried out at any given point during the preparation of a T cell population. For example, it is preferable to carry out the step simultaneously with or during the course of the above-mentioned preparation of the T cell population, or after the step, from the viewpoint of working efficiency.

As the above-mentioned method for transducing a gene, any of methods using a viral vector, and methods without using the vector can be employed in the present invention. The details of those methods have been already published in numerous literatures.

The above-mentioned viral vector is not particularly limited, and a known viral vector ordinarily used in the method for transducing a gene, for example, retroviral vector, lentiviral vector, adenoviral vector, adeno-associated viral vector, simian viral vector, vaccinia viral vector, sendai viral vector, or the like is used. Particularly preferably, as the viral vector, retroviral vector, adenoviral vector, adeno-associated viral vector, lentiviral vector, or simian viral vector is used. As the above-mentioned vector, those lacking replication ability so that the viral vector cannot self-replicate in an infected cell are preferable. In addition, upon the gene transduction, a substance which improves gene-transducing efficiency, such as RetroNectin (registered trademark, manufactured by TAKARA BIO INC.) can be also used.

The retroviral vector and lentiviral vector are used for the purpose of gene therapy or the like because they can stably incorporate a foreign gene inserted therein into chromosomal DNA in the cell into which the vectors are to be transduced. Since the vectors have a high infection efficiency to the cells during division and proliferation, the gene transduction is preferably carried out in the preparation step in the present invention.

As the method for transducing a gene without using a viral vector, for example, a method using a carrier such as liposome or ligand-polylysine, calcium phosphate method, electroporation method, particle gun method or the like can be used without limiting the present invention thereto. In this case, there is transduced a foreign gene incorporated into a plasmid DNA, linear DNA, or RNA.

The foreign gene to be transduced into T cells in the present invention is not particularly limited, and any gene which is desired to be transduced into the above-mentioned cells can be selected. As the gene as described above, besides a gene encoding a protein (for example, enzymes, cytokines, receptors, or the like), for example, a gene encoding an antisense nucleic acid, siRNA (small interfering RNA) or a ribozyme can be used. In addition, an appropriate marker gene which allows for selection of cells into which a gene is transduced may be simultaneously transduced.

The above-mentioned foreign gene can be, for example, inserted into a vector, a plasmid or the like, so as to be expressed under the control of an appropriate promoter, and used. In addition, in order to achieve an efficient transcription of a gene, there may exist in vector other regulating elements which cooperate with a promoter or a transcription initiation site, for example, an enhancer sequence or a terminator sequence. In addition, for the purpose of inserting a foreign gene into a chromosome of T cells in which the gene is to be transduced by homologous recombination, for example, a foreign gene may be arranged between flanking sequences comprising nucleotide sequences each having homology to nucleotide sequences located on both sides of the desired target insertion site of the gene in the chromosome. The foreign gene to be transduced may be one that is a naturally occurring or an artificially generated, or may be one in which DNA molecules having different origins from each other are bound by a known such as ligation. Moreover, the foreign gene may be having a sequence in which a mutation is introduced into a naturally occurring sequence depending upon its purpose.

According to the method of the present invention, for example, a gene encoding an enzyme associated with the resistance to a drug used for the treatment of a patient with cancer or the like can be transduced into T cells, thereby giving the T cells a drug resistance. If the T cells as described above are used, immunotherapy and drug therapy can be used in combination, and therefore, higher therapeutic effects can be obtained. The drug resistance gene is exemplified by, for example, a multidrug resistance gene.

On the other hand, conversely to the above-mentioned embodiment, a gene to give sensitivity to a particular drug can be transduced into T cells, thereby giving the T cells sensitivity to the drug. In this case, the T cells after being transplanted to a living body can be removed by administering the drug. The gene for giving sensitivity to a drug is exemplified by, for example, a thymidine kinase gene.

Other genes to be transduced are exemplified by a gene encoding a TCR recognizing a surface antigen of target cells and a gene encoding a chimeric receptor having an antigen-recognizing site of an antibody to the surface antigen of target cells and containing an intracellular region of TCR (CD3 or the like).

The present invention also provides a method for treating or preventing a disease, including the step of administering to a subject an effective amount of the T cell population obtained by the above-mentioned method. The ter "subject" as used herein is not particularly limited, and preferably refers to a patient with the disease described above, to which the T cell population prepared by the method of the present invention is administered.

In addition, the term "effective amount" as used herein is an amount of the above-mentioned T cell population exhibiting a therapeutic or prophylactic effect, in a case where the T cell population is administered to the above-mentioned subject, as compared to a subject to which the T cell population is not administered. The specific effective amount is properly set depending upon its administration form, administration method, purpose of use, and age, weight, symptom or the like of a subject, and is not constant. The T cell population may be preferably administered in the same effective amount as the above-mentioned medicament. The administration method is also not limited. For example, the T cell population may be administered by drip, injection, or the like, in the same manner as in the case of the above-mentioned medicament.

In addition, the present invention also provides use of the above-mentioned T cell population in the manufacture of a medicament. The method for the medicament is carried out in the same manner as that for the above-mentioned medicament. Also, diseases to which the medicament is administered are not particularly limited, are the same as those of the above-mentioned medicament.

In addition, the present invention can prepare an activated T cell population by stimulating the T cell population obtained by the preparation method of the present invention mentioned above, wherein the T cell population expresses CD45RA and expresses at least one selected from the group consisting of CD62L, CCR7, CD27, and CD28, with at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine. Further, the present invention provides the T cell population obtained by the above-mentioned preparation method. The activated T cell population obtained as described above can be used as an effective ingredient of a medicament, as with the T cell population obtained by the above-mentioned preparation method. Here, the term "stimulating with a stimulating factor" is not particularly limited, so long as the T cell population obtained by the preparation method of the present invention mentioned above is activated by a stimulating factor, and is exemplified by, for example, carrying out a culture in the coexistence of the T cell population obtained by the preparation method of the present invention, and a stimulating factor.

The cell capable of presenting an antigen as used herein is not particularly limited, so long as a cell is generally used as an antigen-presenting cell, and is exemplified by, for example, dendritic cell, $\gamma\delta$T cell, monocyte, B cell, T cell, macrophage, fibroblast, Langerhans cell, a cell population containing at least one cell of the above-mentioned cells, and particularly preferably exemplified by dendritic cell, $\gamma\delta$T cell, T cell, B cell, monocyte, macrophage, a cell population containing at least one cell of the above-mentioned cells. In addition, origin of the cell capable of presenting an antigen may be either autologous or nonautologous for a patient to which the cell is administered, and autologous cell is preferably used. Here, the cell capable of presenting an antigen as used herein means a cell that is capable of presenting an antigen, but not presenting the antigen.

As the cell having presented an antigen as used herein, either a cell in which an appropriate antigen has been artificially added to the above-mentioned cell capable of presenting the antigen, or a cell already presenting an antigen upon collection from a living body can be used. The cell having presented an antigen can be also prepared in the same manner as described in item (5) of Example 5 set forth below, and be used. Here, the phrase "cell capable of presenting the antigen" is not encompassed in the meaning of the phrase "cell having presented an antigen."

The antigen as used herein is not particularly limited, so long as the peptide is presented on an antigen-presenting cell, and is recognized by T cells so that the T cells are efficiently activated. For example, the antigen is exemplified by peptide, glycopeptide, tumor cell extract, ultrasonic-treated product of tumor cell and hydrothermally-treated product of tumor cell, virus, bacterium, protein, and the like.

In addition, specific examples of a CD3 ligand and a CD28 ligand are exemplified by a CD3 ligand and a CD28 ligand described above. Here, the T cell population which expresses CD45RA and expresses at least one selected from the group consisting of CD62L, CCR7, CD27, and CD28, obtained by the preparation method of the present invention mentioned above is co-stimulated with an anti-CD3 antibody and an anti-CD28 antibody, as in Example 3 set forth below, whereby an activated lymphocyte population capable of producing a cytokine can be prepared.

The cytokine as used herein is not particularly limited, so long as a cytokine can act on T cells and activate the T cells. For example, the cytokine is exemplified by IL-2, IFN-$\gamma$, TGF-$\beta$, IL-15, IL-7, IFN-$\alpha$, IL-12, CD40L, IL-27, and the like, and particularly preferably exemplified by IL2, IFN-$\gamma$, and IL-12, from the viewpoint of enhancing a cellular immunity.

The chemokine as used herein is not particularly limited, so long as a chemokine acts on T and shows activity. For example, the chemokine is exemplified by RANTES, CCL21, MIP1$\alpha$, MIP1$\beta$, CCL19, CXCL12, IP-10, and MIG.

The cell capable of producing a as use herein is not particularly limited, so long as a cell is capable of the above-mentioned cytokine. For example, Th1 cell is preferably used, from the viewpoint of enhancing a cellular immunity.

In addition, the T cell population obtained by the preparation method of the present invention mentioned above contains in a high ratio naive T-like cells that are undifferentiated and is not stimulated by antigen. Therefore, as the stimulating factor used herein, it is particularly preferable to use a cell capable of presenting the antigen, a cell having presented an antigen, and/or an antigen. For example, as shown in item (5) of Example 5, item (8) of Example 6, item (2) of Example 7, item (1) of Example 8, item (7) of Example 14, and item (2) of Example 15 set forth below, antigen stimulation is provided to the T cell population obtained by the preparation method of the present invention mentioned above, whereby useful antigen-specific CTLs having very high cytotoxic activities and also having high abilities to recognize an antigen can be induced. In addition, the culture can be carried out in the presence of the above-mentioned fibronectin, a fragment thereof or a mixture thereof, and a known component that is used in the culture of T cells, in addition to the above-mentioned stimulating factors. The T cell population prepared as described above is a very useful T cell population having high therapeutic effects.

The present invention also provides a method for treating or preventing a disease, including the step of administering to a subject an effective amount of the activated T cell population obtained by the above-mentioned method. In addition, the present invention also provides use of the above-mentioned activated T cell population in the manufacture of a medicament. A method for manufacturing the medicament is carried out in the same manner as that for the above-mentioned medicament. Also, diseases to which the medicament is administered are not particularly limited, and are the same as those of the above-mentioned medicament.

Further, the present invention provides a medicament containing:

(a) a preparation containing as an effective ingredient the T cell population obtained by the preparation method of the present invention mentioned above, wherein the T cell population expresses CD45RA and expresses at least one selected from the group consisting of CD62L, CCR7, CD27, and and

(b) a preparation containing as an effective ingredient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine,

wherein the preparations are contained in the medicament as two separate preparations simultaneously or separately administered. As mentioned above, the T cell population obtained by the method of the present invention contains in a high ratio naive T-like cells. Therefore, the above-mentioned stimulating factors are administered simultaneously with or separately from the T cell population to a patient, whereby effects of the administered T cell population can be maximally exhibited and higher therapeutic effects on diseases can be exhibited. Here, as the stimulating factor, one that can provide antigen stimulation, in other words, one that can be used as a vaccine, for example, at least one selected from the group consisting of a cell capable of presenting an antigen, a cell having presented an antigen, an antigen, a CD3 ligand, a CD28 ligand, a cytokine, a chemokine, and a cell capable of producing a cytokine is preferable, and a cell capable of presenting an antigen, a cell having presented an antigen, or an antigen is more preferably used in the present invention.

There can be prepared and formed into a preparation (a) a preparation containing as an effective ingredient the T cell population obtained by the preparation method of the present invention mentioned above, wherein the T cell population expresses CD45RA and expresses at least one selected from the group consisting of CD62L, CCR7, CD27, and CD28, in the medicament, in the same manner as in the case of the medicament containing the T cell population as an effective ingredient as mentioned above. In addition, the content in the medicament, the dose, and the administration form of the T cell population that expresses CD45RA and expresses at least one selected from the group consisting of CD62L, CCR7, CD27, and CD28 are not particularly limited, and can be, for example, the same as those for the medicament containing the T cell population obtained by the preparation method of the present invention above.

In addition, as (b) a preparation containing as an effective ingredient at one stimulating factor from the group consisting of a cell capable of presenting an antigen, a cell having presented an antigen, an antigen, a CD3 ligand, a CD28 ligand, a cytokine, a chemokine, and a cell capable of producing a cytokine, in the medicament, for example, one formed into a preparation by the stimulating with an appropriate can be used. Also, as specific examples of at least one factor selected from the group consisting of a cell capable of presenting an antigen, a cell having presented an antigen, an antigen, a CD3 ligand, a CD28 ligand, a cytokine, a chemokine, and a cell capable of producing a cytokine, for example, the stimulating factors exemplified above can be used.

For example, when a cell capable of presenting an antigen, a cell having presented an antigen, an antigen, or a cell capable of producing a cytokine is used as the stimulating factor, a preparation can be formed by combining the stimulating factor with an appropriate pharmaceutical carrier for drip or injection without particular limitation. In addition, when an antigen peptide is used, the peptide can be combined with an appropriate adjuvant without particular limitation. Also, when a cytokine or chemokine is used, a preparation can be also formed by incorporating the cytokine or chemokine into a liposome by a known method. In addition, as the content of the stimulating factor in the preparation can be appropriately selected depending on the kind of the stimulating factor used, and is not particularly limited. For example, when a cell capable of presenting an antigen, a cell having presented an antigen, or a cell capable of producing a cytokine is used as the stimulating factor, the content is exemplified by, for example, preferably from $1 \times 10^3$ to $1 \times 10^9$ cells/mL, more preferably from $1 \times 10^3$ to $1 \times 10^8$ cells/mL, and even more preferably from $1 \times 10^4$ to $1 \times 10^7$ cells/mL. In addition, the administration for of the stimulating factor can be appropriately selected depending on the kind of the stimulating factor used, and is not particularly limited. For example, the stimulating factor is administered by an intravenous administration, an intra-arterial administration, a administration, an intraperitoneal administration, an oral administration, or the like. The dose is not particularly limited, so long as the dose is effective to treat or ameliorate a disease of patient. For example, when a cell capable of presenting an antigen is administered, the dose is exemplified by, for example, preferably from $1 \times 10^3$ to $1 \times 10^{11}$ cells/day, more preferably from $1 \times 10^3$ to $1 \times 10^{10}$ cells/day, and even more preferably from $1 \times 10^4$ to $1 \times 10^9$ cells/day. Also, when an antigen peptide is administered, the dose is exemplified by, for example, preferably from 0.001 to 100 mg/day, more preferably from 0.003 to 30 mg/day, and particularly preferably from 0.01 to 10 mg/day.

Incidentally, as a preferable example of administration of the preparation (b), for example, it is preferable that a cell capable of presenting an antigen is combined with an antigen to form into the preparation (b) and administered. For example, it is preferable that a dendritic cell is combined with an antigen peptide and administered. In this case, the content and the dose of the cell capable of presenting an antigen and the antigen in the preparation can be implemented as mentioned above.

In addition, the administrations of the preparation (a) and the preparation (b) in the medicament are simultaneously or separately administered to a patient. Here, the term "simultaneously" means that the preparations are administered to a patient as separate preparations simultaneously in terms of time or that the preparation (a) and the preparation (b) are mixed before administration to a patient and administered. For example, in a case where the preparation (a) and the preparation (b) are administered as drops the present invention encompasses an embodiment such that the above preparations are mixed before administration, and administered to a patient. In addition, the term "separately" means that the preparation (a) and the preparation (b) are administered separately in terms of time. Intervals of administrations of the preparation (a) and the preparation (b) are not particularly limited, so long as a simulation of a stimulating factor contained in the preparation (b) is provided to a T cell population contained in the preparation (a) in the body of patient. Preferably, it is preferable that the preparation (b) is administered the preparation (a) is administered. Also, the number of times of administration is not also particularly limited, and each preparation can be administered once or in divided portions of several times.

In addition, the present invention also provides a method for treating a disease, including the steps of (a) administering to a patient the T cell population obtained by the preparation method of the present invention mentioned above, wherein the T cell population expresses CD45RA and expresses at least one selected from the group consisting of CD62L, CCR7, CD27, and CD28, and (b) administering to a patient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine.

The treatment method can exhibit very high therapeutic effects, as mentioned above. Here, as the stimulating factor, one that can provide antigen stimulation, in other words, one that can be used as a vaccine, for example, at least one selected from the group consisting of a cell capable of presenting an antigen, a cell having presented an antigen, an antigen, a CD3 ligand, a CD28 ligand, a cytokine, a chemokine, and a cell capable of producing a cytokine is preferable, and a cell capable of presenting, a cell having presented an or an antigen is more preferably used in the present invention.

In the treatment method, the step of (a) administering to a patient the T cell population obtained by the method of the present invention is not particularly limited, and for example, is carried out by employing the dose and the administration form similar to those in the administration method of the above-mentioned medicament. In addition, the step of (b) administering to a patient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine is not also particularly limited, and for example, can be out by employing the dose and the administration form similar to those for the above-mentioned medicament.

In addition, intervals of administrations of the above-mentioned step of (a) and step of (b) can be implemented in the same manner as in the case of the medicament containing the preparation of (a) and the preparation of (b) mentioned above, in other words, are not particularly limited so long as a simulation of stimulating factor administered by the step of (b) is added to a T cell population administered by the step of (a) in the body of patient, and can be simultaneously or separately carried out. Preferably, it is preferable that the administration step of (b) is carried out after the administration step of (a). Also, the number of times of administration is not also particularly limited, and each preparation can be administered once or in divided portions of several times.


EXAMPLES


The invention will be more specifically described hereinbelow by the Examples, without intending to limit the scope of the present invention thereto.

Example 1: Analysis of CD45RA+ CD62L+ T Cells

(1) Isolation and Storage of PBMCs

Blood component was collected from a human normal individual donor, obtained with informed consent. The collected blood component was diluted 2-fold with phosphate buffered saline (hereinafter referred to as "PBS"), overlaid on Ficoll-paque (manufactured by Amersham Bioscience), and centrifuged at $600 \times g$ for 20 minutes. The peripheral blood mononuclear cells (hereinafter referred to as "PBMCs") in the intermediate layer were collected with a pipette, and washed. The collected PBMCs were suspended in a storage solution of 90% FBS (manufactured by Cambrex Corporation)/10% DMSO (manufactured by SIGMA), or a storage solution of an equivolume of CP-1 (manufactured by KYOKUTO PHARMACEUTICAL INDUSTRIAL CO., LTD.) containing 8% human serum albumin (manufactured by Baxter Limited, hereinafter referred to as "HSA"), and RPMI 1640 medium (manufactured by SIGMA), and stored in liquid nitrogen. During T cell expansion, these stored PBMCS were rapidly melted in water bath at 37°C, and washed with RPMI 1640 medium containing 10 μg/mL DNase (manufactured by Calbiochem). Thereafter, the number of living cells was calculated by trypan blue staining method. The cells were subjected to each experiment.

(2) Immobilization of Anti-Human CD3 Antibody and CH-296 Fragment

An anti-human CD3 antibody and a CH-296 fragment were immobilized to a culture equipment used in the following experiment. Concretely, 1.9 mL each of PBS containing an anti-human CD3 antibody (manufactured by JANSSEN PHARMACEUTICAL K.K.) (final concentration: 5 μg/mL) was added to a 12-well cell culture plate (manufactured by Dickinson). Upon the addition, CH-296 was added to a group with addition of CH-296 so as to have a final concentration of 25 μg/mL.

After these culture equipments were incubated at room temperature for 5 hours, the culture equipments were stored at 4°C until use. Immediately before use, PBS containing the antibody and the CH-296 was removed by aspiration from these culture equipments, and thereafter each well was washed twice with PBS, and then once with the RPMI 1640 medium, and the culture equipments were subjected to each experiment.

(3) Expansion of T Cell Population PBMCs which were prepared in item (1) of Example 1 were suspended in AIM-V (manufactured by Invitrogen) containing 1% human AB serum (manufactured by Cambrex) (hereinafter simply referred to as "1% AIM-V"), so as to have a density of $1 \times 10^6$ cells/mL, to prepare a cell suspension. Thereafter, the 1% AIM-V was added to a plate immobilized with the anti-human CD3 antibody or a plate immobilized with the anti-human CD3 antibody and the CH-296, prepared in item (2) of Example 1, in a volume of 2 mL/well each, and the above cell suspension was added thereto in a volume of 1 mL/well each. IL-2 (PROLEUKIN, manufactured by Chiron) was added thereto so as to have a concentration of 1000 U/mL, and these plates were subjected to culture at 37°C in 5% $CO_2$ (zeroth day of culture). On or after the fourth day from the initiation of culture, subculture was carried out with a fresh 12.5 cm²-cell culture flask (manufactured by Becton Dickenson) to which nothing was immobilized using 1% AIM-V, and IL-2 was added thereto so as to have a concentration of 500 U/mL (volume of culture medium: 6 mL). Concretely, subculture was carried out at a density of $0.05 \times 10^6$ cells/mL on the fourth day from the initiation of culture, at a density of $0.1 \times 10^6$ cells/mL on the seventh day, and at a density of $0.15 \times 10^6$ cells/mL on the tenth day. On the seventh day, the tenth day and the fourteenth day from the initiation of culture, the number of living was counted by trypan blue staining method, and an expansion fold was calculated by comparing the number of the counted cells with the number of cells at the initiation of culture. The results are shown in Table 1.

[Table 1]

Table 1

|  | Expansion Fold (folds) | | |
|---|---|---|---|
|  | 7th Day of Culture | 10th Day of Culture | 14th Day of Culture |
| Control (Without Immobilization of CH-296) | $\times$ 14 | $\times$ 257 | $\times$ 2491 |
| CH-296 | $\times$ 38 | $\times$ 315 | $\times$ 4387 |

As shown in Table 1, in the group in which the culture equipment immobilized with CH-296 at an early stage of T cell expansion was used, expansion folds of the T cell population were high at any stage of culture as compared to those of the control group.

(4) Analysis of CD45RA⁺ CD62L⁺ T Cells at Each Culture Day

The cells prepared at each culture day in item (3) of Example 1 were washed with PBS, and then with PBS containing 1% bovine serum albumin (manufactured by SIGMA, hereinafter referred to as "BSA") (hereinafter referred to as "1% BSA/PBS"). The cells were suspended in 1% BSA/PBS, and thereto was added as a negative control FITC-labeled mouse IgG1/RD1-labeled mouse IgG1/PC5-labeled mouse IgG1 (manufactured by Coulter). Similarly, FITC-labeled mouse anti-human CD62L antibody (manufactured by SIGMA)/RD1-labeled mouse anti-human CD45RA antibody (manufactured by Beckmann Coulter) was added. After each antibody was added, the cells were incubated on ice for 30 minutes. After the incubation, the cells were washed with 1% BSA/PBS, and resuspended in PBS. The cells were subjected to flow cytometry (Cytomics FC500, manufactured by Beckmann Coulter), and a percentage of CD45RA⁺ CD62L⁺ T cells was calculated. The results are shown in Table 2.

[Table 2]

Table 2

|  | Percentage of CD45RA⁺ CD62L⁺ T cells (%) | | |
|---|---|---|---|
|  | 7th Day of Culture | 10th Day of Culture | 14th Day of Culture |
| Control (Without Immobilization of CH-296) | 29.1 | 39.8 | 37.5 |
| CH-296 | 48.9 | 73.8 | 51.3 |

As shown in Table 2, in the group in which a culture equipment immobilized with the CH-296 fragment was used, the results of higher CD45RA⁺ CD62L⁺ T cell population in T cells during culture were obtained, as compared to those of the control group at any of the seventh day, the day, and the fourteenth day from the culture. It was clarified from these results that T cells can be expanded while increasing the percentage of the CD45RA⁺ CD62L⁺ T cell population of the T cell population during the expansion of from 7 to 14 days by the co-existence of the CH-296 fragment at an early stage of expansion.

Example 2: Analysis of CD45RA⁺ CCR7⁺ T Cells

(1) Expansion of T Cell Population

PBMCs which were prepared in item (1) of Example 1 were suspended in AIM-V containing 3% human AB serum (hereinafter simply referred to as "3% AIM-V") so as to have a density of $1 \times 10^6$ cells/mL, to prepare a cell suspension. Thereafter, the 3% AIM-V was added to a plate immobilized with the anti-human CD3 antibody or a plate immobilized with the anti-human CD3 antibody and the CH-296, prepared in item (2) of Example 1, in a volume of 2 mL/well, and the above cell suspension was added thereto in a volume of 1 mL/well each. IL-2 was added thereto so as to have a final concentration of 1000 U/mL, and these plates were subjected to culture at 37°C in 5% $CO_2$ (zeroth day of culture). On the fourth day after the initiation of culture, each group was diluted with 1% human AB serum AIM-V so as to have a density of $0.075 \times 10^6$ cells/mL (volume of liquid: 6 mL), the dilution was transferred to a 12.5 cm²-cell culture flask to which nothing was immobilized, and IL-2 was added thereto so as to have a final concentration of 500 U/mL. The serum concentration was determined, assuming that PBMCs obtained from 30 mL of blood collection were cultured in 10 L of a culture medium. The culture was continued, and on the seventh day, each group was transferred to a fresh

25 cm$^2$-cell culture flask (manufactured by Becton Dickenson), to which nothing was immobilized, using a culture medium (volume of liquid: 12.6 mL), prepared by diluting with 0.05% human AB serum AIM-V so as to have a density of 0.25 × 20$^6$ cells/mL. To each group was added IL-2 so as to have a final concentration of 500 U/mL. On the eleventh day, each group was transferred to a fresh 25 cm$^2$-cell culture flask to which nothing was immobilized, using a culture medium prepared by diluting with the human AB serum AIM-V of the same serum concentration as that of the seventh day so as to have a density of 1.04 × 10$^6$ cells/mL (volume of liquid: 12.6 mL). To each group was added IL-2 so as to have a final concentration of 500 U/mL. On the fourteenth day from the initiation of culture, the number of living cells was counted by trypan blue staining method, and an expansion fold was calculated by comparing the number of the counted cells with the number of cells at the initiation of culture. The results are shown in Table 3.

[Table 3]

Table 3

|  | Serum Concentration (%) | Cultured Days | Expansion Fold (folds) |
|---|---|---|---|
| Control (Without Immobilization of CH-296) | 3 → 1 → 0.05 | 14 Days | × 276 |
| CH-296 | 3 → 1 → 0.05 | 14 Days | × 688 |

As shown in Table 3, in the group in which the culture equipment immobilized with CH-296 at an early stage of T cell expansion was used, expansion folds of the T cells were high as compared to those of the control group. At present, it has been clarified that the CH-296 fragment is suitably used during expansion of T cells, and in this Example, it has been clarified that the CH-296 fragment is also suitably used during a 14-day expansion of T cells.

(2) Analysis of CD45RA$^+$ CCR7$^+$ T Cells in Subsets of CD8$^+$ T Cells and CD8$^-$ T Cells after Culture

The cells prepared in item (1) of Example 2 were washed with PBS, and then with 1% BSA/PBS. The cells were suspended in PBS containing 1% SA, and thereto was added as a negative control FITC-labeled mouse IgG1/RD1-labeled mouse IgG1/PC5-labeled mouse IgG1+ECD-labeled mouse IgG1 (manufactured by Beckman Coulter). Similarly, RD1-labeled mouse anti-human CD45RA antibody/FITC-labeled mouse anti-human CCR7 antibody (manufactured by R & D Systems)/ECD-labeled mouse anti-human CD8 antibody (manufactured by Beckmann Coulter). After each of the antibodies was added, the cells were incubated on ice for 30 minutes. During the course of incubation, the incubation mixture was gently stirred every ten minutes. After the incubation, the cells were washed with 1% BSA/PBS, and resuspended in PBS. The cells were subjected to flow cytometry, and classified into a CD8$^+$ T cell region and a CD8$^-$ T cell region by the stainability of CD8. For each cell population, the percentage of CD45RA$^+$ CCR7$^+$ T cells was calculated. The results are shown in Tables 4 and 5.

[Table 4]

Table 4

| CD8$^+$ T Cells | CD45RA$^+$ CCR7$^+$ T Cells (%) |
|---|---|
| Control (Without Immobilization of CH-296) | 11.9 |
| CH-296 | 37.4 |

[Table 5]

Table 5

| CD8$^-$ T Cells (CD4$^+$ T Cells) | CD45RA$^-$ CCR7$^+$ T Cells (%) |
|---|---|
| Control (Without Immobilization of CH-296) | 9.7 |
| CH-296 | 35.0 |

As shown in Table 4, in the group in which a culture equipment immobilized with the CH-296 fragment was used, the results of higher CD45RA$^+$ CCR7$^+$ T cell population in T cells during culture were obtained, as compared to those of the control group. This phenomenon was the same even in a case where CD8$^-$ T cells, i.e. cell population of which majority were CD4$^+$ T cells, were analyzed (Table 5). It was clarified from these results that T cell population can be expanded while increasing the percentage of the CD45RA$^+$ CC7$^+$ T cell population of the T cells by the co-existence of the CH-296 fragment at an early stage of a 14-day expansion.

Example 3: Analysis of Cytokine-Producing Ability of CD45RA$^+$ CCR7$^+$ T Cells and CD45RA$^-$ CCR7$^-$ T Cells in T Cell Population Expanded Using CH-296

(1) Expansion of T Cell Population PBMCs which were prepared in item (1) of Example 1 were suspended in GT-T503 (manufactured by TAKARA BIO, INC.) containing 0.5% human AB serum and 0.2% HSA (hereinafter simply referred to as "0.5% GT-T503") so as to have a density of $0.25 \times 10^6$ cells/mL, to prepare a cell suspension. Thereafter, the 0.5% GT-T503 was added to a plate immobilized with the anti-human CD3 antibody or a plate immobilized with the anti-human CD3 antibody and the CH-296, prepared in item (2) of Example 1, in a volume of 0.5 mL/well, and the above cell suspension was added thereto in a volume of 1 mL/well each. IL-2 was added thereto so as to have a final concentration of 1000 U/mL, and these plates were subjected to culture at 37˚C in 5% $CO_2$ (zeroth day of culture). On the fourth day after the initiation of culture, the culture medium of each group was diluted about 8-fold with the 0.5% GT-T503, and 6 mL of the dilution was transferred to a fresh 12.5 $cm^2$-cell culture flask to which nothing was immobilized, and IL-2 was added thereto so as to have a final concentration of 500 U/mL. The culture was continued, and on the seventh day, the cell culture medium of each group was diluted about 4.2-fold with the 0.5% GT-T503, and 12.6 mL of the dilution was transferred to a fresh 25 $cm^2$-cell culture flask to which nothing was immobilized. To each group was added IL-2 so as to have a final concentration of 500 U/mL. On the tenth day, the cell culture medium of each group was diluted about 2-fold with 0.5% GT-T503 containing 0.2% HSA, and 12.6 mL of the dilution was each transferred to a fresh 25 $cm^2$-cell culture flask to which nothing was immobilized. To each group was added IL-2 so as to have a final concentration of 500 U/mL. On the fourteenth day from the initiation of culture, the number of living cells was counted by trypan blue staining method, and an expansion fold was calculated by comparing the number of the counted cells with the number of cells at the initiation of culture. The results are shown in Table 6.

[Table 6]

Table 6

| | Expansion Fold (folds) |
|---|---|
| Control (Without Immobilization of CH-296) | $\times$ 172 |
| CH-296 | $\times$ 581 |

As shown in Table 6, in the group in which the culture equipment immobilized with CH-296 at an early stage of the expansion of the T cell population was used (hereinafter referred to as "the CH-296 group"), the expansion fold of the T cell population was high as compared to that of the control group.

(2) Analysis of CD45RA$^+$ CCR7$^+$ T Cells

The cells prepared in item (1) of Example 3 were stained with each antibody and analyzed in the same manner as in item (4) of Example 1, provided that the combinations of the antibodies were as follows. Concretely, staining was carried out with FITC-labeled mouse IgG1/RD1-labeled mouse IgG1/PC5-labeled mouse IgG1 and RD1-labeled mouse anti-human CD45RA antibody/FITC-labeled mouse anti-human CCR7 antibody. These stained cells were analyzed with a flow cytometer, and a percentage of CD45RA$^+$ CCR7$^+$ T cells was calculated. The results are shown in Table 7.

[Table 7]

Table 7

| | CD45RA$^+$ CCR7$^+$ T Cells (%) |
|---|---|
| Control (Without Immobilization of CH-296) | 21.2 |
| CH-296 | 63.7 |

As shown in Table 7, in the CH-296 group, the results of higher CD45RA$^+$ CCR7$^+$ T cell population in the T cell population during culture were obtained, as compared to those of the control group.

(3) Isolation of CD45RA$^+$ CCR7$^+$ T Cells and CD45RA$^-$ CCR7$^-$ T Cells After Expansion of T Cell Population

The cells prepared in item (1) of Example 3 were stained in the same manner as in item (2) of Example 2, and thereafter the cells were washed with 1% BSA/PBS, and suspended in the GT-T503 medium. The cells were subjected to sorting with Moflo (manufactured by DAKO DENMARK A/S) into CD45RA$^+$ CCR7$^+$ T cells and CD45RA$^-$ CCR7$^-$ T cells of CD8$^+$ T cells, and similarly into CD45RA$^+$ CCR7$^+$ T cells and CD45RA$^-$ CCR7$^-$ T cells of CD8$^-$ T cells, ie. a majority of which were CD4$^+$ T cells. In addition, the stained cells before the same sorting were subjected to flow cytometry, and sorted into a CD8$^+$ T cell region and a CD8$^-$ T cell region according to the stainability of CD8. The percentage of CD45RA$^+$ CCR7$^+$ T cells for each cell population was calculated. The results are shown in Tables 8 and 9.

[Table 8]

Table 8 CD8+ T Cells

|  | CD45RA+ CCR7+ T Cells (%) |
|---|---|
| Control (Without Immobilization of CH-296) | 15.9 |
| CH-296 | 52.6 |

[Table 9]

Table 9 CD8- T Cells (CD4+ T Cells)

|  | CD45RA+ CCR7+ T Cells |
|---|---|
| Control (Without Immobilization of CH-296) | 5.4 |
| CH-296 | 34.0 |

As shown in Table 8, regarding the CD8+ T cells, in the CH-296 group, the results of a higher percentage of the CD45RA+ CCR7+ T cell population in T cell population after the culture were obtained, as compared to those of the control group. This phenomenon was the same even in a case where CD8- T cells, i.e. cell population of which majority are CD4+ T cells, were analyzed (Table 9).

(4) Immobilization of Anti-Human CD3 Antibody and Anti-Human CD28 Antibody

An anti-human CD3 antibody and an anti-human CD28 antibody were immobilized to a culture equipment used in the following experiment. Concretely, 80 $\mu$L each of acetate buffer (pH 5.3) containing an anti-human CD3 antibody (2 $\mu$g/mL) was added to a 96-well cell culture plate (manufactured by Becton Dickinson). Thereto was further added 80 $\mu$L each of acetate buffer containing anti-human CD28 antibody (manufactured by DAKO DENMARK A/S) (20 $\mu$g/mL). The anti-human CD3 antibody was added so as to have a final concentration of 1 $\mu$g/mL, and the anti-human CD28 antibody was added so as to have a final concentration of 10 $\mu$g/mL. After these culture equipments were incubated at room temperature for 5 hours, the culture equipments were stored at 4°C until use. Immediately before use, the acetate buffer containing the antibody was removed by aspiration from these culture equipments, and thereafter well was washed twice with PBS, and then once with the GT-T503 medium, and the culture equipments were subjected to each experiment.

(5) Stimulation of Cells

Naive T and central memory T cells have been known to produce IL-2 in a large amount upon stimulation with an antigen in the body. In addition, effector memory T cells have been to produce IFN-$\gamma$ or IL-4 in a large amount upon with an antigen. In order to confirm that each of the cell fractions obtained in item (3) of Example 3 maintains these functions, the cytokine-producing ability when stimulated with an anti-CD3 antibody and an anti-CD28 antibody was determined.

Each of the cell population obtained in item (3) of Example 3 was collected, the cells were then suspended in the 0.5% GT-T503, and the number of cells was counted. Cells were added to each well of the plate immobilized with the anti-human CD3 antibody and the anti-human CD28 antibody prepared in item (4) of Example 3 so as to have a density of $2 \times 10^5$ cells/0.2 mL, and the cells were cultured at 37°C in 5% $CO_2$. After 24 hours, the culture supernatant was collected, and used for the experiment for the determination of cytokine according to ELISA method.

(6) Determination of Cytokine Production According to ELISA Method

As shown in item (3) of Example 3, in the CH-296 group, the results of a higher percentage of the CD45RA+ CCR7+ T cells were obtained, as compared to those of the control group. In order to confirm that this CD45RA+ CCR7+ T cell population maintains its property as naive T-like cells, the cytokine-producing ability of CD45RA+ CCR7+ T cells and CD45RA- CCR7- T cells isolated after the expansion of the T cell population was evaluated. The production of IL-2 or IFN-$\gamma$ was determined with ELISA Development Kit (manufactured by R & D Systems), and the production of IL-4 was determined with READY-SET-GO! Human Interleukin-4 (manufactured by ebioscience). The results of each cytokine production in the control group and the CH-206 group are shown in Tables 10 and 11, respectively.

[Table 10]

Table 10

| Control (Without Immobilization of CH-296) |  | IL-2 (pg/mL) | IFN-$\gamma$ (pg/mL) | IL-4 (pg/mL) |
|---|---|---|---|---|
| CD8+ T Cells | CD45RA+ CCR7+ T Cells | 286.7 | 568.0 | 30.5 |
|  | CD45RA- CCR7- T Cells | 104.9 | 1229.0 | 430.8 |
| CD8- T Cells | CD45RA+ CCR7+ T Cells | 1539.4 | 107.9 | 113.9 |
|  | CD45RA- CCR7- T Cells | 877.0 | 1200.0 | 1004.0 |

[Table 11]

Table 11

| Immobilization of CH-296 | | IL-2 (pg/mL) | IFN-$\gamma$ (pg/mL) | IL-4 (pg/mL) |
|---|---|---|---|---|
| CD8$^+$ T Cells | CD45RA$^+$CCR7$^+$T Cells | 174.4 | 251.3 | < 0 |
| | CD45RA$^-$CCR7$^-$ T Cells | 44.9 | 526.3 | 58.1 |
| CD8$^-$ T Cells | CD45RA$^+$ CCR7$^+$ T Cells | 1958.2 | 144.3 | 20.3 |
| | CD45RA$^-$ CCR7$^-$ T Cells | 611.0 | 587.5 | 727.3 |

As shown in Tables 10 and 11, in the control group and the CH-296 group, the IL-2 production was confirmed. In both the groups, the CD8$^-$ T cells had a larger amount of the cytokine production than that of the CD8$^+$ T cells, and the CD45RA$^+$ CCR7$^+$ T cells had a larger amount of the production than that of the CD45RA$^-$ CCR7$^-$ T cells. It was shown from the above that the CD45RA$^+$ CCR7$^+$ T cell population obtained by the expansion of T cell population had properties as naive T-like cells. On the other hand, IFN-$\gamma$ and IL-4 were dominantly produced in CD45RA$^-$ CCR7$^-$ T cells in both the control group and the CH-296 group, suggesting that the CD45RA$^-$ CCR7$^-$ T cell population is a population in which the the CD45RA$^-$ CCR7$^-$ T cell population is a population in which the effector memory function is maintained.

Example 4: Analysis of Chemotaxis of T Cell Population Expanded Using CH-296

(1) Immobilization of Anti-Human CD3 Antibody and CH-296 Fragment

The immobilization was carried out in the same manner as in item (2) of Example 1, except that PBS containing an anti-human CD3 antibody (final concentration: 5 $\mu$g/mL) was added in a volume of 0.45 mL each.

(2) Expansion of T Cell Population

The same procedures as those in item (1) of Example 3 were carried out that on the day from the initiation of culture, the culture medium was diluted about 14-fold, and 10 mL of the dilution was added to a fresh 25 cm$^2$-cell culture flask (manufactured by Corning), that on the eighth day from the initiation of culture, the culture medium was diluted about 2-fold, and 10 mL of the dilution was added to a fresh 25 cm$^2$-cell culture flask (manufactured by Corning), and that on the eleventh day from the initiation of culture, 10 mL of GT-T503 medium containing 0.2% HSA was added to each of the flask. The results of the expansion folds are shown in Table 12.

[Table 12]

Table 12

| | Expansion Fold (folds) |
|---|---|
| Control (Without Immobilization of CH-296) | $\times$ 414 |
| CH-296 | $\times$ 646 |

As shown in Table 12, in the group in which a culture equipment immobilized with CH-296 at an early stage of the expansion of the T cell population was (hereinafter referred to as "the CH-296 group"), the expansion fold of the T cell population was high as compared to that of the control group.

(3) Analysis of CD45RA$^+$ CCR7$^+$ T Cells

For the cells prepared in item (2) of Example 4, a percentage of CD45RA$^+$ CCR7$^+$ T cells was calculated in the same as in item (2) of Example 3. The results are shown in Table 13.

[Table 13]

Table 13

| | CD45RA$^+$ CCR7$^+$ T Cells (%) |
|---|---|
| Control (Without Immobilization of CH-296) | 31.5 |
| CH-296 | 56.8 |

As shown in Table 13, in the CH-296 group, the results of higher CD45RA$^+$ CCR7$^+$ T cell population in the T cell population during the culture were obtained, as compared to that of the control group.

(4) Analysis of Chemotaxis

In the CH-296 group, the results of a higher percentage of CD45RA$^+$ CCR7$^+$ T cell population have been obtained, as compared to that of the control group. The CCR7-positive cells such as naive T cells and central memory T cells show chemotaxis in response to chemokine CCL21. This is an important event for these cells to migrate from blood vessels to lymph nodes, and it was confirmed if the cells obtained after the expansion had chemotaxis in response to CCL21. The cells obtained in item (2) of Example 4 were centrifuged, the supernatant was removed, and the cells were then suspended in RPMI 1640 medium containing 0.5% BSA (hereinafter shown as a reaction medium) so as to have a density of $5 \times 10^6$ cells/mL. To a lower layer of a Transwell plate (Transwell Polycarbonate, 24-well, 5 $\mu$m pore size, manufactured by Coming) was added 600 $\mu$L of a reaction medium containing CCL21 (manufactured by R & D Systems) at a final concentration of 1 $\mu$g/mL, and 100 $\mu$L of the prepared cell suspension was added to the upper layer. The number of cells migrated to the lower layer after culturing at 37˚C for 2 hours and the number of cells remaining on the upper layer were determined. The percentage of the cells that induced chemotaxis was calculated from the following formula (1):

[Su 1]

$$\text{Percentage (\%) of Cells That Induced Chemotaxis} = (\text{Number of}$$

$$\text{Cells Migrated to Lower Layer} \div (\text{Number of Cells Remaining in Upper}$$

$$\text{Layer} + \text{Number of Cells Migrated to Lower Layer})) \times 100$$

The results are shown in Table 14.

[Table 14]

Table 14

| | Percentage of Cells That Induced Chemotaxis (%) |
|---|---|
| Control (Without Immobilization of CH-296) | 43.2 |
| CH-296 | 58.8 |

As shown in Table 14, cells which showed chemotaxis in response to CCL21 were confirmed in both the control group and the CH-296 group, and the was for the CH-296 group. In other words, it was clarified cells having migration ability to lymph nodes were obtained in a amount by using CH-296 upon the expansion of the T cell population.

Example 5: Induction of Anti-MART-1 CTLs from Cell Population Expanded Using CH-296

(1) Immobilization of Anti-Human CD3 Antibody and CH-296 Fragment The same procedures as in item (1) of Example 4 were out.

(2) Expansion of T Cell Population

The same procedures as in item (2) of Example 4 were carried out. The results of the expansion folds are shown in Table 15.

[Table 15]

Table 15

| | Expansion Fold (folds) |
|---|---|
| Control (Without Immobilization of CH-296) | $\times$ 189 |
| CH-296 | $\times$387 |

As shown in Table 15, in the group in which a culture equipment immobilized with CH-296 at an early stage of the expansion was used (hereinafter referred to as "the CH-296 group"), the expansion fold was high as compared to that of the control group.

(3) Analysis of CD45RA$^+$ CCR7$^+$ and CD45RA$^+$ CD62L$^+$ T Cells

The cells prepared in item (2) of Example 5 were stained with each antibody and analyzed in the same manner as in item (4) of Example 1, provided that the combinations of the antibodies were as follows. Concretely, staining was carried out with FITC-labeled mouse IgG1/RD1-labeled mouse IgG1 (both manufactured by DAKO DENNLARK A/S), RD1-labeled mouse anti-human CD45RA antibody/FITC-labeled mouse anti-human CCR7 antibody, and RD1-labeled mouse anti-human CD45RA antibody/FITC-labeled mouse anti-human CD62L antibody (hereinafter the anti-human CD62L antibody being a product manufactured by eBioscicnce). These stained cells were analyzed with a flow cytometer, and percentages of CD45RA$^+$ CCR7$^+$ T cells and CD45RA$^+$ CD62L$^+$ T cells were calculated. The results are shown in Table 16.

[Table 16]

Table 16

| | Control (Without Immobilization with CH-296) | CH-296 |
|---|---|---|
| CD45RA$^+$ CD62L$^+$ T Cells (%) | 60.2 | 76.9 |
| CD45RA$^+$ CCR7$^+$ T Cells (%) | 47.1 | 75.0 |

As shown in Table 16, in the CH-296 group, values were shown in both the cell surface markers as compared to those of the control group.

(4) Storage of Cultured Cells

Cells on the fourteenth day of the culture prepared in item (2) of Example 5 were suspended in a storage solution composed of equivolumes of 90% FBS/10% DMSO or CP-1 containing 8% HSA, and RPMI 1640 medium, and the suspension was stored in liquid nitrogen. During the induction of the CTLs, these stored cultured were rapidly melted in a water bath at 37°C, and washed with RPMI 1640 medium containing 10 $\mu$g/mL DNase. Thereafter, the number of living cells was calculated by trypan blue staining method. The cells were subjected to each experiment.

(5) Induction of CTLs Specific to Antitumor-Associated Antigen (MART-1)

Using the cells prepared in item (4) of Example 5, the induction of CTLs specific to antitumor-associated antigen (Melanoma antigen recognized by T cells: MART-1) was carried out. The induction of CTLs specific to antitumor-associated (MART-1) was carried out by partly modifying a method by Plebanski M. et al. [Eur. J. Immunol, 25(6), 1783-1787 (1995)]. Concretely, the PBMCs prepared in item (1) of Example 1 as antigen presenting cells, the PBMCs were incubated in a RPMI 1640 medium containing 5% human AB sera (hereinafter simply referred to as "5HRPMI") containing a 40 $\mu$g/mL melanoma antigen MART-1-derived epitope peptide (melanoma MART-1-derived HLA-A2.1 binding peptide described in SEQ ID NO: 22 of Sequence Listing) as an antigen peptide and 3 $\mu$g/mL $\beta_2$ microglobulin (manufactured by Scrips), a 0.1 mM NEAA mixture, 1 mM sodium pyruvate, 2 mM L-glutamine (all manufactured by Cambrex) and 100 $\mu$g/mL streptomycin sulfate (manufactured by MEIJI SEIKA KAISHA, LTD.) in a 5% CO$_2$ incubator at 37°C for 2 hours. After the termination of incubation, the mixture was irradiated with X-rays (1.42 C/kg), and the density was adjusted with the 5HRPMI to 3 to 4 $\times$ 10$^6$ cells/mL.

On the other hand, the cultured cells prepared in item (4) of Example 5 were suspended in the 5HRPMI so as to have a density of 2 $\times$ 10$^6$ cells/mL, and the suspension was added to a 24-well cell culture plate (manufactured by Becton Dickenson) in a volume of 0.5 mL/well each. To each well was added the antigen presenting cells prepared by the above method in a volume of 0.5 mL/well each, and IL-7 (manufactured by R & D Systems) and KLH (manufactured by Calbiochem) were added so as to have a final concentration of 25 ng/mL and 5 $\mu$g/mL, respectively. The plate was cultured in a 5% CO$_2$ incubator at 37°C. On the first day from the initiation of the culture, 1 mL of 5HRPMI containing 60 U/mL IL-2 was added to each well. On the fourth day from the initiation of the culture, a half the volume of the culture supernatant was removed, and 1 mL of 5HRPMI containing 60 U/mL IL-2 was added to each well. In addition, on the seventh day, the antigen presenting cells were prepared in the same manner as above, and the mixture was then irradiated with X-rays (1.42 C/kg), and the cells were prepared so as to have a density of 4 $\times$ 10$^6$ cells/mL, and the cells were added to a fresh 24-well cell culture plate in a volume of 0.5 mL/well each.

The responder cells subjected to a one-week culture (a part of the cells being continued to be cultured for the assay of cytotoxic activity) were suspended in the 5HRPMI, so as to have a density of from 1.8 to 2.0 $\times$ 10$^6$ cells/mL, and the suspension was added to a similar plate in a volume of 0.5 mL/well each. Further, IL-7 was added thereto so as to have a final concentration of 25 ng/mL to re-stimulate the cells. On the eighth day from the initiation of culture, 1 mL of a 5HRPMI containing 60 U/mL IL-2 was added to each well. On the eleventh day from the initiation of culture, the cells in each well were suspended, and divided into two wells in a half the volume each, and 1 mL of 5HRPMI containing 60 U/mL IL-2 was added to each well, and the cells were continued to be cultured until the fifteenth day.

(6) Determination of Expansion Fold

As to the cells obtained in item (5) of Example 5, on the seventh day and the fourteenth day from the initiation of culture, the number of living cells was counted by trypan blue staining method, and an expansion fold was calculated by comparing the number of the counted cells to the number of cells at the initiation of culture. The results are shown in Table 17.

[Table 17]

Table 17

| Days | Control (Without Immobilization with CH-296) | CH-296 |
|---|---|---|
| Seventh Day | $\times$ 1.5 | $\times$ 1.9 |

(continued)

| Days | Control (Without Immobilization with CH-296) | CH-296 |
|---|---|---|
| Fourteenth Day | × 6.0 | × 8.4 |

As shown in Table 17, in the CTL population induced from the CH-296 group, the expansion folds of the CTL population were high as compared to those of the control group. In other words, it was clarified that the CTL population could be obtained in a larger amount by carrying out the induction of CTLs from the cultured cells in which a culture equipment immobilized with CH-296 at an early stage of the expansion of T cell population was used.

(7) Assay of Cytotoxic Activity
The activity of the CTLs on the fifteenth day from the initiation of culture prepared in item (5) of Example 5 was evaluated by a method for assaying cytotoxic activity using calcein-AM [Lichtenfels R. et al.,J. Immunol. Methods 172(2), 227-239 (1994)]. Concretely, HLA-A2.1-restricted cell line T2 cells (ATCC CRL-1992), which were co-cultured overnight together with an epitope peptide or cultured in the absence of an epitope peptide, were suspended in RPMI 1640 medium containing 5% FBS so as to have a density of $1 \times 10^6$ cells/mL, and calcein-AM (manufactured by DOJINDO LABORATORIES) was then thereto so as to have a final concentration of 25 $\mu$M, and the cells were cultured at 37˚C for 1 hour. The cells were washed with a medium without containing calcein-AM, and thereafter calcein-labeled target cells were obtained. The calcein-labeled target cells were mixed with 30-fold amount of K562 cells (Human Science Research Resources Bank JCRB0019), to provide cells for assaying cytotoxic activity. Here, the K562 cells were used for excluding non-specific cytotoxic activity by NK cells admixed in the responder cells.

The CTLs prepared in item (5) of Example 5 were serially diluted with the 5HRPMI as effector cells, so as to have a density of from $3 \times 10^5$ to $3 \times 10^6$ cells/mL. Thereafter, the dilution was previously dispensed to each well of a 96-well cell culture plate in a volume of 100 $\mu$L/well each, and to these plates were added the cells for assaying cytotoxic activity in a volume of 100 $\mu$L/well each prepared so that the calcein-labeled target cells had a density of $1 \times 10^5$/mL. Upon the determination, the ratio of the effector cells (E) to the calcein-labeled target cells (T) is expressed as an E/T ratio, and the determinations were made on E/T ratios of 30, 10, and 3. The plate containing the above cell suspension was centrifuged at 400 × g for 1 minute, and thereafter the cells were incubated in a 5% $CO_2$ incubator at 37˚C for 4 hours. Thereafter, 100 $\mu$L of the culture supernatant was collected from each well, and the amount of calcein released into the culture supernatant was determined with a fluorescence plate reader (spectrofluorometer) (manufactured by BERTHOLD TECHNOLOGIES) (excited at 485 nm/measured at 538 or 535 nm). "Specific Cytotoxic Activity (%)" was calculated in accordance with the following formula (2):

[Su 2]

$$\text{Specific Cytotoxic Activity (\%)} =$$

$$\{(\text{Measured Value in Each Well} - \text{Minimum Released Amount})/$$

$$(\text{Maximum Released Amount} - \text{Minimum Released Amount})\} \times 100$$

In the above formula, the minimum released amount is the amount of calcein released in the well containing only cells for assaying cytotoxic activity, showing the amount of calcein naturally released from the calcein-labeled target cells. In addition, the maximum released amount refers to the amount of calcein released when the cells are completely disrupted by adding 0.1% of the surfactant Triton X-100 (manufactured by Nakalai Tesque Inc.) to the cells for assaying cytotoxic activity. The results of the assay of cytotoxic activity are shown in Table 18.

[Table 18]

Table 18

| Target Cells | E/T | Cytotoxic Activity (%) | |
|---|---|---|---|
| | | Control (Without Immobilization of CH-296) | CH-296 |
| T2 | 30 | <0 | <0 |
| | 10 | <0 | <0 |
| | 3 | <0 | <0 |
| T2+MART-1 Peptide | 30 | 62.0 | 87.2 |

(continued)

| Target Cells | E/T | Cytotoxic Activity (%) | |
| --- | --- | --- | --- |
| | | Control (Without Immobilization of CH-296) | CH-296 |
| | 10 | 29.1 | 60.1 |
| | 3 | 9.2 | 26.3 |

As shown in Table 18, in the CTL population induced from the CH-296 group, the specific cytotoxic activities of the CTL population were high as compared to those of the control group. In other words, it was clarified that the cultured cells in which a culture equipment immobilized with CH-296 at an early stage of the expansion of the T cell population was used could be induced to a CTL population having an even higher specific cytotoxic activity.

Example 6: Allogeneic MLR and Induction of Anti-MART-1 CTLs of Cell Population Expanded Using Gas-Permeable, Culture Bag After Stimulation at Early Stage of CH-296

(1) Immobilization of Anti-CD3 Antibody and CH-296 Fragment

An anti-human CD3 antibody and a CH-296 fragment were immobilized to a culture equipment used in the following experiment. Concretely, 9 mL each of PBS containing an anti-human CD3 antibody (final concentration: 5 $\mu$g/mL) was added to a 75 $cm^2$-cell culture flask (manufactured by Becton Dickinson). Upon the addition, CH-296 was added to a group with addition of CH-296 so as to have a final concentration of 25 $\mu$g/mL.

After these culture equipments were incubated at room temperature for 5 hours, the culture equipments were stored at 4°C until use. Immediately before use, PBS containing the antibody and the CH-296 was removed by aspiration from these culture equipments, and thereafter each was washed twice with PBS, and then once with the RPMI medium, and the culture equipments were subjected to each experiment.

(2) Expansion of T Cell Population

The PBMCs which were prepared in item (1) of Example 1 were suspended in 0.5% GT-T503 so as to have a density of 0.5 $\times$ 10$^6$ cells/mL, to prepare a cell suspension. Thereafter, the 0.5% GT-T503 was previously added to a flask immobilized with the anti-human CD3 antibody or a immobilized with the anti-human CD3 antibody and the CH-296, prepared in item (1) of Example 6, in a volume of 21 mL/flask, and the above cell suspension was added thereto in a volume of 9 mL/flask each. IL-2 was added thereto so as to have a final concentration of 1000 U/mL, and these flasks were subjected to culture at 37°C in 5% $CO_2$ (zeroth day of culture). On the fourth day after the initiation of culture, 14 mL of the culture medium of each group and 186 mL of 0.5% GT-T503 were transferred to a gas-permeable culture bag (200 $cm^2$, manufactured by TAKARA BIO, INC., commercial code: KB210) to which nothing was immobilized, and IL-2 was further added thereto so as to have a final concentration of 500 U/mL. The culture was continued, and on the eighth day, 100 mL of the cell culture medium of each group was removed from the culture bag, leaving 100 mL of the cell culture medium in the bag, and the remaining culture medium was diluted 2-fold with the 0.5% GT-T503, to make up a volume of 200 mL of the cell dilution in the bag. To each group was further added IL-2 so as to each have a final concentration of 500 U/mL. On the eleventh day from the initiation of culture, 200 mL of GT-T503 medium containing 0.2% HSA was added thereto to dilute 2-fold with the cell culture medium of each group. To each group was further added IL-2 so as to each have a final concentration of 500 U/mL. On the fourteenth day from the initiation of culture, the number of living cells was counted by trypan blue staining method, and an expansion fold was calculated by comparing the number of the counted cells with the number at the initiation of culture. The results are shown in Table 19.

[Table 19]

Table 19

| | Expansion Fold (folds) |
| --- | --- |
| Control (Without Immobilization of CH-296) | $\times$ 225 |
| CH-296 | $\times$ 333 |

As shown in Table 19, even in a case where the cells were cultured in a gas-permeable culture bag, in the group in which a culture equipment immobilized with CH-296 at an early of the expansion of the T cell population was used (hereinafter referred to as "the CH-296 group"), the fold of the T cell population was high as compared to that of the control group.

(3) Analysis of CD45RA$^+$ CCR7$^+$ T cells,
CD45RA$^+$ CD62L$^+$ T cells, CD45RA$^+$ CD27$^+$ T cells,
CD45RA$^+$ CD28$^+$ T cells, CD27$^+$ CD28$^+$ T cells, and
CD45RA$^+$ CCR7$^+$ CD62L$^+$ T cells in T Cell Population Cultured in Gas-Permeable Culture Bag

The cells prepared in item (2) of Example 6 were stained with each antibody and analyzed in the same manner as in item (4) of Example 1, provided that the combinations of the antibodies were as follows. Concretely, staining was carried out with FITC-labeled mouse IgG1/RD1-labeled mouse IgG1/PC5 -labeled mouse IgG1, RD1-labeled mouse anti-human CD45RA antibody/FITC-labeled mouse anti-human CCR7 antibody, RD1-labeled mouse anti-human CD45RA antibody/PC5-labeled mouse anti-human CD62L antibody (manufactured by Beckmann-Coulter), RD1-labeled mouse anti-human CD45RA antibody/FITC-labeled mouse anti-human CD28 antibody (manufactured by eBioscience), RD1-labeled mouse anti-human CD45RA antibody/PC5-labeled mouse anti-human CD27 antibody (manufactured by Beckmann-Coulter), and RD1-labeled mouse anti-human CD45RA antibody/FITC-labeled mouse anti-human CCR7 antibody/PC5-labeled mouse anti-human CD62L antibody. These stained cells were analyzed with a flow cytometer, and percentages of CD45RA$^+$ CCR7$^+$ T cells, CD45RA$^+$ CD62L$^+$ T cells, CD45RA$^+$ CD27$^+$ T cells, CD45RA$^+$ CD28$^+$ T cells, CD27$^+$ CD28$^+$ T cells, and CD45RA$^+$ CCR7$^+$ CD62L$^+$ T cells were calculated. The results are shown in Table 20.

[Table 20]

Table 20

|  | Control (Without Immobilization of CH-296) | CH-296 |
|---|---|---|
| CD45RA$^+$ CCR7$^+$ T Cells | 24.4% | 49.0% |
| CD45RA$^+$ CD62L$^+$ T Cells | 40.2% | 60.2% |
| CD45RA$^+$ CD28$^+$ T Cells | 36.9% | 63.0% |
| CD45RA$^+$ CD27$^+$ T Cells | 46.3% | 63.1% |
| CD28$^+$ CD27$^+$ T Cells | 58.9% | 72.1% |
| CD45RA$^+$ CCR7$^+$ CD62L$^+$ T Cells | 23.0% | 47.3% |

As shown in Table 20, in the CH-296 group, a high value was shown in any one of the cell surface markers, as compared to those of the control group. CD27 and CD28 have been known to have high percentages of expression in undifferentiated cells such as naive T cells. Therefore, it was clarified that even when analyzed with these markers, the percentage of the naive T-like cell population is increased in the T cell population by allowing CH-296 to act at an early stage of the culture.

(4) Allogeneic MLR

An allogeneic MLR was carried out using the cells prepared in item (2) of Example 6. Concretely, PBMCs, prepared in the same manner as in item (1) of Example 1 derived from an allogeneic donor (non-self donor: a donor different from the donor used in item (2) of Example 6), were irradiated with X-rays (0.88 C/kg), and the cells were suspended with 5HRPMI so as to have a density of $2 \times 10^6$ cells/mL (stimulator cells). On the other hand, the cultured cells prepared in item (2) of Example 6 were suspended in 5HRPMI so as to have a density of $2 \times 10^6$ cells/mL (responder cells). The stimulator cells and the responder cells prepared were added to a 24-well cell culture plate in a volume of 0.5 mL/well each. IL-2 was added to each well so as to have a final concentration of 500 U/mL, and thereafter the cells on the plate were cultured in a 5% $CO_2$ incubator at 37˚C. On the third day from the initiation of culture, 1 mL of 5HRPMI containing 1000 U/mL IL-2 was added to each well. On the fifth day from the initiation of culture, a half the volume of the culture supernatant was removed, and 1 mL of 5HRPMI containing 1000 U/mL IL-2 was added to each well. In addition, on the seventh day, the cells in each well were suspended, the suspension was divided into two wells of a half the volume each, 1 mL of 5HRPMI containing 1000 U/mL IL-2 was added to each well, and the culture was continued until the tenth day.

(5) Determination of Expansion Fold

As to the cells obtained in item (4) of Example 6, on the seventh day and the tenth day from the initiation of culture, the number of living cells was counted by trypan blue staining method, and an expansion fold was calculated by comparing the number of counted cells to the number of cells at the initiation of culture. The results are shown in Table 21.

[Table 21]

Table 21

| Cultured Days | Stimulator Cell Donor | Responder Cells | |
|---|---|---|---|
|  |  | Control (Without Immobilization with CH-296) | CH-296 |
| Seventh | A | $\times$ 1.7 | $\times$ 2.7 |
| Day | B | $\times$ 1.6 | $\times$ 2.7 |
| Tenth | A | $\times$ 1.8 | $\times$ 3.0 |
| Day | B | $\times$ 2.1 | $\times$ 2.7 |

As shown in Table 21, in a case where an allogeneic MLR was carried out the CH-296 the expansion fold the reaction was high as compared to that of the control group. In other words, it was clarified that when an allogeneic MLR was carried out using cultured cells in which a culture equipment immobilized with CH-296 at an early stage of the expansion of the T cell population was used, alloantigen recognizing cells are proliferated in an even larger amount.

(6) Assay of Cytotoxic Activity

The cytotoxic activity of the cells on the tenth day from the initiation of induction, prepared in item (4) of Example 6 was assayed in the same manner as in item (7) of Example 5. Here, as the target cells upon the determination, self-PBMCs or non-self-PBMCs which were subjected to blastogenesis (blasting) with phytohemagglutinin (hereinafter simply referred to as "PHA") for 10 days were used as calcein-labeled target cells. During the assay for the cytotoxic activity, a 30-fold amount of K562 cells and calcein-labeled target cells were mixed. The results for the assay of cytotoxic activity are shown in Table 22.

[Table 22]

Table 22

| Stimulator Cell Donor | Target Cells | E/T | Cytotoxic Activity (%) | |
|---|---|---|---|---|
| | | | Control (Without Immobilization, of CH-296) | CH-296 |
| A | Non-Self | 30 | 50.6 | 63.8 |
| | PHA Blast | 10 | 29.3 | 44.1 |
| | Cells | 3 | 7 | 16.9 |
| | | 30 | < 0 | < 0 |
| | Self PHA | 10 | <0 | <0 |
| | Blast Cells | 3 | < 0 | <0 |
| B | | 30 | 26.6 | 60.1 |
| | Non-Self PHA Blast | 10 | 9.1 | 38.0 |
| | Cells | 3 | <0 | 13.0 |
| | | 30 | <0 | < 0 |
| | Self PHA | 10 | < 0 | < 0 |
| | Blast Cells | 3 | <0 | <0 |

As shown in Table 22, in a case where an allogeneic MLR was carried out using the CH-296 group, the alloantigen-specific cytotoxic activity after the reaction was high, as compared to that of the control group. In other words, in the cultured cells in which a culture equipment immobilized with CH-296 at an early stage of the expansion of the T cell population was used, it was clarified that a cell population having an even more potent alloantigen-specific cytotoxic activity by the allogeneic MLR could be obtained.

(7) Storage of Cultured Cells

The cells on the fourteenth day of the culture prepared in item (2) of Example 6 were subjected to frozen storage and melting in the same manner as in item (4) of Example 5. The cells were subjected to each experiment.

(8) Induction of CTLs Specific to Antitumor-Associated

Antigen (MART-1)

Using the cells prepared in item (1) of Example 1 and item (7) of Example 6, the induction of CTLs specific to antitumor-associated antigen (MART-1) was carried out in the same manner as in item (5) of Example 5, and the culture was continued for thirteen days, provided that the modifications were made as follows: The features include the cultured cells prepared in item (7) of Example 6 were suspended in 5HRPMI so as to have a density of $1 \times 10^6$ cells/mL; on the second day from the initiation of culture, 1 mL of 5HRPMI containing 60U/mL IL-2 was added to each well, on the sixth day from the initiation of culture, responder cells were suspended in 5HRPMI so as to have a density of from 0.3 to 1.3 $\times 10^6$ cells/mL, and antigen presenting cells were suspended therein so as to have a density of $1.6 \times 10^6$ cells/mL; and on the seventh day from the initiation of culture, 1 mL of 5HRPMI containing 60 U/mL IL-2 was added to each well, and on the tenth day from the initiation of culture, the cells in well were suspended, and divided into two well of a half the volume each, and 1 mL of 5HRPMI containing 60 U/mL IL-2 was added to each well.

(9) Assay of Cytotoxic Activity

The cytotoxic activity of the CTLs on the thirteenth day from the initiation of induction, prepared in item (8) of Example 6 was assayed in the same manner as in item (7) of Example 5, except that an E/T ratio of 90 was newly set. The results of the assay of cytotoxic activity are shown in Table 23.

[Table 23]

Table 23

| Target Cells | E/T | Cytotoxic Activity (%) | | |
|---|---|---|---|---|
| | | PBMCs | Control (Without Immobilization of CH-296) | CH-296 |
| T2 | 90 | 30.96 | N.T. | N. T. |
| | 30 | 9.13 | 23.04 | 15.78 |
| | 10 | 2.42 | 9.63 | 10.56 |
| | 3 | 1.94 | 3.06 | 3.62 |
| | 90 | 59.49 | N.T. | N.T. |
| T2+MART-1 | 30 | 41.37 | 25.46 | 7.53 |
| Peptide | 10 | 25.29 | 8.85 | 77.21 |
| | 3 | 7.75 | 11.26 | 56.18 |
| N. T.: not tested. | | | | |

As shown in Table 23, in the CTL population induced from the CH-296 group, the specific cytotoxic activity of the CTL population was high, as compared to those of the control group and the PBMC group. In other words, it was clarified that the cultured cells in which a culture equipment immobilized with CH-296 at an early stage of the expansion of the T cell population was used could be induced to a CTL population having an even higher specific cytotoxic activity.

Example 7: Induction of Anti-MART-1 CTLs from CD45RA$^+$ CCR7$^+$ CD8$^+$ T Cells and CD45RA$^-$ CCR7$^-$ CD8$^+$ T Cells Derived from Expanded Cells

(1) Isolation of CD45RA$^+$ CCR7$^+$ CD8$^+$ T Cells and CD45RA$^-$ CCR7$^-$ CD8$^+$ T Cells

The cells prepared in item (1) of Example 1 and those prepared in item (7) of Example 6 were stained in the same manner as in item (2) of Example 2. Thereafter, the cells were washed with 1% BSA/PBS, and suspended in an IMDM medium (manufactured by Invitrogen). The cells were subjected to a high-performance cell sorter Moflo, and a CD45RA$^+$ CCR7$^+$ CD8$^+$ fraction and a CD45RA$^-$ CCR7$^-$ CD8$^+$ fraction were respectively isolated, whereby obtaining a fraction having 92 to 99% purity.

(2) Induction of CTLs Specific to Antitumor-Associated Antigen (MART-1)

Using the cells prepared in item (1) of Example 7, the induction of CTLs specific to antitumor-associated antigen (NIART-1) was carried out in the same manner as in item (8) of Example 6, and the culture was continued for thirteen days, provided that the modifications were made as follows: The features include at the initiation of culture, culture initiating cells and antigen presenting cells were added in a volume of 0.25 mL each to a 48-well culture plate (manufactured by Becton Dickenson); and on the second day from the initiation of culture, 0.5 mL of 5HRPMI containing 60 U/mL IL-2 was put to each well, and on the fourth day from the initiation of culture, a half the volume of the culture supernatant was removed and 0.5 mL of 5HRPMI containing 60U/mL IL-2 was added to each well.

(3) Determination of Expansion Fold

As to the obtained in item (2) of Example 7, on the thirteenth day from the initiation of culture, the number of living cells was counted by trypan blue staining and an fold was calculated by comparing the number of counted cells to the number of cells at the initiation of culture. The are shown in Table 24.

[Table 24]

Table 24

| Culture Initiating Cells | Expansion Fold | |
|---|---|---|
| | Control (Without Immobilization of CH-296) | CH-296 |
| CD45RA$^+$ CCR7$^+$ CD8$^+$ T Cells | $\times$ 1.2 | $\times$ 7.8 |
| CD45RA$^-$ CCR7$^-$ CD8$^+$ T Cells | $\times$ 0.8 | $\times$ 1.2 |

As shown in Table 24, in the CTL population induced using CD45RA$^+$ CCR7$^+$ CD8$^+$ T cells isolated from cultured cells in which a culture equipment immobilized with CH-296 at an early stage of the expansion of the T cell population was used (hereinafter referred to as "the CH-296 group"), the expansion fold of the CTL population was high, as compared to that of the control group. In other words, it was clarified that the CTL population was obtained in a larger amount by carrying out the induction of CTLs from the CD45RA$^+$ CCR7$^+$ T cells contained in a high percentage in cultured cells in which a culture equipment immobilized with CH-296 at an early stage of the expansion of the T cell population was used.

(4) Assay of Cytotoxic Activity

The cytotoxic activity of the CTLs on the thirteenth day from the initiation of induction, prepared in item (2) of Example 7 was assayed in the same manner as in item (7) of Example 5. The results for the assay of cytotoxic activity are shown in Table 25.

[Table 25]

Table 25

| Culture Initiating Cells | Target Cells | E/T | Cytotoxic Activity (%) | | |
|---|---|---|---|---|---|
| | | | PBMCs | Control (Without Immobilization of CH-296) | CH-296 |
| CD45RA$^+$ CCR7$^+$ CD8$^+$ T Cells | T2 | 10 | 8.35 | N. T. | 18.62 |
| | | 3 | <0 | 1.77 | 9.8 |
| | T2 + MART-1 Peptide | 10 | 83.59 | N. T. | 89.49 |
| | | 3 | 66.61 | 20.92 | 67.07 |
| CD45RA$^-$ CCR7$^-$ CD8$^+$ T Cells | T2 | 30 | 17.16 | N. T. | N.T. |
| | | 10 | 3.19 | N. T. | N. T. |
| | | 3 | 0.78 | 10.15 | 8.96 |
| | T2+MART-1 Peptide | 30 | 8.66 | N.T. | N. T. |
| | | 10 | <0 | N. T. | N. T. |
| | | 3 | <0 | 9.06 | 3.21 |
| N. T.: Not tested. | | | | | |

As shown in Table 25, in the CTL population induced using the CD45RA$^+$ CCR7$^+$ CD8$^+$ T cells isolated from the CH-296 group, the cytotoxicity activity of the CTL population was high, as compared to those of the control group and the PBMC group. Further, the specific cytotoxic activity was high as compared to that of the CTL population induced using the CD45RA$^-$ CCR7$^-$ CD8$^+$ T cells. In other words, it was clarified that the CD45RA$^+$ CCR7$^+$ CD8$^+$ T cells which are contained in a high percentage in the cultured cells in which a culture equipment immobilized with CH-296 at an early stage of the expansion of the T cell population was used could be induced to a CTL population of which specific cytotoxic activity was even higher.

Example 8: Determination of Antigen-Recognizing Ability of CTLs

(1) Induction of CTLs Specific to Antitumor-Associated Antigen (MART-1)

Using the cells prepared in item (1) of Example 1 and those in item (7) of Example 6, the induction of CTLs specific to antitumor-associated antigen (MART-1) was carried out in the same manner as in item (8) of Example 6, and the culture was continued for fourteen days.

(2) Determination of Antigen-Recognizing Ability

The antigen recognizing ability of the CTLs on the fourteenth day from the initiation of induction, prepared in item (1) of Example 8 was determined by partly modifying a method of Valmori, D. et al [Valmori D. et al., J. Immunol., 160, 1750-1758 (1998)]. In other words, T2 cells were suspended in RPMI 1640 medium containing 5% FBS so as to have a density of $1 \times 10^6$ cells/mL, calcein-AM was then added thereto so as to have a final concentration of 25 $\mu$M, and the cells were cultured at 37˚C for 1 hour. The incubated cells were with a medium without containing calcein-AM, and the density was adjusted to $2 \times 10^5$ cells/mL. The calcein-labeled target cells were dispensed in each well of a 96-well cell culture plate in a volume of 50 $\mu$L/well each. To each well into which these cells were put 5HRPMI containing a 0 to 20 $\mu$M antigen peptide (melanoma antigen MART-1-derived epitope peptide) in a volume of 50 $\mu$L/well each. The above-mentioned plate was incubated in a 5% $CO_2$ incubator at 37˚C for 1 hour. To each well after the incubation was added a cell suspension containing a 30 fold-amount of K562 cells (adjusted to a density of $6 \times 10^6$ cells/mL) in a volume of 50 $\mu$L/well each.

The CTLs prepared in item (1) of Example 8 were adjusted with 5HRPMI so as to have a density of $6 \times 10^6$ cells/mL as effector cells, and thereafter to each well of the plate the cells were added in a volume of 50 $\mu$L/well each. The plate to which the above-mentioned cell suspension was put was centrifuged at $400 \times g$ for 1 minute, and the cells were then incubated in a 5% $CO_2$ incubator at 37˚C for 4 hours. Thereafter, 100 $\mu$L of the culture supernatant was collected from each well, and the amount of calcein released into the culture supernatant was determined with the fluorescence plate reader (spectrofluorometer) (485 nm/535 nm). The "specific cytotoxic activity (%)" was calculated in accordance with the formula (2) mentioned above. The results of the assay of antigen-recognizing ability are shown in Table 26. The antigen-recognizing ability was expressed as a relative value of the cytotoxic activity in each concentration of peptide added when the cytotoxic activity at a concentration of peptide added to the target cells of 10 $\mu$M was assumed to be 100.

[Table 26]

Table 26

| Final Concentration of Peptide Added to | Relative Value to Cytotoxic Activity at 10 $\mu$M Added Peptide | | |
|---|---|---|---|
| Target Cells ($\mu$M) | PBMCs | Control (Without Immobilization of CH-296) | CH-296 |
| 10.0 | 100 | 100 | 100 |
| 1.0 | 124.36 | 74.39 | 91.44 |
| 0.1 | 72.54 | 67.59 | 87.02 |
| 0.01 | 72.77 | 57.34 | 78.88 |
| 0.001 | 29.63 | 36.83 | 74.3 |
| 0.0001 | 18.39 | 9.8 | 41.72 |
| 0 | 16.24 | 5.69 | 5.16 |

As shown in Table 26, in the CTL population induced from the CH-296 group, even target cells with antigen peptide added of an even lower concentration were killed, as compared to those of the control group and the PBMC group, so that a specific antigen-recognizing ability of the CTL population was high. In other words, it was clarified that the cultured cells in which a culture equipment immobilized with CH-296 at an early stage of the expansion of the T cell population was used could be induced to a CTL population having an even higher specific antigen-recognizing ability.

Example 9: Viability Test of Cell Population of Expansion of T Cell Population Using CH-296
(1) Expansion of T Cell Population
The procedures were carried out in the same manner as in item (1) of Example 3 except that an acetate buffer (pH 5.3) was used in the immobilization of an anti-human CD3 antibody and a CH-296 fragment, and that on the fourth day from the initiation of culture, the culture medium was diluted about 14-fold, and 6.3 ml of the dilution was placed in a fresh 25 cm$^2$-culture flask, and on the eighth day from the initiation of culture, the culture medium was diluted about 2 fold, and 6.3 ml of the dilution was transferred to a fresh 25 cm$^2$-culture flask. The results are shown in Table 27.

[Table 27]

Table 27

| | Expansion Fold (folds) |
|---|---|
| Control (Without Immobilization of CH-296) | $\times$ 277 |
| CH-296 | $\times$ 344 |

As shown in Table 27, in the group in which a culture equipment immobilized with the CH-296 fragment (hereinafter referred to as "the CH-296 group") at an early stage of the expansion of the T cell population was used, a high expansion fold was obtained as compared to that of the control.

(2) Analysis of CD45RA$^+$ CCR7$^+$ T Cells and CD45RA$^+$ CD62L$^+$ T Cells
The cells prepared in item (1) of Example 9 were stained with each antibody and analyzed in the same manner as in item (4) of Example 1, provided that the combinations of the antibodies were as follows. Concretely, staining was carried out with FITC-labeled mouse IgG1/RD1-labeled mouse IgG1/PC5-labeled mouse IgG1 and FITC-labeled mouse anti-human CCR7 antibody/RD1-labeled mouse anti-human CD45RA antibody/PC5-labeled mouse anti-human CD62L antibody. These stained cells were analyzed with a flow cytometer, and percentages of CD45RA$^+$ CCR7$^+$ T cells and CD45RA$^+$ CD62L$^+$ T cells were calculated. The results are shown in Tables 28 and 29.

[Table 28]

Table 28

| | CD45RA$^+$ CCR7$^+$ T Cells |
|---|---|
| Control (Without Immobilization of CH-296) | 27.2 |
| CH-296 | 60.5 |

[Table 29]

Table 29

| | CD45RA$^+$ CD62L$^+$ T Cells (%) |
|---|---|
| Control (Without Immobilization of CH-296) | 71.3 |
| CH-296 | 84.0 |

From the results of Tables 28 and 29, in the CH-296 group, the results of higher CD45RA$^+$ CCR7$^+$ T cell population and higher CD45RA$^+$ CD62L$^+$ T cell population were obtained, as compared to those of the control group.

(3) Culture Using Feeder Cells of Cells of Item (1)

The cells after expansion obtained in item (1) of Example 9 were adjusted with 5HRPMI so as to have a density of 2 $\times$ 10$^6$ cells/mL, and the cell suspension was put to a 24-well cell culture plate in a volume of 0.5 mL/well each. In addition, the self PBMCs prepared in item (1) of Example 1 were suspended in 5HRPMI, the suspension was then irradiated with X-rays (0.90 C/kg), and re-suspended in 5HRPMI so as to have a density of 2 $\times$ 10$^6$ cells/mL (to give feeder cells). These feeder cells were added to the above-mentioned 24-well cell culture plate in a volume of 0.5 mL/well each, and the plate was cultured in a 5% CO$_2$ incubator at 37˚C for 7 days. During this period, the culture was carried out without adding any cytokines such as IL-2 at all.

(4) Analysis of Annexin V$^+$ 7AAD$^+$ Cells

The cells obtained in item (3) of Example 9 were collected, and stained in accordance with the protocol of Annexin V/7AAD Kit (manufactured by Beckman Coulter) in order to determine the cells which underwent apoptosis. The cells were subjected to flow cytometry, and a percentage of the Annexin V$^+$ 7AAD$^+$ cells, i.e. cells which underwent apoptosis for each of the cell population. The results are shown in Table 30.

[Table 30]

Table 30

| | Annexin V$^+$ 7AAD$^+$ cells (%) |
|---|---|
| Control (Without Immobilization of CH-296) | 57.6 |
| CH-296 | 44.9 |

It was shown from the results of Table 30, in the group in which a culture equipment immobilized with CH-296 at an early stage of the expansion of the T cell population was used, the Annexin V$^+$ 7AAD$^+$ cell population was lower, so that the percentage of apoptotic cells was lower, as compared to those of the control group. In other words, it was clarified that cells having high viability can be dominantly proliferated, even under the environment of, for example, a low concentration IL-2 by using a CH-296 fragment in the expansion of the T cell population.

Example 10: Studies on Effects of T Cell Transfer Using Model of Syngeneic Tumor in Mouse

In order to confirm the effects on the tumors of T cells subjected to expansion using CH-296, a test was conducted using a model of syngeneic tumor in mouse.

(1) Preparation of Mouse CH-296

The mouse CH-296 as shown in SEQ ID NO: 23 of Sequence Listing was obtained by designing a mouse fibronectin fragment based on a sequence of human CH-296, and constructing a plasmid in accordance with a conventional method, thereby obtaining the mouse CH-296 in a genetic engineering manner using the plasmid. In other words, *Escherichia coli* HB101 harboring the plasmid was cultured, and subjected to induction for expression. Thereafter, the cells were disrupted to prepare crude proteins, and the proteins were then subjected to purification with a cation exchanging column, an anion exchanging column, or a gel filtration column to give an intended protein. The obtained protein was subjected to bacterial filtration, and thereafter stored at -80˚C until use.

(2) Preparation of Splenic Lymphocytes from Cancer-Bearing Mouse

IMC carcinoma (hereinafter referred to as "IMC") was implanted to the abdominal cavity of female CDF$_1$ mice (manufactured by Nippon SLC, Inc.) to generate ascites, and the ascites were implanted to different mice every 7 days, and subcultured. Ascites on the seventh day from the subculture were collected, washed with PBS, and then suspended in PBS so as to have a density of $5 \times 10^7$ cells/mL. This cell was implanted subcutaneously to the side of the abdomen of $CDF_1$ mice in a volume of 0.1 mL, to form a solid tumor. After 21 days, the spleen was excised, and homogenized in RPMI 1640 medium using a slide glass. A portion of seven mice was collected together in a tube using RPMI 1640 medium to make up a volume of 45 mL, and the mixture was allowed to stand on ice for 5 minutes. Thereafter, the mixture was transferred to a fresh tube through a 40 $\mu$m Cell Strainer (manufactured by Becton Dickenson). After centrifugation, the supernatant was removed, and the residue was subjected to hemolysis including the procedural steps of suspending the residue in 2 mL of ACK buffer (0.15 M $NH_4Cl$, 0.01 M $KHCO_3$, 0.01 mM $Na_2EDTA$, pH 7.4), further adding 2 mL of the ACK buffer thereto to suspend the mixture, and thereafter adding RPMI 1640 medium so as to make up a volume of a cell suspension of 50 mL. After centrifugation, the supernatant was removed, the residue was suspended in 10 mL of the RPMI 1640 medium, and the suspension was transferred to a fresh tube through the Cell Strainer. The RPMI 1640 medium was added so as to make up a volume of a cell suspension of 40 mL. Thereafter, the cell suspension was centrifuged, the supernatant was removed, and thereafter the residue was suspended in a mixture prepared by mixing equivolumes of the RPMI 1640 medium and CP-1 containing 8% HSA, and the suspension was stored in liquid nitrogen until use.

(3) Immobilization of Anti-Mouse CD3 Antibody and Mouse CH-296 Fragment

An anti-mouse CD3 antibody and a mouse CH-296 fragment were immobilized to a culture equipment used in the following experiment. Concretely, 400 $\mu$L each of acetate buffer (pH 5.3) containing an anti-mouse CD3 antibody (manufactured by R & D Systems) (final concentration: 14 $\mu$g/mL) was added to the 24-well cell culture plate, and the culture equipment was incubated overnight at 4°C. Thereafter, the mouse CH-296 prepared in item (1) of Example 10 was added so as to have a final concentration of 25 $\mu$g/mL, and the culture equipment was incubated at room temperature for additional 5 hours. Immediately before use, acetate buffer containing the antibody and the mouse CH-296 was removed by aspiration from these culture equipments, and thereafter each well was washed twice with PBS, and then once with the RPMI 1640 medium, and the culture equipment was subjected to each experiment.

(4) Purification of Splenic Lymphocytes with Nylon Fibers

The splenic lymphocytes prepared in item (2) of Example 10 was purified using nylon fibers in order to increase the purity of the lymphocytes. A 10 mL syringe (manufactured by TERUMO CORPORATION) was filled with 0.6 g of nylon fibers (manufactured by Wako Pure Chemical Industries, Ltd.), equilibrated with PBS, and then sterilized at 121°C for 20 minutes. This column was equilibrated with RPMI 1640 medium containing 10% FBS (manufactured by DAINIPPON PHARMACEUTICAL CO., LTD.), and incubated at 37°C in a 5% $CO_2$ incubator for 1 hour. The splenic lymphocytes prepared in item (2) of Example 10 was suspended in 2 to 3 mL of the RPMI 1640 medium containing 10% FBS in a density so as not to exceed $2 \times 10^8$ cells, the suspension was applied to the column, and thereafter the column was incubated at 37°C in a 5% $CO_2$ incubator for 1 hour. Fifteen milliliters of RPMI 1640 medium containing 10% FBS previously warmed to 37°C was added to the column, and the eluted cells were collected.

(5) Expansion of Mouse T Cell Population

The lymphocytes prepared in item (4) of Example 10 were suspended in RPMI 1640 medium containing 10% FBS, 0.1 mM NEAA mixture, 1 mM sodium pyruvate, and 50 $\mu$M 2-mercaptoethanol (manufactured by Nakalai Tesque, Inc.) (hereinafter referred to as "mRPMI medium"), so as to have a density of $1.5 \times 10^6$ cells/mL. Thereafter, the mRPMI medium was previously added to the plate immobilized with the anti-mouse CD3 antibody and the mouse CH-296 prepared in item (3) of Example 10 in a volume of 0.7 mL/well, the above cell suspension was added thereto in a volume of 0.5 mL/well each, and these plates were cultured at 37°C in 5% $CO_2$ (zeroth day of culture). On the day of the culture, the cell suspension was diluted using the mRPMI medium so as to have a density of $1 \times 10^5$ cells/mL, and an entire amount was transferred to a fresh 75 $cm^2$-cell culture flask to which nothing was immobilized. At this time, mouse IL-2 (manufactured by R & D Systems) was added so as to have a final concentration of 100 U/mL, or mouse IL-7 (manufactured by R & D Systems) was added so as to have a final concentration of 10 ng/mL. On the fifth day of the culture, subculture was carried out in the same manner as that on the second day of the culture except that the cell suspension was diluted so as to have a density of $1.5 \times 10^6$ cells/mL. On the seventh day of the culture, the cells were collected, and subjected to a test with the following model of syngeneic tumor.

(6) Evaluation of Tumor Rejecting Action of T Cell Population Transfer in Model of Syngeneic Tumor of $CDF_1$-IMC

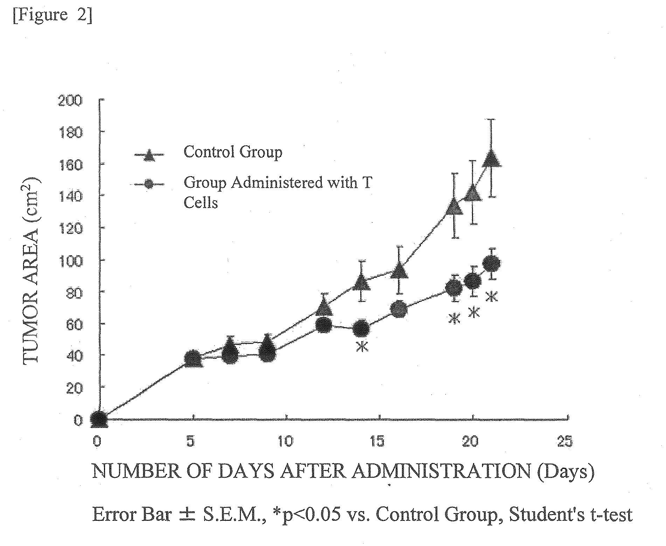

The IMC was implanted subcutaneously to the right side of the abdomen of 6-week old female $CDF_1$ mice under anesthesia in the same manner as in item (2) of Example 10. The cells prepared in item (5) of Example 10 were suspended in PBS so as to have a density of $3 \times 10^8$ cells/mL, and the suspension was administered from the veins of the mouse tails in an amount of 0.1 mL (zeroth day of administration). Mouse IL-2 ($3 \times 10^4$ U/0.2 mL) was administered to the abdominal cavity for four consecutive days from the zeroth day of administration. As a control, a group without administration of the expanded cells was set. The size of the tumor was expressed as a tumor area ($cm^2$) by regularly determining length and width of the tumor up to 21 days after the implantation of IMC. The results are shown in Figure 2. Figure 2 is a graph showing the number of days from implanting the IMC and the size of the tumor, wherein solid triangles denote the control group, and the solid circles denote the group administered with T cells. As shown in Figure 2, it was found that the group administered with the cells that were cultured in the presence of CH-296 (the group administered with T cells) significantly suppresses the formation of tumor.

<u>Example 11: Evaluation of Human T Cell Expanded Using CH-296 by GVHD Induction in NOD/Scid Mice</u>

(1) Expansion of T Cell Population

In order to the cells in a large amount, a culture equipment was Concretely, 21 mL of PBS containing an anti-human CD3 antibody (final concentration: 5 μg/mL) was added to a 175 $cm^2$-culture flask (manufactured by Becton Dickenson). At this time, CH-296 was added to the group with addition of CH-296, so as to have a final concentration of 25 μg/mL, the culture was incubated at room temperature for 5 hours. Immediately before use, PBS containing the antibody and CH-296 removed by aspiration from these culture equipments, and thereafter each flask washed with PBS and once with the RPMI medium. Fresh PBMCs were by collecting 54 mL of blood from human normal individual The PBMCs were suspended in GT-T503 medium containing 0.5% self plasma and 0.2% HSA (hereinafter referred to as "0.5% self plasma GT-T503"), so as to have a density of $0.5 \times 10^6$ cells/mL, the was added in a volume of 21 mL each to a flask immobilized with an anti-CD3 antibody, or a flask immobilized with an anti-CD3 antibody and CH-296, which was previously prepared, and IL-2 was added thereto so as to have a final concentration of 1000 U/mL. These flasks were initiated to be cultured at 37˚C in 5% $CO_2$ (zeroth day of culture). On the following day, the 0.5% self plasma GT-T503 was added to the flask in a volume of 49 mL each, and IL-2 was added to the culture medium so as to have a final concentration of 1000 U/mL based on the culture medium. On the fourth day from the initiation of culture, 42 mL of the culture medium of each group and 158 mL of the 0.5% self plasma GT-T503 were transferred to a gas-permeable culture bag (600 $cm^2$, manufactured by TAKARA BIO, INC, commercial product code: KB610) to which nothing was immobilized. Further, IL-2 was added thereto so as to have a final concentration of 500 U/mL, and the culture was continued. On the sixth day from the initiation of culture, the 0.5% self plasma GT-T503 in a volume of 400 mL and IL-2 were added thereto so as to have a final concentration of IL-2 of 500 U/mL (600 mL culture medium). After two days, a half volume of the culture medium of the 600 mL culture medium was removed, and the 0.5% self plasma GT-T503 of the same volume and IL-2 (final concentration: 500 U/mL) were added thereto (eighth day of culture). On the eleventh day from the initiation of culture, GT-T503 medium containing 0.2% HSA in a volume of 600 mL and IL-2 were added so as to have a final concentration of IL-2 of 500 U/mL, and the culture was continued up until the fourteenth day. The cells after the culture were suspended in a storage liquid composed of equivolumes of CP-1 containing 8% HSA and a RPMI 1640 medium, and the suspension was stored in liquid nitrogen until use.

(2) Grouping

On the day before the administration of the cells, the body weights of ten 8-week old female NOD/scid mice (produced from CLEA Japan, Inc.) were measured, and grouped into 4 groups. The group members in this Example were as follows.

Group A: Solvent + Feeder + Human IL-2 + Anti-Asialo GM1 Antibody
Group B: PBLs + Feeder + Human IL-2 + Anti-Asialo GM1 Antibody
Group C: Cells after Expansion (Without Immobilization of CH-296) + Feeder + Human IL-2 + Anti-Asialo GM1
Group D: Cells after Expansion (with Immobilization of CH-296) + Feeder + Human IL-2 + Anti-Asialo GM1
Group A and Group B satisfied n = 2, and Group C and Group D satisfied n=3.

(3) Administration of Anti-Asialo GM1 Antibody

It has been known that the treatment with an anti-asialo GM1 antibody serves to remove NK cells in NOD/scid mice, thereby enhancing engraftment of human cells. On the day before the administration of the expanded cells, 20 μL of an anti-asialo GM1 antibody (manufactured by Wako Pure Chemicals Industries, Ltd.) was diluted with physiological saline containing 0.4% HSA (hereinafter simply referred to as "0.4% HSA-added physiological saline"), and a 0.4 mL portion was administered to the abdominal cavity.

(4) Preparation of Administered Cells and Administration

The expanded cells which were freeze-stored in item (1) of Example 11 and the freeze-stored PBMCs of the same donor prepared in the same manner as in item (1) of Example 1 were rapidly melted in a water bath at 37˚C. The concentration of peripheral blood lymphocytes (hereinafter referred to as "PBLs") was calculated by assuming that a percentage of the CD3-positive cells of the PBMCs was 70%. Using a 4% HSA-added physiological as a solvent, a part of PBLs was suspended in the solvent as feeder cells in an amount of $0.5 \times 10^7$ cells/mouse, and PBLs and the cells after the expansion were each suspended in the solvent in an amount of $1.0 \times 10^8$ cells/mouse so as to each have a necessary cell count, as cells for administration. All the mice were irradiated with X-rays (0.090 C/kg) before the administration of the cells, and 0.3 mL of the furnished cells were administered into the abdominal cavity, starting sequentially from Group A.

(5) Administration of Human IL-2

The human IL-2 used upon the expansion of the T cells was prepared into a solution with 0.4% HSA-added physiological saline so as to have a concentration of $2 \times 10^4$ U/mouse, and 0.2 mL of the solution was administered to the abdominal cavity of the mice starting from those mice to which administration of the cells in item (4) of Example 11 was finished.

The human IL-2 was administered once a day for 4 consecutive times from the day of the administration of the cells.

(6) Change with Time in Body Weight of Mice After Administration of Cells

The body weights were determined in an appropriate interval from the day of the administration to the twenty-first day. As a result, in Group B administered with PBLs, a weight loss was observed from the eighth day, and one mouse died on the thirteenth day. In the other Groups, no mice died up until the twenty-first day, and all cases survived. When a percentage of weight loss was observed, although some fluctuations were found in Group A and Group C during the course of administration, the original body weight was maintained on the twenty-first day, and in Group D, a slight weight loss tendency was observed. In Xeno-GVHD reaction in which weight loss is used as an index, it was shown that the reaction was dominantly found in PBLs, showing that the expanded cells under the conditions of immobilization with CH-296 were more effective than those under the conditions of without the immobilization.

(7) Engraftment of Human T Cells in Spleen

The Groups other than Group B were subjected to excision of the spleen upon anatomy on the twenty-first day from the initiation of the administration of the cells, and a percentage of the human CD3-positive cells in the spleen was analyzed by flow cytometry. Since one mouse died on the thirteenth day in Group B, this mouse was subjected to excision of the spleen immediately after the death, and the remaining one mouse was also autopsied on the thirteenth day and subjected to excision of the spleen. The excised spleen was homogenized with a slide glass, and the homogenized spleen was filtered with a nylon mesh and subjected to a centrifugal procedure. The supernatant was removed, the residue was hemolyzed with the ACK buffer, and thereafter washed with the RPMI 1640 medium, to prepare cells. The staining was carried out using a FITC-labeled mouse anti-human CD3 antibody (manufactured by DAKO DENMARK A/S). As a result, it was shown that the percentage of the CD3-positive cells of the splenic cells in Group B analyzed on the thirteenth day was about 70%, and that in Group D analyzed on the twenty-first day was about 59%. The percentage of positive cells was less than 1% in Group A, and several percent even in Group C. It could be seen from the results that the cells expanded under the conditions of the immobilization with CH-296 had a high grafting percentage in the spleen as compared to those under the conditions without the immobilization, and increased the percentage for 21 days.

Example 12: Expansion of Lymphocytes with Addition of IL-2, IL-12, IFN-γ, or Anti-IL-4 Antibody

(1) Immobilization of Anti-Human CD3 Antibody and CH-296 Fragment

The same procedures as in item (2) of Example 1 were carried out, except that an ACD-A solution (pH 5.0) was used in the immobilization of the anti-human CD3 and the CH-296 fragment, and that the amount of the ACD-A solution added to the 12-well cell culture plate was changed to 0.45 mL.

(2) Expansion of T Cell Population Using GT-T503 Medium

PBMCs prepared in item (1) of Example 1 were suspended in a 0.5% GT-T503 so as to have a density of 0.25 $\times$ 10$^6$ cells/mL, and thereafter a 0.5% GT-T503 was previously added to the plate immobilized the anti-human CD3 antibody, or the plate immobilized with the anti-human CD3 antibody and CH-296, prepared in item (1) of Example 12 in a volume of 0.5 mL/ well. The cell suspension was added in a volume of 1 mL/well each, and these plates were cultured at 37˚C in 5% CO$_2$ (zeroth day of culture). On the fourth day from the initiation of culture, the culture medium of each group was diluted about 14-fold with the 0.5% GT-T503, and 6.3 mL of the dilution was transferred to a fresh 25 cm$^2$-cell culture flask to which nothing was immobilized. The culture was continued, and on the seventh day, the culture medium of each group was diluted about 2-fold with the 0.5% GT-T503, and 6.3 mL of the dilution was transferred to a fresh 25 cm$^2$-cell culture flask to which nothing was immobilized. On the eleventh day from the initiation of culture, the cell culture medium of each group was diluted about 2-fold with GT-T503 medium containing 0.2% HSA, and 12.6 mL of the dilution was respectively transferred to a fresh 25 cm$^2$-cell culture flask to which nothing was immobilized. Here, IL-2 was added from the zeroth day of culture so as to have a final concentration of 100 U/mL. In addition, as to agents other than IL-2, on the fourth day of culture, IL-12 (manufactured by R & D Systems) was added so as to have a final concentration of 50 U/mL, IFN-γ (manufactured by R & D Systems) was added so as to have a final concentration of 20 ng/mL, or an anti-human IL-4 antibody (manufactured by Becton Dickenson) was added so as to have a final concentration of 2 μg/mL; on the seventh day and the eleventh day of culture, each cytokine and antibody was added to the freshly added medium so as to the above-mentioned final concentration. On the fourteenth day from the initiation of culture, the number of live cells was counted by means of trypan blue staining method, and the expansion fold was calculated in comparison of the number of the counted cells with the number of cells at the initiation of culture. The results are shown in Table 31.

[Table 31]

Table 31

| | Expansion Fold (folds) |
|---|---|
| Control (Without Immobilization of CH-296) | $\times$ 388 |
| CH-296 | $\times$ 416 |

As shown in Table 31, in the group in which a culture equipment immobilized with CH-296 at an early stage of the T cell expansion was used, the expansion fold of the T cell population was high, as compared to that of the control group.

(3) Analysis of CD45RA$^+$ CCR7$^+$ T Cells and CD45RA$^+$ CD62L$^+$ T Cells

The cells prepared in item (2) of Example 12 were stained with each antibody and analyzed in the same manner as in item (4) of Example 1, provided that the combinations of the antibodies were as follows. Concretely, staining was carried out with FITC-labeled mouse IgG1/RD1-labeled mouse IgG1/PC5-labeled mouse IgG1+ECD-labeled mouse IgG1 as a negative control, RD1-labeled mouse anti-human CD45RA antibody/FITC-labeled mouse anti-human CCR7 antibody/ECD-labeled mouse anti-human CD4 antibody/PC5-labeled mouse anti-human CD8 antibody, or RD1-labeled mouse anti-human CD45RA antibody/FITC-labeled mouse anti-human CD62L antibody/ECD-labeled mouse anti-human CD4 antibody/PC5-labeled mouse anti-human CD8 antibody. These stained cells were subjected to flow cytometry, percentages of CD45RA$^+$ CCR7$^+$ T cells and CD45RA$^+$ CC62L$^+$ T cells in the entire region of T cells, in the region of CD8$^+$ T cells, or in the region of CD4$^+$ T cells were calculated. The results are shown in Table 32.

[Table 32]

Table 32

| | | CD45RA$^+$ CCR7$^+$ T Cells (%) | CD45RA$^+$ CD62L$^+$ T Cells (%) |
|---|---|---|---|
| Entire T Cells | Control (Without Immobilization of CH-296) | 13.8 | 25.8 |
| | CH-296 | 40.2 | 58.4 |
| CD8$^+$ T Cells | Control (Without Immobilization of CH-296) | 17.1 | 33.4 |
| | CH-296 | 47.4 | 69.7 |
| CD4$^+$ T Cells | Control (Without Immobilization of CH-296) | 7.7 | 11.6 |
| | CH-296 | 21.8 | 25.0 |

As shown in Table 32, in the group in which a culture equipment immobilized with CH-296 fragment was used, the results of higher CD45RA$^+$ CCR7$^+$ T cell population and CD45RA$^+$ CD62L$^+$ T cell population in the T cells during the culture were obtained, as compared to those of the control group. It was clarified from the above in the culture in which IL-2, IL-12, IFN-$\gamma$, an anti-human IL-4 antibody was added to a GT-T503 medium that the T cell population could be expanded while increasing the percentage of CD45RA$^+$ CCR7$^+$ or CD45RA$^+$ CD62L$^+$ T cell population of T cells by allowing the CH-296 fragment to be coexistent at an early stage of the expansion.

Example 13: Allogeneic MLR of Cell Population Expanded Using Gas-Permeable Culture Bag After Stimulation at Early Stage of CH-296 from Freshly Separated PBMCs

(1) Separation of PBMCs from Fresh Blood

Fifty milliliters of blood was collected from human normal individual donor from which informed consent was obtained, and thereafter PBMCs were separated from the collected blood in the same manner as in item (1) of Example 1. The collected PBMCs were subjected to trypan blue staining and Turk's staining methods (Turk's stain solution: manufactured by Nakalai Tesque Inc.), thereby calculating the number of cells, and subjected to each experiment as they were without frozen storage.

(2) Immobilization of Anti-Human CD3 and CH-296 The immobilization was out in the same as in item (1) of Example 6.

(3) Expansion of T Cell Population

The PBMCs prepared in item (1) of Example 13 were suspended in 0.5% self plasma so as to have a density of $0.5 \times 10^6$ cells/mL to prepare a cell suspension. Thereafter, the 0.5% GT-T503 was previously added to a flask immobilized with the anti-human CD3 antibody, or a flask immobilized with the anti-human CD3 antibody and the CH-296, prepared in item (2) of Example 13, in a volume of 21 mL/flask, and the above cell suspension was added thereto in a volume of 9 mL/flask each. IL-2 was added thereto so as to have a final concentration of 1000 U/mL, and these flasks were subjected to culture at 37°C in 5% CO$_2$ (zeroth day of culture). On the fourth day after the initiation of culture, 21 mL of the culture medium of each group and 279 mL of 0.5% self plasma GT-T503 were transferred to a gas-permeable culture bag (300 cm$^2$, manufactured by TAKARA BIO, INC., commercial code: KB610) to which nothing was immobilized, and IL-2 was further added thereto so as to have a final concentration of 500 U/mL. The culture was continued, and on the eighth day, 150 mL of the cell culture medium of each group was removed from the culture bag, leaving 150 mL of the cell culture medium in the bag, and the remaining culture medium was diluted 2-fold with the 0.5% self plasma GT-T503, to make up a volume of 300 mL of the cell culture medium of each group. To each group was further added IL-2 so as to each have a final concentration of 500 U/mL. On the eleventh day from the initiation of culture, 300 mL of 0.5% GT-T503 medium containing 0.2% HSA was added thereto to dilute 2-fold the cell culture medium of each group. To each group was further added IL-2 so as to each have a final concentration of 500 U/mL. On the fourteenth day from the initiation of culture, the number of living cells was counted by trypan blue staining method, and an expansion fold was calculated by comparing the number of the counted cells with the number at the initiation of culture. The results are shown in Table 33.

[Table 33]

Table 33

|  | Expansion Fold (folds) |
| --- | --- |
| Control (Without Immobilization of CH-296) | $\times$ 885 |
| CH-296 | $\times$ 1271 |

As shown in Table 33, even in a case where the cells were cultured in a gas-permeable culture bag, in the group in which a culture equipment immobilized with CH-296 at an early stage of the expansion of the T cell was used (hereinafter referred to as "the CH-296 group"), the expansion fold of the T cell population was high as compared to that of the control group.

(4) Analysis of $CD45RA^+ CCR7^+$ T cells,
$CD45RA^+ CD62L^+$ T cells, $CD45RA^+ CD27^+$ T cells,
$CD45RA^+ CD28^+$ T cells, $CD27^+ CD28^+$ T cells, and
$CD45RA^+ CCR7^+ CD62L^+$ T cells in T Cell Population Cultured in Gas-Permeable Culture Bag
The cells prepared in item (3) of Example 13 were stained with each antibody and analyzed in the same as in item (3) of Example 6. The results are shown in Table 34.

[Table 34]

Table 34

|  | Control (Without Immobilization of CH-296) | CH-296 |
| --- | --- | --- |
| $CD45RA^+ CD62L^+$ T Cells | 77.15% | 85.21% |
| $CD45RA^+ CCR7^+$ T Cells | 50.46% | 60.17% |
| $CD45RA^+ CD28^+$ T Cells | 65.94% | 72.88% |
| $CD45RA^+ CD27^+$ T Cells | 70.93% | 85.89% |
| $CD28^+ CD27^+$ T Cells | 65.54% | 76.42% |
| $CD45RA^+ CCR7^+ CD62L^+$ T Cells | 50.05% | 59.32% |

As shown in Table 34, in the CH-296 group, high values were shown in any one of the cell surface markers, as compared to those of the control group.

(5) Storage of Cultured Cells
The cells on the fourteenth day of the culture prepared in item (3) of Example 13 were subjected to frozen storage and melting in the same manner as in item (4) of Example 5. The cells were subjected to each experiment.

(6) Allogeneic MLR
The allogeneic MLR was carried out in the same manner as in item (4) of Example 6 using the cells prepared in item (5) of Example 13, except the modifications were made as follows. The features include stimulator cells and responder cells were prepared so as to have a cell density of 1 or $2 \times 10^6$ cells/mL; and on the fifth day, the cells in each well were suspended, the suspension was then divided into two wells of a half the volume each, 1 mL of 5HRPMI containing 1000 U/mL IL-2 was added to each well, and the culture was continued until the seventh day.

(7) Determination of Expansion Fold
As to the cells obtained in item (6) of Example 13, on the seventh day from the initiation of culture, the number of living cells was counted by trypan blue staining method, and an expansion fold was calculated by comparing the number of counted cells to the number of cells at the initiation of culture. The results are shown in Table 35.

[Table 35]

Table 35

| Cultured | Number of Seeded | Responder Cells | |
|---|---|---|---|
| Days | Cells at Initiation of Culture Cells/well | Control (Without Immobilization with CH-296) | CH-296 |
| Seventh Day | $0.5 \times 10^6$ | $\times 1.29$ | $\times 2.24$ |
| | $1.0 \times 10^6$ | $\times 2.29$ | $\times 3.72$ |

As shown in Table 35, in a case where an allogeneic MLR was carried out using the CH-296 group, the expansion fold after the reaction was high as compared to that of the control group. In other words, it was clarified that when an allogeneic MLR was carried out using cultured cells in which a culture equipment immobilized with CH-296 at an early stage of expansion of T cell population was used, alloantigen recognizing cells are proliferated in an even larger amount.

(8) Assay of Cytotoxic Activity

The cytotoxic activity of the cells on the seventh day from the initiation of induction, prepared in item (6) of Example 13 was assayed in the same as in item (6) of Example 6, except that as the target cells upon the determination, scif-PBMCs or non-self-PBMCs which were subjected to blastogenesis (blasting) with PHA for 7 days were used as calcein-labeled target cells. During the assay for the cytotoxic activity, a 30-fold amount of K562 cells and calcein-labeled target cells were mixed. An E/T ratio was set at 90 to 3. The results for the assay of cytotoxic activity are shown in Table 36.

[Table 36]

Table 36

| Number of Seeded Target Cells Cells at Initiation of Culture | | E/T | Cytotoxic Activity (%) | |
|---|---|---|---|---|
| Cells/well | | | Control (Without Immobilization of CH-296) | CH-296 |
| $0.5 \times 10^6$ | | 90 | 33.18 | 49.5 |
| | Non-Self PHA | 30 | 17.17 | 28.31 |
| | Blast Cells | 10 | 6.13 | 10.81 |
| | | 3 | 3.66 | 6.12 |
| | | 90 | 2.01 | 2.78 |
| | Self PHA | 30 | <0 | 1.67 |
| | Blast Cells | 10 | <0 | <0 |
| | | 3 | 4.07 | 1.1 |
| $1.0 \times 10^6$ | | 90 | 69.5 | 73.42 |
| | Non-Self PHA | 30 | 53.68 | 66.57 |
| | Blast Cells | 10 | 30.29 | 43.79 |
| | | 3 | 11.78 | 18.82 |
| | | 90 | 3.72 | 4.21 |
| | Self PHA | 30 | 2.08 | 3.59 |
| | Blast Cells | 10 | < 0 | 1.14 |
| | | 3 | <0 | <0 |

As shown in Table 36, in a case where an allogeneic MLR was carried out from the CH-296 group, the alloantigen-specific cytotoxic activity after the reaction was high, as compared to that of the control group. In other words, in the cultured cells in which a culture equipment immobilized with CH-296 at an early stage of the expansion of the T cell population was used, it was clarified that a cell population having an even stronger alloantigen-specific cytotoxic activity could be obtained by the allogeneic MLR.

Example 14: Allogeneic MLR and Induction of MART-1 CTLs of Cell Population Expanded Using Gas-Permeable Culture Bag Upon Stimulation at Early Stage or Subsequent Culture of CH-296

(1) Immobilization of Anti-CD3 Antibody and CH-296 Fragment

An anti-human CD3 antibody and a CH-296 fragment were immobilized to a culture equipment used in the following experiment. Concretely, 9 mL each of PBS containing an anti-human CD3 antibody (final concentration: 5 $\mu$g/mL) was added to a gas-permeable culture bag (culture area:75 cm$^2$, manufactured by TAKARA BIO, INC., commercial code: KB620). Upon the addition, CH-296 was added to a group with addition of CH-296 the same volumes of PBS containing an anti-human CD3 antibody (final concentration: 5 $\mu$g/mL) and CH-296 (final concentration: 25 $\mu$g/mL).

After these culture equipments were incubated at room temperature for 5 hours, the culture equipments were stored at room temperature until use. Immediately before use, PBS containing the antibody and the CH-296 was removed with a syringe from these culture equipments, and thereafter each bag was washed twice with PBS, and then once with the RPMI medium, and the bags were subjected to each experiment.

(2) Expansion of T Cell Population

The same procedures as those in item (3) of Example 13 were carried out except that the bag prepared in item (1) of Example 14, not the culture flask, was used as a culture equipment upon stimulation with CH-296. The results on the fourteenth day after the initiation of culture are shown in Table 37.

[Table 37]

Table 37

|  | Expansion Fold (folds) |
| --- | --- |
| Control (Without Immobilization of CH-296) | $\times$ 500 |
| CH-296 | $\times$ 967 |

As shown in Table 37, even in a case where the cells were cultured using a gas-permeable culture bag upon stimulation at early stage or subsequent culture of CH-296, in the group in which a culture equipment immobilized with CH-296 at an early stage of the expansion of the T cell population was used (hereinafter referred to as "the CH-296 group"), the expansion fold of the T cell population was high as compared to that of the control group.

(3) Analysis of CD45RA$^+$ CCR7$^+$ T cells,
CD45RA$^+$ CD62L$^+$ T cells, CD45RA$^+$ CD27$^+$ T cells,
CD45RA$^+$ CD28$^+$ T cells, CD27$^+$ CD28$^+$ T cells, and
CD45RA$^+$ CCR7$^+$ CD62L$^+$ T cells in T Cell Population Cultured in Gas-Permeable Culture Bag Upon Stimulation at Early Stage or Subsequent Culture of CH-296

The cells prepared in item (2) of Example 14 were stained with each antibody and analyzed in the same manner as in item (3) of Example 6.

The results are shown in Table 38.

[Table 38]

Table 38

|  | Control (Without Immobilization of CH-296) | CH-296 |
| --- | --- | --- |
| CD45RA$^+$ CD62L$^+$ T Cells | 71.73% | 85.03% |
| CD45RA$^+$ CCR7$^+$ T Cells | 47.49% | 67.46% |
| CD45RA$^+$ CD28$^+$ T Cells | 59.81% | 79.83% |
| CD45RA$^+$ CD27$^+$ T Cells | 64.69% | 85.37% |
| CD28$^+$ CD27$^+$ T Cells | 59.99% | 82.15% |
| CD45RA$^+$ CCR7$^+$ CD62L$^+$ T Cells | 46.14% | 65.94% |

As shown in Table 38, in the CH-296 group, high values were shown in any one of the cell surface as compared to those of the control group.

(4) Allogeneic MLR

The allogeneic MLR was carried out in the same manner as in item (4) of Example 6 using the cells prepared in item (2) of Example 14.

(5) Assay of Cytotoxic Activity

The cytotoxic activity of the cells on the tenth day from the initiation of induction, prepared in item (4) of Example 14 was assayed in the same manner as in item (6) of Example 6, that an E/T ratio of 90 was newly set. The results for the assay of cytotoxic activity are shown in Table 39.

[Table 39]

Table 39

| Target Cells | E/T | Cytotoxic Activity (%) | |
| --- | --- | --- | --- |
| | | Control (Without Immobilization of CH-296) | CH-296 |
| Non-Self PRA Blast Cells | 90 | 65.05 | 72.73 |
| | 30 | 44.92 | 61.43 |
| | 10 | 21.81 | 37.2 |
| | 3 | 7.23 | 17.99 |
| Self PHA last Cells | 90 | 0.69 | 0.17 |
| | 30 | <0 | < 0 |
| | 10 | < 0 | <0 |
| | 3 | <0 | < 0 |

As shown in Table 39, in a case where an allogeneic MLR was out from the CH-296 the alloantigen-specific cytotoxic activities the reaction were high, as compared to those of the control group. In words, in the cultured cells in which a culture equipment immobilized with CH-296 at an early stage of the expansion of the T cell population was used, it was clarified that a cell having an even stronger alloantigen-specific cytotoxic activity by the allogeneic MLR could be obtained.

(6) Storage of Cultured Cells

The cells on the fourteenth day of the culture prepared in item (2) of Example 14 were subjected to frozen storage and melting in the same manner as in item (4) of Example 5. The cells were subjected to each experiment.

(7) Induction of CTLs Specific to Antitumor-Associated Antigen (MART-1)

Using the cells prepared in item (1) of Example 1 and item (6) of Example 14, the induction of CTLs specific to antitumor-associated antigen (MART-1) was carried out in the same manner as in item (5) of Example 5, and the culture was continued for fourteen days, provided that the modifications were made as follows: The features include the responder cells were suspended in 5HRPMI so as to have a density of from $1 \times 10^6$ cells/mL, and the suspension was added to a 24-well cell culture plate in a volume of 0.5 mL/well each; on the third day from the initiation of culture, a half the volume of the culture supernatant was removed, and 1 mL of 5HRPMI containing 60 U/mL IL-2 was then added to well; after one week, the cultured cells were adjusted to a density of from 1.5 to 3.0 $\times 10^6$ cells/mL, and the antigen presenting cells were adjusted to a density of $1.0 \times 10^6$ cells/mL, and each cell suspension was added in a volume of 0.5 mL/well; and on the tenth day from the initiation of culture, a half the volume of the culture supernatant was removed, and 1 mL of 5HRPMI containing 60 U/mL IL-2 was then added to each well.

(8) Assay of Cytotoxic Activity

The cytotoxic activity of the CTLs on the day from the initiation of induction, prepared in item (7) of Example 14 was assayed in the same manner as in item (9) of Example 6. The results of the assay of cytotoxic activity are shown in Table 40.

[Table 40]

Table 40

| Target Cells | E/T | Cytotoxic Activity (%) | | |
| --- | --- | --- | --- | --- |
| | | PBMCs | Control (Without Immobilization of CH-296) | CH-296 |
| T2 | 90 | 3.92 | <0 | <0 |
| | 30 | <0 | <0 | <0 |
| | 10 | <0 | <0 | <0 |
| | 3 | <0 | <0 | <0 |
| T2 + MART-1 Peptide | 90 | 59.86 | 61.55 | 65.59 |
| | 30 | 61.78 | 56.21 | 64.66 |
| | 10 | 44.21 | 43.64 | 51.79 |
| | 3 | 7.26 | 20.36 | 27.06 |

As shown in Table 40, in the CTL population induced from the CH-296 group, the specific cytotoxic activity of the CTL population was high, as compared to those of the control group and the PBMC group. In other words, it was clarified that the cultured cells in which a culture equipment immobilized with CH-296 at an early stage of the expansion of the T cell population was used could be induced to a CTL population having an even higher specific cytotoxic activity.

Example 15: Induction of Anti-MART-1 CTLs from CD45RA$^+$ CCR7$^+$ CD8$^+$ T Cells and CD45RA$^-$ CCR7$^-$ CD8$^+$ T Cells Derived from Expanded Cells Using Gas-Permeable Culture Bag Upon Stimulation at Early Stage or Subsequent Culture of CH-296

(1) Isolation of CD45RA$^+$ CCR7$^+$ CD8$^+$ T Cells and CD45RA CCR7$^-$ CD8$^+$ T Cells

The cells prepared in item (1) of Example 1 and those prepared in item (6) of Example 14 were isolated into a CD45RA$^+$ CCR7$^+$ CD8$^+$ fraction and a CD45RA$^-$ CCR7$^-$ CD8$^+$ fraction, respectively, in the same manner as in item (1) of Example 7, thereby obtaining a fraction having 93 to 99% purity.

(2) Induction of CTLs Specific to Antitumor-Associated Antigen. (MART-1)

Using the cells prepared in item (1) of Example 15, the induction of CTLs specific to antitumor-associated (MART-1) was carried out in the same manner as in item (7) of Example 14, and the culture was continued for fourteen days, provided that the modifications were made as follows: The features include the responder cells were suspended in 5HRPMI so as to have a density of from $1 \times 10^6$ cells/mL, and the suspension was added to a 48-well cell culture plate in a volume of 0.25 mL/well each; on the first day from the initiation of culture, 0.5 mL of 5HRPMI containing 60 U/mL IL-2 was added to each well; on the third day from the initiation of culture, a half the volume of the culture supernatant was removed, and 0.5 of 5HRPMI containing 60 U/mL IL-2 was then added to each well; after one week, the cultured cells were adjusted to a density of from 0.2 to $1.7 \times 10^6$ cells/mL, and the antigen presenting cells were adjusted to a density of $1.0 \times 10^6$ cells/mL, and each cell suspension was added to a 24-well cell culture plate in a volume of 0.5 mL/well; and on the tenth day from the initiation of culture, a half the volume of the culture supernatant was removed, and 1 mL of 5HRPMI containing 60 U/mL IL-2 was then added to each well.

(3) Determination of Expansion Fold

As to the cells obtained in item (2) of Example 15, on the fourteenth day the initiation of culture, the number of living cells was counted by trypan blue staining method, and an expansion fold was calculated by comparing the number of counted cells to the number of cells at the initiation of culture. The results are shown in Table 41.

[Table 41]

Table 41

| Culture Initiating Cells | Expansion Fold | |
| --- | --- | --- |
| | Control (Without Immobilization of CH-296) | CH-296 |
| CD45RA$^+$ CCR7$^+$ CD8$^+$ T Cells | ×2.36 | ×5.38 |
| CD45RA$^-$ CCR7$^-$ CD8$^+$ T Cells | × 3.14 | × 4.96 |

As shown in Table 41, in the CIL population induced using CD45RA$^+$ CCR7$^+$ CD8$^+$ T cells isolated from cultured cells in which a culture equipment immobilized with CH-296 at an early stage of the expansion of the T cell population was used (hereinafter referred to as "the CH-296 group"), the expansion fold of the CTL population was high, as compared to that of the control group. In other words, it was clarified that the CTL population was obtained in a larger amount by carrying out the induction of CTLs from the CD45RA$^+$ CCR7$^+$ T cells contained in a high percentage of cultured cells in which a culture equipment immobilized with CH-296 at an early stage of the expansion of the T cell population was used.

(4) Assay of Cytotoxic Activity

The cytotoxic activity of the cells on the fourteenth day from the initiation of induction, prepared in item (2) of Example 15 was assayed in the same as in item (9) of Example 6, except that an E/T ratio was set at from 30 to 3. The for the assay of cytotoxic activity are shown in Table 42.

[Table 42]

Table 42

| Culture Initiating Cells | Target Cells | E/T | Cytotoxic Activity (%) | | |
| --- | --- | --- | --- | --- | --- |
| | | | PBMCs | Control (Without Immobilization of CH-296) | CH-296 |
| CD45RA$^+$ CCR7$^+$ CD8$^+$ T Cells | T2 | 30 | <0 | N.T. | <0 |
| | | 10 | <0 | <0 | <0 |
| | | 3 | <0 | <0 | <0 |
| | | 30 | 56.77 | N.T. | 73.96 |
| | T2 + MART-1 Peptide | 10 | 45.7 | 64.91 | 73.62 |
| | | 3 | 35.36 | 58.58 | 58.49 |

(continued)

| CultureInitiating Cells | Target Cells | E/T | Cytotoxic Activity (%) | | |
|---|---|---|---|---|---|
| | | | PBMCs | Control (Without Immobilization of CH-296) | CH-296 |
| CD45RA⁻ CCR7⁻ CD8⁺ T Cells | T2 | 30 | < 0 | N.T. | < 0 |
| | | 10 | <0 | <0 | <0 |
| | | 3 | < 0 | < 0 | < 0 |
| | T2+MART-1 Peptide | 30 | 9.75 | N. T. | < 0 |
| | | 10 | < 0 | 1.5 | < 0 |
| | | 3 | < 0 | 1.5 | 3.87 |

N.T.: Not tested.

As shown in Table 42, in the CTL population using the CD45RA⁺ CCR7⁺ CD8⁺ T cells isolated from the group, the cytotoxicity activities of the CTL population high, as compared to those of the control group and the PBMC group. Further, the specific cytotoxic activities were high as compared to those of the CTL population induced using the CD45RA⁻ CCR7⁻ CD8⁺ T cells. In other words, it was clarified that the CD45RA⁺ CCR7⁺ CD8⁺ T cells which are contained in a high percentage in the cultured cells in which a culture equipment immobilized with CH-296 at an early stage of the expansion of the T cell population was used could be induced to a CTL population of which specific cytotoxic activities were even higher.

Example 16: Determination of Antigen-Recognizing Ability of CTLs

(1) Induction of CTLs Specific to Antitumor-Associated Antigen (MART-1)

Using the cells prepared in item (1) of Example 1 and item (6) of Example 14, the induction of CTLs specific to antitumor-associated antigen (MART-1) was carried out in the same manner as in item (7) of Example 14, and the culture was continued for fifteen days.

(2) Determination of Antigen-Recognizing Ability

The antigen recognizing ability of the CTLs on the fifteenth day from the initiation of induction, prepared in item (1) of Example 16 was determined in the same manner as in item (2) of Example 8. The results of the determination of antigen-recognizing ability are shown in Table 43. The antigen-recognizing ability was expressed as a relative value of the cytotoxic activity in each added peptide concentration when the cytotoxic activity at a concentration of a peptide added to the target cells of 10 $\mu$M was assumed to be 100.

[Table 43]

Table 43

| Final Concentration of Peptide Added to | Relative Value to Cytotoxic Activity at 10 $\mu$M Added Peptide | | |
|---|---|---|---|
| Target Cells ($\mu$M) | PBMCs | Control (Without Immobilization of CH-296) | CH-296 |
| 10.0 | 100 | 100 | 100 |
| 1.0 | 101.58 | 86.33 | 99.59 |
| 0.1 | 88.24 | 80.78 | 89.13 |
| 0.01 | 72.03 | 64.48 | 67.28 |
| 0.001 | 25.18 | 26.72 | 48.35 |
| 0.0001 | 11.47 | 2.45 | 21.59 |
| 0.00001 | 6.32 | 3.07 | 15.6 |
| 0 | 8.4 | <0 | 8.17 |

As shown in Table 43, in the CTL population induced from the CH-296 group, even target cells to which an antigen peptide was added at an even lower concentration were killed, as compared to those of the control group and the PBMC group, so that a specific antigen-recognizing ability of the CTL population was high. In other words, it was clarified that the cultured cells in which a culture equipment immobilized with CH-296 at an early stage of the expansion of the T cell population as used could be induced to a CTL population having an even higher antigen-recognizing ability.

Example 17: Evaluation of Cytokine-Producing Ability of CD45RA$^+$ CCR7$^+$ T Cells Derived from Cells Expanded Using Gas-Permeable Culture Bag upon Stimulation at Early Stage and Subsequent Culture of CH-296
(1) Isolation of CD45RA$^+$ CCR7$^+$ T Cells and CD45RA$^-$ CCR7$^-$ T Cells
The cells prepared in item (6) of Example 14 were stained and subjected to in the same manner as in item (3) of Example 3.

(2) Immobilization of Anti-Human CD3 Antibody and Anti-Human CD28 Antibody and Stimulation of Cells
In order to stimulate isolated cells, an anti-human CD3 antibody and an anti-human CD28 antibody were immobilized to a culture plate in the same manner as in item (4) of Example 3. of the cell population obtained in item (1) of Example 17 was suspended in 0.5% GT-T503, cells were added to each well of the prepared plate so as to have a density of 2 $\times$ 10$^5$ cells/0.2 mL, and cultured at 37˚C in 5% $CO_2$. After 24 hours, the culture supernatant was collected, and used for the experiment for the determination of cytokine according to ELISA method.

(3) Determination of IL-2 Production According to ELISA Method
In expansion of lymphocytes for clinical purposes, a gas-permeable culture bag that is capable of culturing the cells in a closed system has been used. Even when the cells were expanded using the bag in Example 14, in the CH-296 group, the results of a higher percentage of the CD45RA$^+$ CCR7$^+$ T cells were as compared to those of the control group. In order to confirm that this CD45RA$^+$ CCR7$^+$ T cell population maintains its property as naive T-like cells, the IL-2-producing ability of CD45RA$^+$ CCR7$^+$ T cells and CD45RA$^-$ CCR7$^-$ T cells was evaluated in the same as in item (6) of Example 3. The results are shown in Table 44.

[Table 44]

Table 44

| Immobilization of CH-296 | | IL-2 (pg/mL) |
|---|---|---|
| CD8$^+$ T Cells | CD45RA$^+$ CCR7$^+$ T Cells | 347.2 |
| | CD45RA$^-$ CCR7$^-$ T Cells | < 0 |
| CD8$^-$ T Cells | CD45RA$^+$ CCR7$^+$ T Cells | 1686.7 |
| | CD45RA$^-$ CCR7$^-$ T Cells | 215.6 |

As shown in Table 44, the IL-2 production in CD45RA$^+$ CCR7$^+$ T cells was dominant in both the CD8$^+$ T cells and CD8$^-$ T cells. As already shown in item (6) of Example 3, it was shown also in this example that CD45RA$^+$ CCR7$^+$ T cell population of frozen cells that were cultured in a bag using CH-296 had properties that serve as naive T-like cells.

Example 18: Analysis of CD45RA$^+$ CCR7$^+$ T Cells in T Cell Population Expanded Using H-296, CH-271, H-271, or C-CS1
(1) Immobilization of Anti-Human CD3 Antibody and H-296, CH-271, H-271, or C-CS1 Fragment
The same procedures as in item (1) of Example 4 were carried out, except that the CH-296 fragment was not used but each of the fragments H-296, CH-271, H-271, or C-CS1 was added instead to the group with addition of the fragment so as to have a final concentration of 25 $\mu$g/mL.

(2) Expansion of T Cell Population
The same procedures as those in item (1) of Example 3 were carried out except that on the fourth day from the initiation of culture, the culture medium was diluted about 14-fold, and 10 mL of the dilution was added to a fresh 25 cm$^2$ cell culture flask (manufactured by Corning), and that the subculture was not carried out on the seventh day. The culture was continued for 9 days. The results of the expansion folds on the eighth day from the initiation of culture are shown in Table 45.

[Table 45]

Table 45

| | Expansion Fold (folds) |
|---|---|
| Control (Without Immobilization of CH-296) | $\times$ 207 |
| H-296 | $\times$ 264 |
| CH-271 | $\times$ 250 |
| H-271 | $\times$ 221 |
| C-CS1 | $\times$ 252 |

As shown in Table 45, in the group in which a culture equipment immobilized with H-296, CH-271, H-271, or C-CS1 at an early stage of the expansion of the T cell population was used (hereinafter referred to as "the fragment group"), the expansion of the T cell population was as compared to that of the control group.

(3) Analysis of CD45RA⁺ CCR7⁺ T Cells

For the cells prepared in item (2) of Example 18, a percentage of CD45RA$^+$ CCR7$^+$ T cells on the ninth day from the initiation of culture was calculated in the same manner as in item (2) of Example 3. The results are shown in Table 46.

[Table 46]

Table 46

|  | CD45RA$^+$ CCR7$^+$ T Cells (%) |
| --- | --- |
| Control (Without Immobilization of CH-296) | 38.7 |
| H-296 | 58.15 |
| CH-271 | 56.3 |
| H-271 | 49.75 |
| C-CS 1 | 44.79 |

As shown in Table 46, in the fragment group, the results of higher CD45RA$^+$ CCR7$^+$ T cell population in the T cell population during the culture were obtained, as compared to that of the control group.

INDUSTRIAL APPLICABILITY

According to the present invention, a method for producing a T cell population is provided. The method is suitably used in, for example, immunotherapy, as a T cell population containing a high percentage of T cells capable of expressing CD45RA and at least one member selected from the group consisting of CD62L, CCR7, CD27 and CD28. Accordingly, the method of the present invention is expected to greatly contribute to medicinal fields.

BRIEF DESCRIPTION OF THE DRAWINGS

[Figure 1] A schematic view showing a domain structure of fibronectin.
[Figure 2] A graph showing a suppressive action of tumor formation of mouse by administration of T cells.

SEQUENCE FREE TEXT

SEQ ID NO: 1: Partial region of fibronectin named III-8.
SEQ ID NO: 2: Partial region of fibronectin named III-9.
SEQ ID NO: 3: Partial region of fibronectin named III-10.
SEQ ID NO: 4: Partial region of fibronectin named III-11.
SEQ ID NO: 5: Partial region of fibronectin named III-12.
SEQ ID NO: 6: Partial region of fibronectin named III-13.
SEQ ID NO: 7: Partial region of fibronectin named III-14.
SEQ ID NO: 8: Partial region of fibronectin named CS-1.
SEQ ID NO: 9: Fibronectin fragment named C-274.
SEQ ID NO: 10: Fibronectin fragment named H-271.
SEQ ID NO: 11: Fibronectin fragment named H-296.
SEQ ID NO: 12: Fibronectin fragment named CH-271.
SEQ ID NO: 13: Fibronectin fragment named CH-296.
SEQ ID NO: 14: Fibronectin fragment named C-CS1.
SEQ ID NO: 15: Fibronectin fragment named CH-296Na.
SEQ ID NO: 16: Fibronectin fragment named CHV-89.
SEQ ID NO: 17: Fibronectin fragment named CHV-90.
SEQ ID NO: 18: Fibronectin fragment named CHV-92.
SEQ ID NO: 19: Fibronectin fragment named CHV-179.
SEQ ID NO: 20: Fibronectin fragment named CHV-181.
SEQ ID NO: 21: Fibronectin fragment named H-275-Cys.
SEO ID NO: 22: Epitope peptide derived from melanoma antigen MART-1.

SEQ ID NO: 23: Mouse fibronectin fragment named CH-296.

## Claims

1. A method for preparing a T cell population, wherein the T cell population expresses CD45RA and expresses at least one selected from the group consisting of CD62L, CCR7, CD27, and CD28, **characterized in that** the method comprises the step of culturing a cell population comprising T cells, in the presence of fibronectin, a fragment thereof or a mixture thereof.

2. The method according to claim 1, wherein the total cultured days comprising the culture step are from 4 to 14 days.

3. The method according to claim 1 or 2, wherein the culture in the presence of fibronectin, a fragment or a mixture thereof is carried out at least at the initiation of culture.

4. The method according to claim 3, **characterized in that** the culture in the presence of fibronectin, a fragment thereof or a mixture thereof is carried out at least for one day or more.

5. The method according to any one of claims 1 to 4, wherein the culture step in the presence of fibronectin, a fragment thereof or a mixture thereof is carried out in the presence of a CD3 ligand.

6. The method according to claim 5, wherein the CD3 ligand is an anti-CD3 antibody.

7. The method according to any one of claims 1 to 6, wherein the fibronectin fragment is a polypeptide (m) comprising at least any one of the amino acid sequences shown in SEQ ID NOs: 1 to 8 of Sequence Listing, or a polypeptide (n) comprising at least one amino acid sequence having substitution, deletion, insertion or addition of one or the plural number of amino acids in any one of said amino acid sequences, wherein the polypeptide (n) has a function equivalent to that of said polypeptide (m).

8. The method according to claim 7, wherein the fibronectin fragment is a polypeptide comprising all of the amino acid sequences shown in SEQ ID NOs: 1 to 3 and 5 to 8 of Sequence Listing.

9. The method according to any one of claims 1 to 8, further comprising the step of separating a cell population that expresses at least one selected from the group consisting of CD45RA, CD62L, CCR7, CD27, and CD28.

10. The method according to any one of claims 1 to 9, further comprising the step of transducing a foreign gene the cell population.

11. The method according to claim 10, the foreign gene is transduced using retrovirus vector, adenovirus vector, adeno-associated virus vector, lentivirus vector or simian virus vector.

12. A T cell population obtained by the method as defined in any one of claims 1 to 11, wherein the T cell population expresses CD45RA and expresses at least one selected from the group consisting of CD62L, CCR7, CD27, and CD28.

13. A medicament comprising as an effective ingredient the T cell population obtained by the method as defined in any one of claims 1 to 11, wherein the T cell population expresses CD45RA and expresses at least one selected from the group consisting of CD62L, CCR7, CD27, and CD28.

14. A method for treating or preventing a disease, comprising the step of administering to a subject an effective amount of the T cell population obtained by the method as defined in any one of claims 1 to 11, wherein the T cell population expresses CD45RA and expresses at least one selected from the group consisting of CD62L, CCR7, CD27, and CD28.

15. Use of the T cell population obtained by the method as defined in any one of claims 1 to 11, wherein the T cell population expresses CD45RA and expresses at least one selected from the group consisting of CD62L, CCR7, CD27, and CD28, in the manufacture of a medicament.

16. A method for preparing a T cell population, **characterized in that** the method comprises the step of stimulating the T cell population obtained by the method as defined in any one of claims 1 to 11, wherein the T cell population expresses CD45RA and expresses at least one selected from the group consisting of CD62L, CCR7, CD27, and CD28, with at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine.

17. A T cell population obtained by the method as defined in claim 16.

18. A medicament comprising as an effective ingredient the T cell population obtained by the method as defined in claim 16.

19. A method for treating or preventing a disease, comprising the step of administering to a subject an effective amount of the T cell population obtained by the method as defined in claim 16.

20. Use of the T cell population obtained by the method as in claim 16 in the manufacture of a medicament.

21. A medicament comprising:

(a) a preparation comprising as an effective ingredient the T cell population obtained by the method as defined in any one of claims 1 to 11, wherein the T cell population expresses CD45RA and expresses at least one selected from the group consisting of CD62L, CCR7, CD27, and CD28; and
(b) a preparation comprising as an effective ingredient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine,

wherein the preparations are comprised in the medicament as two separate preparations simultaneously or separately administered.

**22.** A method for treating a disease, **characterized in that** the method comprises the following steps (a) and (b) of:

(a) administering to a patient the T cell population obtained by the method as defined in any one of claims 1 to 11, wherein the T cell population expresses CD45RA and expresses at least one selected from the group consisting of CD62L, CCR7, CD27, and CD28; and
(b) administering to a patient at least one stimulating factor selected from the group consisting of a cell capable of presenting an antigen, a cell having presented an antigen, an antigen, a CD3 ligand, a CD28 ligand, a cytokine, a chemokine, and a cell capable of producing a cytokine.

[Figure 1]

EP 1 939 278 A1

76

Binding to
VLA-5

IIICS

SH

cs-1

SH

NH₂ — 1 2 3 4 5 6 7 8 9 10 11 12 13 14 — COOH

(DNA)

S S
| |
S-S

Fibrin-Heparin
Binding Domain

Collagen Binding
Domain

Cell Binding Domain

Heparin
Binding
Domain

Binding to
VLA-4

Fibrin
Binding
Domain

S-S
Binding
Domain

Type I Repeating Sequence

Type II Repeating Sequence

Type III Repeating Sequence

[Figure  2]

Error Bar ± S.E.M., *p<0.05 vs. Control Group, Student's t-test

## INTERNATIONAL SEARCH REPORT

International application No.

PCT/JP2006/319105

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
C12N5/06, A61K35/14, A61P1/16, A61P31/04, A61P31/10, A61P31/12, A61P31/16, A61P35/00, A61P37/04, C12N5/10, A61K35/76, C07K14/78, C12N15/09

Documentation searched other than minimum documentation to the extent that such documents are included in the fields searched
Jitsuyo Shinan Koho        1922-1996    Jitsuyo Shinan Toroku Koho    1996-2006
Kokai Jitsuyo Shinan Koho   1971-2006    Toroku Jitsuyo Shinan Koho    1994-2006

Electronic data base consulted during the international search (name of data base and, where practicable, search terms used)
BIOSIS/MEDLINE/WPIDS(STN), CA(STN), JSTPlus(JDream2),
SwissProt/PIR/Geneseq

**C. DOCUMENTS CONSIDERED TO BE RELEVANT**

| Category* | Citation of document, with indication, where appropriate, of the relevant passages | Relevant to claim No. |
|---|---|---|
| X <br> <br> Y | WO 2005/019450 A1 (Takara Bio Inc.), <br> 03 March, 2005 (03.03.05), <br> & EP 1666589 A1        & AU 2004-267313 A1 <br> & KR 2006-039940 A      & MX 2006-002039 A1 | 1-13,15-18, <br> 20 <br> 21 |
| Y | WO 2004/018667 A1 (Kirin Brewery Co., Ltd.), <br> 04 March, 2004 (04.03.04), <br> & AU 2003-254950 A1      & TW 2004-013406 A | 21 |
| P,X <br> <br> P,Y | Nobuko MURAKI et al., "RetroNectin® o Kumiawaseta T Saibo Kakudai Baiyo (I): Kakudai Baiyo ga Anteika shi, NaiveT-yo Saibo ga Kohiritsu de Zoshoku", Dai 65 Kai Annual Meeting of the Japan Cancer Association Kiji, 28 August, 2006 (28.08.06), page 330 | 1-13,15-18, <br> 20 <br> 21 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search <br> 22 December, 2006 (22.12.06) | Date of mailing of the international search report <br> 09 January, 2007 (09.01.07) |
| Name and mailing address of the ISA/ <br> Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2006/319105 |

C (Continuation).   DOCUMENTS CONSIDERED TO BE RELEVANT

| Category* | Citation of document, with indication, where appropriate, of the relevant passages | Relevant to claim No. |
| --- | --- | --- |
| <u>P,X</u><br><br>P,Y | Mitsuko IDENO et al., "RetroNectin® o Kumiawaseta T Saibo Kakudai Baiyo (II): Kakudai Baiyo sareta T Saibo Shudan wa NaiveT-yo Saibo no Hiritsu ga Takaku, Takai Kogen Ninshikino o Hakki", Dai 65 Kai Annual Meeting of the Japan Cancer Association Kiji, 28 August, 2006 (28.08.06), page 330 | 1-13,15-18,<br><u>20</u><br><u>21</u> |
| A | Davis LS et al., , "Fibronectin promotes proliferation of naive and memory T cells by signaling through both the VLA-4 and VLA-5 integrin molecules.", J. Immunol., 1990, Vol.145 , No.3, pp.785-793 | 1-13,15-18<br>20,21 |
| A | GATTINONI L, et al.," Acquisition of full effector function in vitro paradoxically impairs the in vivo antitumor efficacy of adoptively transferred CD8+ T cells", J. Clin. Invest. , 2005. June, Vol.115, No.6, pp.1616-1626 | 1-13,15-18,<br>20,21 |
| A | STURM A, et al.," Dual function of the extracellular matrix: stimulatory for cell cycle progression of naive T cells and antiapoptotic for tissue-derived memory T cells", J. Immunol., 2004, Vol.173, No.6, pp.3889-3900 | 1-13,15-18,<br>20,21 |
| A | MATSUYAMA T, et al., "Activation of CD4 cells by fibronectin and anti-CD3 antibody. A synergistic effect mediated by the VLA-5 fibronectin receptor complex", J. Exp. Med., 1989, Vol.170, No.4, pp.1133-1148 | 1-13,15-18,<br>20,21 |
| A | HALVORSON MJ, et al., "alpha4 and alpha5 integrins costimulate the CD3-dependent proliferation of fetal thymocytes", Cell. Immunol., 1998, Vol.189, No.1, pp.1-9 | 1-13,15-18,<br>20,21 |
| A | SIMON MM, et al., "The outer surface lipoprotein A of Borrelia burgdorferi provides direct and indirect augmenting/co-stimulatory signals for the activation of CD4+ and CD8+ T cells", Immunol. Lett., 1995, Vol.46, No.3, pp.137-142 | 1-13,15-18,<br>20,21 |
| A | DARDALHON V et al., "Highly efficient gene transfer in naive human T cells with a murine leukemia virus-based vector", Blood , 2000, Vol.96, No.3, pp.885-893 | 1-13,15-18,<br>20,21 |

Form PCT/ISA/210 (continuation of second sheet) (April 2005)

**INTERNATIONAL SEARCH REPORT**

| International application No. |
| --- |
| PCT/JP2006/319105 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | RIDDELL SR et al., "The use of anti-CD3 and anti-CD28 monoclonal antibodies to clone and expand human antigen-specific T cells", J. Immunol. Methods , 1990, Vol.128, No.2, pp.189-201 | 1-13,15-18, 20,21 |
| A | Hiroaki SAGAWA et al., "Ko-CD3 Kotai to RetroNectin® o Kumiawaseru Koto ni yoru LAK Saibo Inyu Ryoho no Kairyo", Dai 62 Kai Annual Meeting of the Japan Cancer Association Kiji, 2003, page 438 | 1-13,15-18, 20,21 |
| A | Mitsuko IDENO et al., "Soshikitai Human Fibronectin Fragment Shigeki ni yoru Kasseika CTL no Tairyo Expansion-ho", Dai 62 Kai Annual Meeting of the Japan Cancer Association Kiji, 2003, page 175 | 1-13,15-18, 20,21 |

Form PCT/ISA/210 (continuation of second sheet) (April 2005)

**INTERNATIONAL SEARCH REPORT**

International application No.

PCT/JP2006/319105

Continuation of A. CLASSIFICATION OF SUBJECT MATTER
(International Patent Classification (IPC))

*C12N5/06*(2006.01)i, *A61K35/14*(2006.01)i, *A61P1/16*(2006.01)i,
*A61P31/04*(2006.01)i, *A61P31/10*(2006.01)i, *A61P31/12*(2006.01)i,
*A61P31/16*(2006.01)i, *A61P35/00*(2006.01)i, *A61P37/04*(2006.01)i,
*C12N5/10*(2006.01)i, *A61K35/76*(2006.01)n, *C07K14/78*(2006.01)n,
*C12N15/09*(2006.01)n

(According to International Patent Classification (IPC) or to both national
classification and IPC)

Form PCT/ISA/210 (extra sheet) (April 2005)

**INTERNATIONAL SEARCH REPORT**

International application No.

PCT/JP2006/319105

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 14, 19, 22

because they relate to subject matter not required to be searched by this Authority, namely:

The inventions as set forth in claims 14, 19 and 22 are relevant to methods for treatment of the human body by therapy and thus relate to a subject matter which this International Searching Authority is not required, under the provisions of Article 17(2)(a)(i) of the PCT (continued to extra sheet)

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable,
the                              payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

| INTERNATIONAL SEARCH REPORT | International application No. |
| --- | --- |
| | PCT/JP2006/319105 |

Continuation of Box No.II-1 of continuation of first sheet(2)

and Rule 39.1(iv) of the Regulations under the PCT, to search.

Form PCT/ISA/210 (extra sheet) (April 2005)

## REFERENCES CITED IN THE DESCRIPTION

**Patent documents cited in the description**

- WO 03016511 A
- WO 03080817 A
- WO 2005019450 A
- US 5198423 A
- US 5102988 A

- JP 3104178 B
- JP 2729712 B
- WO 9718318 A
- WO 0009168 A

**Non-patent literature cited in the description**

- **DEANE F. MOMER.** FIBRONECTIN. ACADEMIC PRESS INC, 1988, 1-8
- **KIMIZUKA F.** *J. Biochem.,* 1991, vol. 110 (2), 284-291
- **HANENBERG H.** *Human Gene Therapy,* 1997, vol. 8 (18), 2193-2206
- **GATTINONI L.** *J. Clin. Invest.,* 2005, vol. 115 (6), 1616-1626
- **BENIGNI F.** *J. Immunol.,* 2005, vol. 175 (2), 739-748
- **RUOSLAHTI E. et al.** *J. Biol. Chem.,* 1981, vol. 256 (14), 7277-7281
- **KIMIDUKA F. et al.** *J. Biochem.,* 1991, vol. 110, 284-291

- **KORNBRIHTT A. R. et al.** *EMBO J.,* 1985, vol. 4 (7), 1755-1759
- **SEKIGUCHI K. et al.** *Biochemistry,* 1986, vol. 25 (17), 4936-4941
- **WILLIAMS D. A. et al.** *Nature,* 1991, vol. 352, 438-441
- **PLEBANSKI M. et al.** *Eur. J. Immunol,* 1995, vol. 25 (6), 1783-1787
- **LICHTENFELS R. et al.** *J. Immunol. Methods,* 1994, vol. 172 (2), 227-239
- **VALMORI D. et al.** *J. Immunol.,* 1998, vol. 160, 1750-1758